# EUROPÄISCHE PATENTSCHRIFT

(11) **EP 1 622 894 B1**
(45) Veröffentlichungstag und Bekanntmachung des Hinweises auf die Patenterteilung: **14.01.2009**
(21) Anmeldenummer: 04728552.3
(22) Anmeldetag: 21.04.2004
(51) Int. Cl.: C07D 403/06, A61P 35/00, A61K 31/4164

(54) **ANTIANDROGENE PYRROLIDINE MIT TUMORHEMMENDER WIRKSAMKEIT**
ANTITUMORAL ANTIANDROGENIC PYRROLIDINES
PYRROLIDINES ANTIANDROGENES A ACTION ANTITUMORALE

(30) Priorität: 09.05.2003 DE 10322108
(43) Veröffentlichungstag der Anmeldung: 08.02.2006
(73) Patentinhaber: Bayer Schering Pharma Aktiengesellschaft, 13353 Berlin (DE)
(72) Erfinder: CLEVE, Arwed, 10707 Berlin (DE); SCHULZE, Volker, 16562 Bergfelde (DE); ZOPF, Dieter, 14167 Berlin (DE); HOFFMANN, Jens, 16567 Mühlenbeck (DE); REICHEL, Andreas, 10179 Berlin (DE); PARCZYK, Karsten, 12207 Berlin (DE)
(86) Internationale Anmeldenummer: PCT/EP2004/004225
(87) Internationale Veröffentlichungsnummer: WO 2004/099188

(56) Entgegenhaltungen:
- WO-A-00/37430
- WO-A-97/00071
- US-A- 5 411 981
- VAN DORT M E ET AL: "Design, synthesis and pharmacological characterization of 4-(4,4-dimethyl-3-(4-hydroxybutyl)-5-oxo-2 -thioxo-1-imidazolidinyl)-2 iodobenzonitrile as a high-affinity nonsteroidal androgen receptor ligand" JOURNAL OF MEDICINAL CHEMISTRY, AMERICAN CHEMICAL SOCIETY. WASHINGTON, US, Bd. 43, Nr. 17, 2000, Seiten 3344-3347, XP002973524 ISSN: 0022-2623

## Beschreibung

Die vorliegende Erfindung betrifft antiandrogene *N*-[ω-[3-[4-Cyan-3-(trifluormethyl)-phenyl]-5,5-dimethyl-4-oxo-2-thioxoimidazolidin-1-yl]alkyl]-substituierte Pyrrolidine, mit einem stark ausgeprägten antiproliferativen Wirkungsprofil, Verfahren zu deren Herstellung, sowie pharmazeutische Präparate enthaltend die erfindungsgemäßen Pyrrolidine und deren Verwendung zur Herstellung von Arzneimitteln.

In den Industrieländern ist das Prostatakarzinom hinter dem Lungenkarzinom die zweite Hauptursache für den Tod durch Krebs bei Männern. Bei Männern über 55 Jahre sind 4% der Todesfälle auf eine Prostatatumorerkrankung zurückzuführen und es wird vermutet, dass der Anteil bei Männern über 80 auf bis zu 80% der Todesfälle ansteigt. Die Sterberate ist zwar immer noch relativ niedrig, sie steigt jedoch jährlich um etwa 14%. Die Anzahl der Männer, bei denen ein Prostatatumor diagnostiziert wurde, ist in den letzten Jahren um 30% gestiegen, was allerdings weniger auf eine steigende Anzahl von Neuerkrankungen, sondern vielmehr darauf zurückzuführen ist, dass die Bevölkerung generell älter wird, dass die Diagnoseverfahren sich verbessert haben und dass systematische Screeningprogramme eingeführt wurden (E. J. Small, D. M. Reese, Curr. Opi. Oncol. 2000, 12, 265-272).

Der Prostatatumor wächst in frühen Stadien androgenabhängig. Solange der Tumor lokal auf die Prostata begrenzt ist, kann er durch einen chirurgischen Eingriff entfernt oder durch Strahlentherapie behandelt werden, wobei diese Methoden mit entsprechenden Risiken verbunden sind. In den Fällen, in denen der Tumor nicht mehr lokal begrenzt ist und schon Metastasen gebildet hat, wird der Tumor palliativ durch Reduktion der Testosteronspiegel im Blut behandelt. Dies erfolgt entweder chirurgisch durch Kastration oder medikamentös durch Behandlung mit Antiandrogenen (Bicalutamid, Cyproteronacetat, Flutamid), LHRH-Agonisten (Buserelin, Zoladex), LHRH-Antagonisten (Cetrorelix) oder 5α-Reduktasehemmern (Finasterid). Da bei einer chirurgischen Kastration die adrenale Androgensynthese unbeeinflusst bleibt, wird in jüngerer Zeit häufig eine kombinierte chirurgische und medikamentöse Behandlung durchgeführt (S. Leewansangtong, E. D. Crawford, Endocrine-Related Cancer 1998, 5, 325-339). Diese Behandlung hat aber nur vorübergehenden Erfolg, da es in der Regel nach spätestens zwei Jahren zum erneuten Wachstum des Tumors kommt, der in den meisten Fällen dann hormonunabhängig ist (L. J. Denis, K. Griffith, Semin. in Surg. Onc. 2000, 18, 52-74). Gegen diese fortgeschrittenen Stadien gibt es bis heute trotz intensiver Forschung in den letzten 50 Jahren keine wirksame Behandlung. Die 5-Jahres Überlebensrate liegt bei diesen Patienten unter 15%.

Es gibt verschiedene Hinweise, die zeigen, dass der Androgenrezeptor bei der Entwicklung und dem Wachstum des Prostatatumors nicht nur in den frühen hormon-abhängigen, sondern auch in späten hormonunabhängigen Stadien der Tumorprogression, eine wichtige Rolle spielt.

Der Androgenrezeptor gehört zur Familie der Steroidhormonrezeptoren, die als Transkriptionsfaktoren wirken. Der Androgenrezeptor bindet Androgene, wodurch er stabilisiert und vor einem schnellen proteolytischen Abbau geschützt wird. Nach Hormonbindung wird er in den Kern transportiert, wo er durch Bindung an sogenannte androgenresponsive DNA-Elemente, die in Promoterregionen liegen, bestimmte Gene aktiviert (D. J. Lamb et. al. Vitam. Horm. 2001, 62, 199-230).

Untersuchungen an Prostatatumoren zeigen, dass in 30% der fortgeschrittenen Tumore eine Amplifikation des Androgenrezeptorgenlocus detektiert wurde. In anderen Fällen wurde eine Reihe von Mutationen im Androgenrezeptorgen gefunden, die in verschiedenen Domänen des Androgenrezeptormoleküls lokalisiert sind und zu veränderten Rezeptoreigenschaften führen. Mutierte Rezeptoren können entweder eine höhere Affinität für Androgene besitzen, konstitutiv aktiv werden, ihre Ligandenspezifität ändern, sodass sie von anderen Steroidhormonen oder sogar Antiandrogenen aktiviert werden, über Wechselwirkungen mit Molekülen aus anderen wachstumsfördernden Signalübertragungswegen aktiviert werden, die Interaktion mit Cofaktoren ändern, oder andere Zielgene aktivieren (J. P. Elo, T. Visakorpi, Ann. Med. 2001, 33, 130-41).
Die Identifizierung von Antiandrogenen, die nicht nur den natürlichen Androgenrezeptor, sondern auch dessen mutierte Formen hemmen und einen verstärkten antiproliferativen Effekt an Tumorzellen aufweisen, wäre vermutlich sehr hilfreich, um Prostatatumoren in verschiedenen Stadien zu behandeln. Solche Verbindungen können den Zeitraum bis zum Wiederauftreten des Tumorwachstums deutlich verlängern.

Untersuchungen mit nichtsteroidalen Antiandrogenen haben gezeigt, dass sie gegenüber den steroidalen Verbindungen Vorteile aufweisen und daher zu bevorzugen sind. So kann mit nichtsteroidalen Verbindungen eine selektivere Wirkung mit geringeren adversen Nebenwirkungen erzielt werden. Im Gegensatz zu den steroidalen Antiandrogenen fehlt den bekannten Nichtsteroiden Bicalutamid und Flutamid z.B. die progestagene Aktivität und außerdem führt deren Verwendung zu einer Erhöhung der Testosteronspiegel im Serum, das klinisch zu einer Erhaltung der Potenz führen könnte (P. Reid, P. Kantoff, W. Oh, Investigational New Drugs 1999, 17, 271-284).

Nichtsteroidale Antiandrogene werden in US 5,411,981 bzw. US Re. 35956 (Phenylimidazolidinderivate), in WO 97/00071 (spezifisch substituierte Phenyldimethylhydantoine sowie deren Imino- bzw. Thionderivate) in WO 00/37430 (Phenylalanine, Phenylhydantoine sowie Phenylharnstoffe), in WO 01/58855 (Aminopropanilide) und in EP 1122242 (substituierte Cyanphenylpiperazine) beschrieben.

US Re. 35956 beschreibt unter anderem [4-Cyan-3-(trifluormethyl)phenyl]-substituierte Thiohydantoine mit einem kurzkettigen, endständig substituierten Rest, wobei es sich bei der Kette bevorzugt um eine C₁-C₄-Kette handelt.
Die in US Re. 35956 explizit offenbarten Verbindungen wirken zwar antiandrogen, jedoch nur wenig antiproliferativ in Zellen, die aus humanen Prostatakarzinomen stammen.
Für eine effektive Therapie von androgenabhängigen Tumoren und/oder anderen proliferativen Erkrankungen ist eine zusätzliche antiproliferative Wirkung erforderlich.

Die Aufgabe der vorliegenden Erfindung besteht daher darin, oral bioverfügbare antiandrogene Verbindungen mit gesteigerter antiproliferativer Wirkung zur Verfügung zu stellen, die das Wachstum androgenabhängiger gut- oder bösartiger Tumoren hemmen, bzw. androgenabhängige proliferative Erkrankungen lindern oder heilen können.

Diese Aufgabe wird erfindungsgemäß durch die *N*-[ω-[3-[4-Cyan-3-(trifluormethyl)-phenyl]-5,5-dimethyl-4-oxo-2-thioxoimidazolidin-1-yl]alkyl]-substituierten Pyrrolidine der allgemeinen Formel I gelöst: 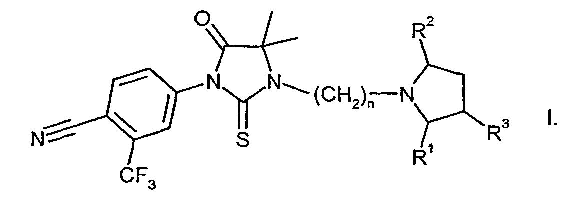 worin
- n: eine ganze Zahl zwischen 6 und 9,
- R¹ und R²: unabhängig voneinander ein Wasserstoffatom, eine unverzweigte C₁-C₄-Alkylgruppe, eine verzweigte C₃-C₅-Alkylgruppe, eine unverzweigte Hydroxy-C₁-C₄-alkylgruppe, eine verzweigte Hydroxy-C₃-C₅-alkylgruppe, eine unverzweigte C₁-C₄-Alkoxy-C₁-C₄-alkylgruppe, eine verzweigte C₁-C₄-Alkoxy-C₃-C₅-alkylgruppe, eine unverzweigte C₁-C₄-Alkanoyloxy-C₁-C₄-alkylgruppe, eine verzweigte C₁-C₄-Alkanoyloxy-C₃-C₅-alkylgruppe, eine (Pyrrolidin-1-yl)methylgruppe, eine Carboxygruppe, eine C₁-C₄-Alkoxycarbonylgruppe oder eine Aminocarbonylgruppe,
oder
- R¹ und R²: gemeinsam eine 2-Hydroxypropan-1,3-diylbrücke;
- R³: ein Wasserstoffatom oder eine Hydroxygruppe
bedeuten können.

Es wurde festgestellt, dass sich die antiproliferative Wirkung von Verbindungen des *N-*[3-[4-Cyan-3-(trifluormethyl)phenyl]-5,5-dimethylthiohydantoin-Typs überraschenderweise unter Erhalt der antiandrogenen Wirksamkeit steigern lässt, wenn der N-1-Stickstoff einen Pyrrolidin-1-ylalkyl-Substituenten trägt. Die erfindungsgemäßen Verbindungen zeichnen sich durch eine Alkylenkette eines definierten Längenbereiches, die den Pyrrolidinkern mit dem Thiohydantoinkern verbindet, aus. Je nach Kombination der heterocyclischen Endgruppe mit der Länge der diese mit dem Thiohydantoinkern verbindenden Alkylenkette kann ein zusätzlicher Effekt mehr oder weniger ausgeprägt auftreten, der zur Destabilisierung des Androgenrezeptors führt.

Die vorliegende Erfindung umfasst ein Verfahren zur Herstellung der erfindungsgemäßen Verbindungen der allgemeinen Formel I, in dem Verbindungen der allgemeinen Formel II 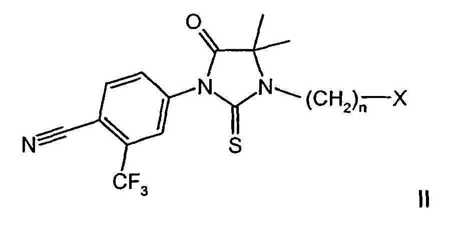 worin
- n: eine ganze Zahl zwischen 6 und 9,
- X: eine Abgangsgruppe,
mit Verbindungen der allgemeinen Formel III 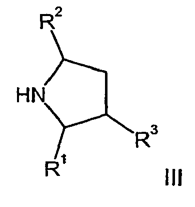 worin
- R¹ und R²: unabhängig voneinander ein Wasserstoffatom, eine unverzweigte C₁-C₄-Alkylgruppe, eine verzweigte C₃-C₅-Alkylgruppe, eine unverzweigte Hydroxy-C₁-C₄-alkylgruppe, eine verzweigte Hydroxy-C₃-C₅-alkylgruppe, eine unverzweigte C₁-C₄-Alkoxy-C₁-C₄-alkylgruppe, eine verzweigte C₁-C₄-Alkoxy-C₃-C₅-alkylgruppe, eine unverzweigte C₁-C₄-Alkanoyloxy-C₁-C₄-alkylgruppe, eine verzweigte C₁-C₄-Alkanoyloxy-C₃-C₅-alkylgruppe, eine (Pyrrolidin-1-yl)methylgruppe, eine Carboxygruppe, eine C₁-C₄-Alkoxycarbonylgruppe oder eine Aminocarbonylgruppe,
oder
- R¹ und R²: gemeinsam eine 2-Hydroxypropan-1,3-diylbrücke;
- R³: ein Wasserstoffatom oder eine Hydroxygruppe
bedeuten können,
in Gegenwart einer organischen Base umgesetzt werden.

Bei der Alkenylkette -(CH₂)ₙ- handelt es sich um die n-Hexylen-, n-Heptylen-, n-Octylen- oder *n*-Nonylengruppe.

Bei den unverzweigten Hydroxy-C₁-C₄-alkylgruppen kann es sich um eine Hydroxymethyl- (HOCH₂-), 2-Hydroxyethyl- (HOCH₂CH₂-), 1-Hydroxyethyl- [CH₃CH(OH)-], 3-Hydroxypropyl- (HOCH₂CH₂CH₂-), 2-Hydroxypropyl- [CH₃CH(OH)CH₂CH₂-], 1-Hydroxypropyl- [CH₃CH₂CH(OH)-], 4-Hydroxybutyl- (HOCH₂CH₂CH₂CH₂-), 3-Hydroxybutyl- [CH₃CH(OH)CH₂CH₂-], 2-Hydroxybutyl- [CH₃CH₂CH(OH)CH₂-] oder 1-Hydroxybutyl-Gruppe [CH₃CH₂CH₂CH(OH)-] handeln.

Bei den verzweigten Hydroxy-C₃-C₅-alkylgruppen kann es sich um eine 1-Hydroxy-1-methylethyl- [(CH₃)₂C(OH)-], 2-Hydroxy-1-methylethyl- [HOCH₂CH(CH₃)-], 1-Hydroxy-1-methylpropyl- [CH₃CH₂C(CH₃)(OH)-], 2-Hydroxy-1-methylpropyl- [CH₃CH(OH)CH(CH₃)-], 3-Hydroxy-1-methylpropyl- [HOCH₂CH₂CH(CH₃)-], 1-(Hydroxymethyl)propyl-[CH₃CH₂C(CH₂OH)-], 1-Hydroxy-2-methylpropyl- [(CH₃)₂CHCH(OH)-], 2-Hydroxy-2-methylpropyl- [CH₃C(OH)(CH₃)CH₂-], 3-Hydroxy-2-methylpropyl- [HOCH₂CH(CH₃)CH₂-], 1-(Hydroxymethyl)butyl- [CH₃CH₂CH₂CH(CH₂OH)-], 1-Hydroxy-1-methylbutyl-[CH₃CH₂CH₂C(CH₃)(OH)-], 2-Hydroxy-1-methylbutyl- [CH₃CH₂CH(OH)CH(CH₃)-], 3-Hydroxy-1-methylbutyl- [CH₃CH(OH)CH₂CH(CH₃)-], 4-Hydroxy-1-methylbutyl-[HOCH₂CH₂CH₂CH(CH₃)-], 3-Hydroxy-1-ethylpropyl- [HOCH₂CH₂CH(CH₂CH₃)-], 2-Hydroxy-1-ethylpropyl- [CH₃CH(OH)CH(CH₂CH₃)-], 1-Hydroxy-1-ethylpropyl-[CH₃CH₂C(CH₂CH₃)(OH)-], 1-Hydroxy-3-methylbutyl- [CH₃CH(CH₃)CH₂CH(OH)-], 2-Hydroxy-3-methylbutyl- [(CH₃)₂CHCH(OH)CH₂-], 3-Hydroxy-3-methylbutyl-[CH₃C(CH₃)(OH)CH₂CH₂-], 4-Hydroxy-3-methylbutyl- [HOCH₂CH(CH₃)CH₂CH₂-], 1-(Hydroxymethyl)-1-methylpropyl- [CH₃CH₂C(CH₃)(CH₂OH)-], 2-Hydroxy-1,1-dimethylpropyl- [CH₃CH(OH)C(CH₃)₂-], 3-Hydroxy-1,1-dimethylpropyl-[HOCH₂CH₂C(CH₃)₂-], 1-(Hydroxymethyl)-2-methylpropyl- [(CH₃)₂CHCH(CH₂OH)-], 1-Hydroxy-1,2-dimethylpropyl- [(CH₃)₂CHC(OH)(CH₃)-], 2-Hydroxy-1,2-dimethylpropyl-[(CH₃)₂C(OH)CH(CH₃)-], 3-Hydroxy-1,2-dimethylpropyl- [HOCH₂CH(CH₃)CH(CH₃)-], 1-Hydroxy-2,2-dimethylpropyl- [(CH₃)₃CCH(OH)-], 3-Hydroxy-2,2-dimethylpropyl-[HOCH₂C(CH₃)₂CH₂-], 1-Hydroxy-2-methylbutyl- [CH₃CH₂CH(CH₃)CH(OH)-], 2-Hydroxy-2-methylbutyl- [CH₃CH₂C(CH₃)(OH)CH₂-], 3-Hydroxy-2-methylbutyl-[CH₃CH(OH)CH(CH₃)CH₂-], 4-Hydroxy-2-methylbutyl- [HOCH₂CH₂CH(CH₃)CH₂-] oder 3-Hydroxy-2-ethylpropyl- [HOCH₂CH(CH₂CH₃)CH₂-]-Gruppe handeln.

Bei den unverzweigten C₁-C₄-Alkoxy-C₁-C₄-alkylengruppen kann es sich um eine Alkoxymethyl- (AlkOCH₂-), 2-Alkoxyethyl- (AlkOCH₂CH₂-), 1-Alkoxyethyl- [CH₃CH(OAlk)-], 3-Alkoxypropyl- (AlkOCH₂CH₂CH₂-), 2-Alkoxypropyl- [CH₃CH(OAlk)CH₂CH₂-], 1-Alkoxypropyl- [CH₃CH₂CH(OAlk)-], 4-Alkoxybutyl- (AlkOCH₂CH₂CH₂CH₂-), 3-Alkoxybutyl- [CH₃CH(OAlk)CH₂CH₂-], 2-Alkoxybutyl- [CH₃CH₂CH(OAlk)CH₂-] oder 1-Alkoxybutyl-Gruppe [CH₃CH₂CH₂CH(OAlk)-] handeln.

Bei den verzweigten C₁-C₄-Alkoxy-C₃-C₅-alkylgruppen kann es sich um eine 1-Alkoxy-1-methylethyl- [(CH₃)₂C(OAlk)-], 2-Alkoxy-1-methylethyl- [AlkOCH₂CH(CH₃)-], 1-Alkoxy-1-methylpropyl- [CH₃CH₂C(CH₃)(OAlk)-], 2-Alkoxy-1-methylpropyl-[CH₃CH(OAlk)CH(CH₃)-], 3-Alkoxy-1-methylpropyl- [AlkOCH₂CH₂CH(CH₃)-], 1-(Alkoxymethyl)propyl- [CH₃CH₂C(CH₂OAlk)-], 1-Alkoxy-2-methylpropyl-[(CH₃)₂CHCH(OAlk)-], 2-Alkoxy-2-methylpropyl- [CH₃C(OAlk)(CH₃)CH₂-], 3-Alkoxy-2-methylpropyl- [AlkOCH₂CH(CH₃)CH₂-], 1-(Alkoxymethyl)butyl-[CH₃CH₂CH₂CH(CH₂OAlk)-], 1-Alkoxy-1-methylbutyl- [CH₃CH₂CH₂C(CH₃)(OAlk)-], 2-Alkoxy-1-methylbutyl- [CH₃CH₂CH(OAlk)CH(CH₃)-], 3-Alkoxy-1-methylbutyl-[CH₃CH(OAlk)CH₂CH(CH₃)-], 4-Alkoxy-1-methylbutyl- [AlkOCH₂CH₂CH₂CH(CH₃)-], 3-Alkoxy-1-ethylpropyl- [AlkOCH₂CH₂CH(CH₂CH₃)-], 2-Alkoxy-1-ethylpropyl-[CH₃CH(OAlk)CH(CH₂CH₃)-], 1-Alkoxy-1-ethylpropyl- [CH₃CH₂C(CH₂CH₃)(OAlk)-], 1-Alkoxy-3-methylbutyl- [CH₃CH(CH₃)CH₂CH(OAlk)-], 2-Alkoxy-3-methylbutyl-[(CH₃)₂CHCH(OAlk)CH₂-], 3-Alkoxy-3-methylbutyl- [CH₃C(CH₃)(OAlk)CH₂CH₂-], 4-Alkoxy-3-methylbutyl- [AlkOCH₂CH(CH₃)CH₂CH₂-], 1-(Alkoxymethyl)-1-methylpropyl-[CH₃CH₂C(CH₃)(CH₂OAlk)-], 2-Alkoxy-1,1-dimethylpropyl- [CH₃CH(OAlk)C(CH₃)₂-], 3-Alkoxy-1,1-dimethylpropyl- [AlkOCH₂CH₂C(CH₃)₂-], 1-(Alkoxymethyl)-2-methylpropyl-[(CH₃)₂CHCH(CH₂OAlk)-], 1-Alkoxy-1,2-dimethylpropyl- [(CH₃)₂CHC(OAlk)(CH₃)-], 2-Alkoxy-1,2-dimethylpropyl- [(CH₃)₂C(OAlk)CH(CH₃)-], 3-Alkoxy-1,2-dimethylpropyl- [Al-kOCH₂CH(CH₃)CH(CH₃)-], 1-Alkoxy-2,2-dimethylpropyl- [(CH₃)₃CCH(OAlk)-], 3-Alkoxy-2,2-dimethylpropyl- [AlkOCH₂C(CH₃)₂CH₂-], 1-Alkoxy-2-methylbutyl-[CH₃CH₂CH(CH₃)CH(OAlk)-], 2-Alkoxy-2-methylbutyl- [CH₃CH₂C(CH₃)(OAlk)CH₂-], 3-Alkoxy-2-methylbutyl- [CH₃CH(OAlk)CH(CH₃)CH₂-], 4-Alkoxy-2-methylbutyl- [Al-kOCH₂CH₂CH(CH₃)CH₂-] oder 3-Alkoxy-2-ethylpropyl- [AlkOCH₂CH(CH₂CH₃)CH₂-]-Gruppe handeln.

Bei den C₁-C₄-Alkoxygruppen kann es sich beispielsweise um eine Methoxy-, Ethoxy-, *n*-Propoxy-, iso-Propoxy-, *n*-Butoxy-, *sec*-Butoxy-, *iso*-Butoxy- oder *tert*-Butoxygruppe handeln.

Bei den C₁-C₄-Alkanoylgruppen kann es sich beispielsweise um eine Formyl-, Acetyl-, Propanoyl-, Butanoyl- oder *iso*-Butanoylgruppe handeln.

Bei den unverzweigten C₁-C₄-Alkanoyloxy-C₁-C₄-alkylengruppen kann es sich um eine Alkanoyloxymethyl- (AlkCOOCH₂-), 2-Alkanoyloxyethyl- (AlkCOOCH₂CH₂-), 1-Alkanoyloxyethyl- [CH₃CH(OCOAlk)-], 3-Alkanoyloxypropyl- (AlkCOOCH₂CH₂CH₂-), 2-Alkanoyloxypropyl- [CH₃CH(OCOAlk)CH₂CH₂-], 1-Alkanoyloxypropyl-[CH₃CH₂CH(OCOAlk)-], 4-Alkanoyloxybutyl- (AlkCOOCH₂CH₂CH₂CH₂-), 3-Alkanoyloxybutyl- [CH₃CH(OCOAlk)CH₂CH₂-], 2-Alkanoyloxybutyl-[CH₃CH₂CH(OCOAlk)CH₂] oder 1-Alkanoyloxybutyl-Gruppe [CH₃CH₂CH₂CH(OCOAlk)-] handeln.

Bei den verzweigten C₁-C₄-Alkanoyloxy-C₃-C₅-alkylgruppen kann es sich um eine 1-Alkanoyloxy-1-methylethyl- [(CH₃)₂C(OCOAlk)-], 2-Alkanoyloxy-1-methylethyl- [Alk-COOCH₂CH(CH₃)-], 1-Alkanoyloxy-1-methylpropyl- [CH₃CH₂C(CH₃)(OCOAlk)-], 2-Alkanoyloxy-1-methylpropyl- [CH₃CH(OCOAlk)CH(CH₃)-], 3-Alkanoyloxy-1-methylpropyl- [AlkCOOCH₂CH₂CH(CH₃)-], 1-(Alkanoyloxymethyl)propyl-[CH₃CH₂C(CH₂OCOAlk)-], 1-Alkanoyloxy-2-methylpropyl- [(CH₃)₂CHCH(OCOAlk)-], 2-Alkanoyloxy-2-methylpropyl- [CH₃C(OCOAlk)(CH₃)CH₂-], 3-Alkanoyloxy-2-methylpropyl-[AlkCOOCH₂CH(CH₃)CH₂-], 1-(Alkanoyloxymethyl)butyl- [CH₃CH₂CH₂CH(CH₂OCOAlk)-], 1-Alkanoyloxy-1-methylbutyl- [CH₃CH₂CH₂C(CH₃)(OCOAlk)-], 2-Alkanoyloxy-1-methylbutyl- [CH₃CH₂CH(OCOAlk)CH(CH₃)-], 3-Alkanoyloxy-1-methylbutyl-[CH₃CH(OCOAlk)CH₂CH(CH₃)-], 4-Alkanoyloxy-1-methylbutyl- [Alk-COOCH₂CH₂CH₂CH(CH₃)-], 3-Alkanoyloxy-1-ethylpropyl- [Alk-COOCH₂CH₂CH(CH₂CH₃)-], 2-Alkanoyloxy-1-ethylpropyl-[CH₃CH(OCOAlk)CH(CH₂CH₃)-], 1-Alkanoyloxy-1-ethylpropyl-[CH₃CH₂C(CH₂CH₃)(OCOAlk)-], 1-Alkanoyloxy-3-methylbutyl-[CH₃CH(CH₃)CH₂CH(OCOAlk)-], 2-Alkanoyloxy-3-methylbutyl-[(CH₃)₂CHCH(OCOAlk)CH₂-], 3-Alkanoyloxy-3-methylbutyl-[CH₃C(CH₃)(OCOAlk)CH₂CH₂-], 4-Alkanoyloxy-3-methylbutyl- [Alk-COOCH₂CH(CH₃)CH₂CH₂-], 1-(Alkanoyloxymethyl)-1-methylpropyl-[CH₃CH₂C(CH₃)(CH₂OCOAlk)-], 2-Alkanoyloxy-1,1-dimethylpropyl-[CH₃CH(OCOAlk)C(CH₃)₂-], 3-Alkanoyloxy-1,1-dimethylpropyl- [Alk-COOCH₂CH₂C(CH₃)₂-], 1-(Alkanoyloxymethyl)-2-methylpropyl-[(CH₃)₂CHCH(CH₂OCOAlk)-], 1-Alkanoyloxy-1,2-dimethylpropyl-[(CH₃)₂CHC(OCOAlk)(CH₃)-], 2-Alkanoyloxy-1,2-dimethylpropyl-[(CH₃)₂C(OCOAlk)CH(CH₃)-], 3-Alkanoyloxy-1,2-dimethylpropyl- [Alk-COOCH₂CH(CH₃)CH(CH₃)-], 1-Alkanoyloxy-2,2-dimethylpropyl- [(CH₃)₃CCH(OCOAlk)-], 3-Alkanoyloxy-2,2-dimethylpropyl- [AlkCOOCH₂C(CH₃)₂CH₂-], 1-Alkanoyloxy-2-methylbutyl- [CH₃CH₂CH(CH₃)CH(OCOAlk)-], 2-Alkanoyloxy-2-methylbutyl-[CH₃CH₂C(CH₃)(OCOAlk)CH₂-], 3-Alkanoyloxy-2-methylbutyl-[CH₃CH(OCOAlk)CH(CH₃)CH₂-], 4-Alkanoyloxy-2-methylbutyl- [Alk-COOCH₂CH₂CH(CH₃)CH₂-] oder 3-Alkanoyloxy-2-ethylpropyl- [Alk-COOCH₂CH(CH₂CH₃)CH₂-]-Gruppe handeln.

Bei der Abgangsgruppe X kann es sich um ein Halogen oder eine Sulfonestergruppe handeln.
Beim Halogen kann es sich um Chlor, Brom oder lod handeln, wobei lod bevorzugt ist. Bei der Sulfonestergruppe kann es sich beispielsweise um eine Mesylat-, Benzolsulfonat-, Tosylat-, Brosylat-, Triflat- oder Nonaflat-Gruppe handeln.

Bei der organischen Base handelt es sich um tertiäre Amin- oder Amidbasen, wie beispielsweise Triethylamin oder Ethyldiisopropylamin.

Für die Bildung von pharmazeutisch verträglichen Salzen der erfindungsgemäßen Verbindungen der allgemeinen Formel I. kommen, nach den dem Fachmann bekannten Methoden, als anorganische Säuren unter anderem Chlorwasserstoffsäure, Bromwasserstoffsäure, Schwefelsäure und Phosphorsäure, Salpetersäure, als Carbonsäuren unter anderem Essigsäure, Propionsäure, Hexansäure, Octansäure, Decansäure, Oleinsäure, Stearinsäure, Maleinsäure, Fumarsäure, Bernsteinsäure, Benzoesäure, Ascorbinsäure, Oxalsäure, Salicylsäure, Weinsäure, Zitronensäure, Milchsäure, Glykolsäure, Äpfelsäure, Mandelsäure, Zimtsäure, Glutaminsäure, Asparaginsäure, als Sulfonsäuren unter anderem Methansulfonsäure, Ethansulfonsäure, Toluolsulfonsäure, Benzolsulfonsäure sowie Naphthalinsulfonsäure in Betracht.

Die nachstehend unter dem Kapitel "Herstellungsverfahren" genannten Beispiele 1 bis 58 der erfindungsgemäßen Verbindungen sind besonders bevorzugt.

### Pharmakologische Untersuchungen

Die erfindungsgemäßen Verbindungen wurden in verschiedenen Modellen getestet.
Die erfindungsgemäßen Verbindungen der allgemeinen Formel I zeichnen sich dadurch aus, dass es sich dabei um Verbindungen mit antiandrogener Wirkung handelt, die das Prostatatumorwachstum hemmen, gleichzeitig eine hohe, ggf. orale Bioverfügbarkeit aufweisen, und ggf. den Androgenrezeptor destabilisieren.
Die *in vitro* Tests zu den Einflüssen auf die Aktivitäten des Androgenrezeptors wurden folgendermaßen durchgeführt:

In den hier aufgeführten Schemata wurden die folgenden Abkürzungen benutzt:

| | |
|---|---|
| Bicalutamid: | *N*-[4-Cyan-3-(trifluormethyl)phenyl]-3-[(4-fluorphenyl)sulfonyl]-2-hydroxy-2-methylpropanamid |
| R1881: | Methyltrienolon, 17β-Hydroxy-17α-methylestra-4,9,11-trien-3-on |
| CPA: | Cyproteronacetat, 17-(Acetyloxy)-6-chlor-1β,2β-dihydro-3'*H*-cyclopropa[1,2]pregna-1,4,6-triene-3,20-dion |

### Modell 1: Inhibition der Proliferation von LNCaP-Zellen

Für den Proliferationsassay werden 6000 LNCaP-Zellen/well (Horoszewicz et al. Cancer Res. 1983, 43, 1809-1817) in einer Microtiterplatte (96-well) in 50 µl RPMI1640-Medium mit 5% CCS ausgesät und wie in Modell 1 kultiviert. Nach 24 Stunden erhalten die Zellen 50 µl zweifach konzentrierte in Kulturmedium verdünnte Testsubstanz. Die Lösungsmittelkonzentration beträgt 0,5% DMSO. Nach 4 Tagen erhalten die Zellen weitere 100 µl einfach-konzentrierte, in Kulturmedium verdünnte Testsubstanz. Nach 7 bis 8 Tagen wird die Proliferationsrate der Zellen mittels Kristallviolettassay bestimmt (Gillies et al. Anal. Biochem. 1986, 159, 109-113). Für die Bestimmung des Antagonismus wird die Substanzbehandlung in Gegenwart von 0,1 nM R1881 durchgeführt (1 : 1000 Verdünnung aus ethanolischer Lösung). Kontrollzellen erhalten nur 0,5% DMSO. Für den Agonismus werden die Zellen nur mit Testsubstanz (ohne R1881) behandelt.

Tabelle 1 zeigt die inhibitorische Wirkung von Testsubstanzen auf die Proliferation der humanen androgenabhängigen Prostatazelllinie LNCaP. Die Inhibition der Zellproliferation ist eine wichtige Vorraussetzung für den therapeutischen Einsatz der Substanzen bei der Behandlung des Prostatakarzinoms. Die ausgewählten erfindungsgemäßen Testsubstanzen inhibieren die Zellproliferation in Gegenwart von 0,1 nM des synthetischen Androgens R1881 mit einer deutlich niedrigeren IC₅₀ (ca. 50 x 10⁻⁹ M), wie das zugelassene nichtsteroidale Antiandrogen Bicalutamid (380 x 10⁻⁹ M). Bei einer Substanzkonzentration von 1 µM ist die Proliferation verglichen mit dem Zellwachstum in Gegenwart von 0,1 nM R1881 um mindestens 80% reduziert. Bis zu einer getesteten Konzentration von 10 µM wurde bei keiner der Testsubstanzen eine proliferationsstimulierende Wirkung beobachtet.
Es wurde überraschend gefunden, dass das Ausmaß der proliferationshemmenden Wirkung von *N*-[ω-[3-[4-Cyan-3-(trifluormethyl)phenyl]-5,5-dimethyl-4-oxo-2-thioxoimidazolidin-1-yl]alkyl]-Derivaten gleichzeitig abhängig vom Kettensubstituenten und der Kettenlänge ist. Insbesondere für die hier beanspruchten Pyrrolidin-substituierten Verbindungen wird die stärkste antiproliferative Wirkung bei Kettenlängen zwischen C₆ und C₉ beobachtet. Die analogen Verbindungen mit den kürzeren Kettenlängen n = 4 (Vergleich 1) und n = 5 (Vergleich 2) zeigen eine deutlich reduzierte antiproliferative Aktivität. Für die getesteten Beispiele aus US Re. 35956 [Examples 71 und 77] der Kettenlänge n = 2 und 4 konnte keine bzw. nur marginale antiproliferative Wirkung nachgewiesen werden.

**Tab. 1. Inhibition der Proliferation von LNCaP-Zellen durch Testsubstanzen.**

| **Beispiel** | **Testsubstanz** | **IC50 [10⁻⁹ M]** | **% Inhibition bei 1 µM** |
|---|---|---|---|
| **Bicalutamid** | *N*-[4-Cyan-3-(trifluormethyl)phenyl]-3-[(4-fluorphenyl)sulfonyl]-2-hydroxy-2-methylpropanamid | **380** | **85** |
| **Vergleich 1** | 4-[4,4-Dimethyl-5-oxo-3-[4-(pyrrolidin-1-yl)butyl]-2-thioxoimidazolidin-1-yl]-2-(trifluormethyl)benzonitril | **4300** | **20** |
| **Vergleich 2** | 4-[4,4-Dimethyl-5-oxo-3-[5-(pyrrolidin-1-yl)pentyl]-2-thioxoimidazolidin-1-yl]-2-(trifluormethyl)benzonitril | **2100** | **38** |
| **Example 71 USRe 35956** | 4-[3-(2-Hydroxyethyl)-4,4-dimethyl-5-oxo-2-thioxoimidazolidin-1-yl]-2-(trifluormethyl)benzonitril | **> 10000** | **9** |
| **Example 77 USRe 35956** | 4-[3-(4-Hydroxybutyl)-4,4-dimethyl-5-oxo-2-thioxoimidazolidin-1-yl]-2-(trifluormethyl)benzonitril | **> 10000** | **34** |
| **1** | 4-[4,4-Dimethyl-5-oxo-3-[8-(pyrrolidin-1-yl)octyl]-2-thioxoimidazolidin-1-yl]-2-(trifluormethyl)benzonitril | **45** | **98** |
| **2** | 4-[4,4-Dimethyl-5-oxo-3-[8-(pyrrolidin-1-yl)octyl]-2-thioxoimidazolidin-1-yl]-2-(trifluormethyl)benzonitril hydrochlorid | **41** | **100** |
| **4** | 4-[4,4-Dimethyl-5-oxo-3-[7-(pyrrolidin-1-yl)heptyl]-2-thioxoimidazolidin-1-yl]-2-(trifluormethyl)benzonitril | **45** | **98** |
| **6** | 4-[3-[6-[(2R)-2-(Hydroxymethyl)pyrrolidin-1-yl]hexyl]-4,4-dimethyl-5-oxo-2-thioxoimidazolidin-1-yl]-2-(trifluormethyl)benzonitril | **55** | **102** |
| **8** | 4-[3-[7-[(2*R*)-2-(Hydroxymethyl)pyrrolidin-1-yl]heptyl]-4,4-dimethyl-5-oxo-2-thioxoimidazolidin-1-yl]-2-(trifluormethyl)benzonitril | **11** | **101** |
| **9** | 4-[3-[7-[(2*S*)-2-(Hydroxymethyl)pyrrolidin-1-yl]heptyl]-4,4-dimethyl-5-oxo-2-thioxoimidazolidin-1-yl]-2-(trifluormethyl)benzonitril | **26** | **92** |
| **10** | 4-[3-[8-[(2*R*)-2-(Hydroxymethyl)pyrrolidin-1-yl]octyl]-4,4-dimethyl-5-oxo-2-thioxoimidazolidin-1-yl]-2- (trifluormethyl)benzonitril | **< 10** | **101** |
| **11** | 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril | **13** | **102** |
| **12** | 4-[3-[9-[(2*R*)-2-(Hydroxymethyl)pyrrolidin-1-yl]nonyl]-4,4-dimethyl-5-oxo-2-thioxoimidazolidin-1-yl]-2-(trifluormethyl)benzonitril | **24** | **93** |
| **13** | 4-[3-[9-[(2*S*)-2-(Hydroxymethyl)pyrrolidin-1-yl]nonyl]-4,4-dimethyl-5-oxo-2-thioxoimidazolidin-1-yl]-2-(trifluormethyl)benzonitril | **16** | **88** |
| **16** | 4-[3-[7-[(2*R*)-2-(Methoxymethyl)pyrrolidin-1 -yl]heptyl]-4,4-dimethyl-5-oxo-2-thioxoimidazolidin-1-yl]-2-(trifluormethyl)benzonitril | **18** | **98** |
| **17** | 4-[3-[7-[(2*S*)-2-(Methoxymethyl)pyrrolidin-1-yl]heptyl]-4,4-dimethyl-5-oxo-2-thioxoimidazolidin-1-yl]-2-(trifluormethyl)benzonitril | **32** | **99** |
| **18** | 4-[3-[8-[(2*R*)-2-(Methoxymethyl)pyrrolidin-1-yl]octyl]-4,4-dimethyl-5-oxo-2-thioxoimidazolidin-1-yl]-2-(trifluormethyl)benzonitril | **11** | **103** |
| **19** | 4-[3-[8-[(2*S*)-2-(Methoxymethyl)pyrrolidin-1-yl]octyl]-4,4-dimethyl-5-oxo-2-thioxoimidazolidin-1yl]-2-(trifluormethyl)benzonitril | **25** | **97** |
| **20** | 4-[3-[9-[(2*R*)-2-(Methoxymethyl)pyrrolidin-1-yl]nonyl]-4,4-dimethyl-5-oxo-2-thioxoimidazolidin-1-yl]-2-(trifluormethyl)benzonitril | **29** | **101** |
| **21** | 4-[3-[9-[(2*S*)-2-(Methoxymethyl)pyrrolidin-1-yl]nonyl]-4,4-dimethyl-5-oxo-2-thioxoimidazolidin-1-yl]-2-(trifluormethyl)benzonitril | **47** | **94** |
| **23** | 4-[3-[7-(3-Hydroxy-8-azabicyclo[3.2.1]oct-8-yl)heptyl]-4,4-dimethyl-5-oxo-2-thioxoimidazolidin-1-yl]-2-(trifluormethyl)benzonitril | **48** | **79** |
| **24** | 4-[3-[8-(3-Hydroxy-8-azabicyclo[3.2.1]oct-8-yl)octyl]-4,4-dimethyl-5-oxo-2-thioxoimidazolidin-1-yl]-2-(trifluormethyl)benzonitril | **13** | **102** |
| **27** | 4-[3-[7-[(*R*)-3-Hydroxypyrrolidin-1-yl]heptyl]-4,4-dimethyl-5-oxo-2-thioxoimidazolidin-1-yl]-2-(trifluormethyl)benzonitril | **57** | **95** |
| **28** | 4-[3-[8-[(*R*)-3-Hydroxypyrrolidin-1-yl]octyl]-4,4-dimethyl-5-oxo-2-thioxoimidazolidin-1-yl]-2-(trifluormethyl)benzonitril | **11** | **102** |
| **29** | 4-[3-[9-[(*R*)-3-Hydroxypyrrolidin-1-yl]nonyl]-4,4-dimethyl-5-oxo-2-thioxoimidazolidin-1-yl]-2-(trifluormethyl)benzonitril | **35** | **99** |
| **31** | 4-[3-[7-[(*S*)-3-Hydroxypyrrolidin-1-yl]heptyl]-4,4-dimethyl-5-oxo-2-thioxoimidazolidin-1-yl]-2-(trifluormethyl)benzonitril | **20** | **104** |
| **32** | 4-[3-[8-[(*S*)-3-Hydroxypyrrolidin-1-yl]octyl]-4,4-dimethyl-5-oxo-2-thioxoimidazolidin-1-yl]-2-(trifluormethyl)benzonitril | **12** | **101** |
| **35** | 4-[4,4-Dimethyl-3-[7-(2-methylpyrrolidin-1-yl)heptyl]-5-oxo-2-thioxoimidazolidin-1-yl]-2-(trifluormethyl)benzonitril | **30** | **92** |
| **36** | 4-[4,4-Dimethyl-3-[8-(2-methylpyrrolidin-1-yl)octyl]-5-oxo-2-thioxoimidazolidin-1-yl]-2-(trifluormethyl)benzonitril | **15** | **96** |
| **37** | 4-[3-[8-[(2R,5S)-rel-2,5-Dimethylpyrrolidin-1-yl]octyl]-4,4-dimethyl-5-oxo-2-thioxoimidazolidin-1-yl]-2-(trifluormethyl)benzonitril | **11** | **99** |
| **39** | 4-[3-[7-[(2*S*)-2-(1-Hydroxy-1-methylethyl)pyrrolidin-1-yl]heptyl]-4,4-dimethyl-5-oxo-2-thioxoimidazolidin-1-yl]-2-(trifluormethyl)benzonitril | **41** | **108** |
| **40** | 4-[3-[8-[(2*S)*-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril | **11** | **118** |
| **41** | 4-[3-[9-[(2*S*)-2-(1-Hydroxy-1-methylethyl)pyrrolidin-1-yl]nonyl]-4,4-dimethyl-5-oxo-2-thioxoimidazolidin-1-yl]-2-(trifluormethyl)benzonitril | **48** | **89** |
| **43** | 4-[3-[7-[(2*R*)-2-(1-Hydroxy-1-methylethyl)pyrrolidin-1-yl]heptyl]₋4,4-dimethyl-5-oxo-2-thioxoimidazolidin-1-yl]-2-(trifluormethyl)benzonitril | **26** | **113** |
| **44** | 4-[3-[8-[(2*R*)-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril | **11** | **115** |
| **47** | 4-[3-[8-[(2*S*)-2-(1-Hydroxy-1-ethylpropyl)pyrrolidin-1-yl]octyl]-4,4-dimethyl-5-oxo-2-thioxoimidazolidin-1-yl]-2-(trifluormethyl)benzonitril | **27** | **99** |
| **49** | (*S*)-1-[6-[3-[4-Cyan-3-(trifluormethyl)phenyl]-5,5-dimethyl-4-oxo-2-thioxoimidazolidin-1-yl]hexyl]pyrrolidin-4-carboxamid | **32** | **110** |
| **50** | (*S*)-1-[7-[3-[4-Cyan-3-(trifluormethyl)phenyl]-5,5-dimethyl-4-oxo-2-thioxoimidazolidin-1-yl]heptyl]pyrrolidin-4-carboxamid | **15** | **110** |
| **51** | (*S*)-1-[8-[3-[4-Cyan-3-(trifluormethyl)phenyl]-5,5-dimethyl-4-oxo-2-thioxoimidazolidin-1-yl]octyl]pyrrolidin-4-carboxamid | **20** | **97** |
| **52** | (*R*)-1-[6-[3-[4-Cyan-3-(trifluormethyl)phenyl]-5,5-dimethyl-4-oxo-2-thioxoimidazolidin-1-yl]hexyl]pyrrolidin-4-carboxamid | **12** | **100** |
| **53** | (*R*)-1-[7-[3-[4-Cyan-3-(trifluormethyl)phenyl]-5,5-dimethyl-4-oxo-2-thioxoimidazolidin-1-yl]heptyl]pyrrolidin-4-carboxamid | **49** | **97** |
| **54** | (*R*)-1-[8-[3-[4-Cyan-3-(trifluormethyl)phenyl]-5,5-dimethyl-4-oxo-2-thioxoimidazolidin-1-yl]octyl]pyrrolidin-4-carboxamid | **64** | **93** |

### Modell 2: Antiandrogene Wirkung selektiver Testsubstanzen auf das Wachstum der akzessorischen Geschlechtsdrüsen der Maus

Die Funktion und die Größe der akzessorischen Geschlechtsdrüsen (Prostata und Samenblase) sind abhängig von Androgenen. Bei kastrierten Tieren wird durch Androgenapplikation ein Wachstum dieser Organe induziert. Die gleichzeitige Behandlung mit Antiandrogenen hemmt dieses Wachstum dosisabhängig.
Für die Prüfung der Testsubstanzen wurden die Mäuse kastriert. Am gleichen Tag wurde die Behandlung mit Testosteronpropionat (0,03 mg/Maus) und den Testsubstanzen (1 x täglich 10 oder 30 mg/kg p.o. in Benzylbenzoat-Rizinusöl oder Ethanol /Erdnußöl (10:90) formuliert). Die Behandlung erfolgte über 7 Tage und zum Versuchsabschluss wurden die Gewichte von Samenblase und Prostata bestimmt. Die prozentuale Hemmung des Samenblasenwachstums wurde in Bezug auf die Kontrollgruppen (mit und ohne Testosteron) berechnet. Als Referenzsubstanz wurde Cyproteronacetat (30 mg/kg s.c. und p.o.) eingesetzt.
Die Ergebnisse sind in der Tabelle 2 dargestellt.
Die getesteten erfindungsgemäßen Verbindungen zeigen eine mindestens so gute antiandrogene Wirkung an der Samenblase der Maus wie die Vergleichssubstanzen CPA und Bicalutamid.

**Tab. 2. Wirkung selektiver Testsubstanzen auf das Testosteron stimulierte Wachstum der Samenblase.**

| **Beispiel** | **Testsubstanz** | **% Inhibition des MSB Wachstums** | **Dosis [mg/kg]** |
|---|---|---|---|
| **1** | 4-[4,4-Dimethyl-5-oxo-3-[8-(pyrrolidin-1-yl)octyl]-2-thioxoimidazolidin-1-yl]-2-(trifluormethyl)benzonitril | **99** **82** | **30 p.o.** **10 p.o.** |
| **2** | 4-[4,4-Dimethyl-5-oxo-3-[8-(pyrrolidin-1-yl)octyl]-2-thioxoimidazolidin-1-yl]-2-(trifluormethyl)benzonitril hydrochlorid | **95** | **30 p.o.** |
| **10** | 4-[3-[8-[(2*R*)-2-(Hydroxymethyl)pyrrolidin-1-yl]octyl]-4,4-dimethyl-5-oxo-2-thioxoimidazolidin-1-yl]-2-(trifluormethyl)benzonitril | **87** | **10 p.o.** |
| **11** | 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril | **95** | **30 p.o.** |
| **CPA** | 17-(Acetyloxy)-6-chlor-1β,2β-dihydro-3'H-cyclopropa[1,2]pregna-1,4,6-triene-3,20-dion | **85** | **30 s.c.** |
| **Bicalutamid** | *N*-[4-Cyan-3-(trifluormethyl)phenyl]-3-[(4-fluorphenyl)sulfonyl]-2-hydroxy-2-methylpropanamid | **86** **82** | **30 s.c.** **30 p.o.** |

### Modell 3: Antiandrogene Wirkung einer selektiven Testsubstanz auf das Wachstum von humanen Prostatakarzinomxenografts in vivo

In der vorliegenden Erfindung wurde die Wirkung des erfindungsgemäßen Beispiele **1**: 4-[4,4-Dimethyl-5-oxo-3-[8-(pyrrolidin-1-yl)octyl]-2-thioxoimidazolidin-1-yl]-2-(trifluormethyl)benzonitril, **11**: 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril und **23**: 4-[3-[7-(3-Hydroxy-8-azabicyclo[3.2.1]oct-8-yl)heptyl]-4,4-dimethyl-5-oxo-2-thioxoimidazolidin-1-yl]-2-(trifluormethyl)benzonitril auf das Tumorwachstum in vivo mittels Maus-Xenograft-Modellen untersucht, bei denen die erfindungsgemäßen Verbindungen täglich 1 x verabreicht wurden.

Das CWR22 Tumormodell [M. A. Wainstein, F. He, D. Robinson, H. J. Kung, S. Schwartz, J. M. Giaconia, N. L. Edgehouse, T. P. Pretlow, D. R. Bodner, E. D. Kursh, Cancer Res. 1994, 1; 54(23), 6049-52] ist ein hormonabhängiges humanes Prostatakarzinommodell. Das Tumormodell wurde durch "serial passaging" von Prostatakarzinomgewebe, das während einer OP entnommen wurde, auf immundefizienten Nacktmäusen etabliert und weiter propagiert. Das androgenabhängige LNCaP Prostatakarzinommodell wurde ebenfalls von einem Patiententumor etabliert. Dieses Tumormodell wächst sowohl in Zellkultur als auch als Xenotransplantat auf immundefizienten Mäusen (Culig, Hoffmann Brit. J. Cancer, 1999, 242-251). Für Therapieversuche wurden 6 Wochen alte männliche Nacktmäuse, (NMRI-Maus, M&B, Bomholdtgard, Denmark) mit Testosteron-Pellets (12,5 mg, 90 Tage Freisetzung; IRA, Sarasota, FL) supplementiert. Den Tieren wurden entweder LNCaP Zellen (1.5 x 10⁶ Zellen) oder kleine CWR22 Tumorfragmente (2 x 2 mm) subkutan in die linke Flanke implantiert. Nachdem die Tumoren eine Größe von 20-25 mm² erreicht hatten, wurde die Behandlung mit der Erfindungssubstanz begonnen. [M. A. Wainstein, F. He, D. Robinson, H. J. Kung, S. Schwartz, J. M. Giaconia, N. L. Edgehouse, T. P. Pretlow, D. R. Bodner, E. D. Kursh, Cancer Res. 1994, 1; 54(23), 6049-52].
Die Ergebnisse sind in den Diagrammen 1-4 dargestellt.

In der vorliegenden Erfindung wurde die Wirkung von den erfindungsgemäßen Verbindungen auf das Tumorwachstum *in vivo* mittels zweier verschiedener Maus-Xenograft-Modelle untersucht, bei dem die erfindungsgemäßen Verbindungen 1 x täglich über den gesamten Behandlungszeitraum oral verabreicht wurden. Im Vergleich zu den unbehandelten Kontrolltieren resultierte eine Hemmung des Tumorwachstums. Retardation des Tumorwachstums zeigte sich als signifikant in kastrierten Mäusen. Die Behandlung wurde gut vertragen.

In beiden Modellen (CWR22; LNCaP) ist die Hemmung des Tumorwachstums durch die erfindungsgemäßen Verbindungen der Behandlung mit dem Antiandrogen Bicalutamid überlegen.

### Modell 4: Pharmakokinetik in Ratten nach intravenöser und peroraler Gabe

Die pharmakokinetischen Eigenschaften der erfindungsgemäßen Verbindungen wurden an den Beispielen **1**: 4-[4,4-Dimethyl-5-oxo-3-[8-(pyrrolidin-1-yl)octyl]-2-thioxoimidazolidin-1-yl]-2-(trifluormethyl)benzonitril und **11**: 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril untersucht.

**Tab. 3. Pharmakokinetische Eigenschaften ausgewählter Beispiele**

| **Ermittelter Parameter** | **Beispiel 1** | **Beispiel 11** |
|---|---|---|
| **Verteilungsvolumen V_{dss} [l/kg]** | 37 | 11 |
| **Systemische Serum Clearance CL [ml/min/kg]** | 76 | 20 |
| **Halbwertzeit i.v. T_{½} [h]** | 5,6 | 5,7 |
| **Halbwertzeit p.o. T_{½} [h]** | 6,1 | 8,0 |
| **Absorption Tₘₐₓ [h]** | 5 | 7 |
| **Orale Bioverfügbarkeit [%]** | 72 | 65 |

Die ermittelten pharmakokinetischen Daten zeigen sehr vorteilhafte pharmakokinetischen Eigenschaften der erfindungsgemäßen Verbindungen in der Ratte, die zu einer hohen oralen Bioverfügbarkeit und einer langen Halbwertzeit (>5h) führen. Die Daten deuten auf eine hohe intestinale Absorption und einen relativ geringen Leber-first-pass-Effekt (erhöhte metabolische Stabilität) hin.

### Modell 5: Destabilisierung des AR in LNCaP-Zellen durch Testsubstanzen

In einer 25 cm² Zellkulturflasche werden 2x10⁶ LNCaP-Zellen in 6 ml RPMI 1640 ohne Phenolrot mit 4 mM Glutamin und 5% Aktivkohle-behandeltem Serum (CCS) ausgesät und über Nacht bei 37°C, 5% CO₂ in feuchter Atmosphäre kultiviert. Am nächsten Tag werden die Zellen mit der Testsubstanz bei einer Konzentration von 10 bzw. 1 µM behandelt, wobei die Endkonzentration des Lösungsmittels 0,5% DMSO beträgt. Als Kontrolle werden Zellen nur mit 0,5% DMSO behandelt. Nach einer Inkubationszeit von 24 Stunden erfolgt ein Mediumwechsel mit erneuter Substanzzugabe und weiteren 24 Stunden Inkubation. Nach 48 Stunden werden die Zellen mit PBS gewaschen, mit PBS/20mM EDTA abgelöst, nochmals mit PBS-Ca²⁺/Mg²⁺ gewaschen und anschließend für mindestens 2 Stunden als Zellpellet bei -80°C eingefroren. Danach wird das Zellpellet in 200 µl Lysepuffer (50 mM Tris/HCl pH 7,5; 150 mM NaCl, 1,5 mM MgCl₂, 0,2% SDS, 10% Glycerin, 1 mM DTT, 0,01 x Complete-EDTA Proteaseinhibitoren (Roche, Mannheim)) resuspendiert und mit 10U Benzonase (Merck, Darmstadt) für 10 Minuten bei 4°C behandelt. Nach Zugabe von 5 mM EDTA wird unlösliches Material pelletiert und 25 µg des Zellextraktes in einem 4-12% SDS-Polyacrylamidgel (Invitrogen) aufgetrennt. Anschließend werden die Proteine auf Nitrozellulose (HyBondECL, Amersham) transferiert und mit monoklonalen Antikörpern gegen den Androgenrezeptor (AR441; Santa Cruz Biotechnologies; 1 : 400 Verdünnung) und Aktin (ICN, 1:5000 - 1 : 20000 Verdünnung) inkubiert. Nach Inkubation mit dem Sekundärantikörper (anti mouse IgG-HRP, Amersham oder -AP, Invitrogen,) wird der Westernblot mittels Chemilumineszenz (ECL, Amersham; Western Breeze, lnvitrogen) entwickelt und die Lichtsignale werden mit einem Chemilmager^{™} (Kodak) quantifiziert. Die Menge des Androgenrezeptors wird im Verhältnis zu Aktin als Prozent der DMSO-Kontrolle berechnet.

Tabelle 4 zeigt die Wirkung ausgewählter Testsubstanzen bei Konzentrationen von 10 bzw. 1 µM auf den Gehalt an Androgenrezeptorprotein in der humanen Prostatazelllinie LNCaP. Die Angaben entsprechen dem prozentualen Anteil des AR-Gehaltes von Zellen, die nur mit dem Lösungsmittel DMSO behandelt wurden (=Kontrolle). Die Behandlung der Zellen mit den aufgeführten Testsubstanzen führt wie bei Beispiel 56 (4-[3-[7-[(2S)-4,4-Dimethyl-5-oxo-2-(pyrrolidin-1-ylmethyl)pyrrolidin-1-yl]heptyl]-2-thioxoimidazolidin-1-yl]-2-(trifluormethyl)benzonitril) bei einer Behandlungskonzentration von 1 µM zu einer Erniedrigung des AR-Gehaltes auf bis zu ein Viertel der Kontrolle (24%). Die Vergleichssubstanz Bicalutamid beeinflusst den AR-Gehalt nicht, während das synthetische Androgen R1881 das AR-Protein stabilisiert. Letzteres ist aus der Literatur bekannt (J. A. Kemppainen et. al. J. Biol. Chem. 1992, 267, 968-974).
Durch die Erniedrigung des AR-Gehaltes, die vermutlich durch eine Destabilisierung des AR-Proteins erfolgt, soll die inhibitorische Wirkung der Antihormone auf die Zellproliferation noch verstärkt werden.

**Tab. 4. AR-Gehalt (%) in LNCaP-Zellen nach Behandlung mit ausgewählten Testsubstanzen**

| **Beispiel** | **Testsubstanz** | **AR-Gehalt [%]** | |
|---|---|---|---|
| | | **bei 10 µM** | **bei 1 µM** |
| **1** | 4-[4,4-Dimethyl-5-oxo-3-[8-(pyrrolidin-1-yl)octyl]-2-thioxoimidazolidin-1-yl]-2-(trifluormethyl)benzonitril | **63** | |
| **2** | 4-[4,4-Dimethyl-5-oxo-3-[8-(pyrrolidin-1-yl)octyl]-2-thioxoimidazolidin-1-yl]-2-(trifluormethyl)benzonitril hyd-rochlorid | **50** | |
| **9** | 4-[3-[7-[(2*S*)-2-(Hydroxymethyl)pyrrolidin-1-yl]heptyl]-4,4-dimethyl-5-oxo-2-thioxoimidazolidin-1-yl]-2-(trifluormethyl)benzonitril | | **63** |
| **11** | 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril | **50** | |
| **37** | 4-[3-[8-[(2*R*,5*S*)-*rel*-2,5-Dimethylpyrrolidin-1-yl]octyl]-4,4-dimethyl-5-oxo-2-thioxoimidazolidin-1-yl]-2-(trifluormethyl)benzonitril | | **57** |
| **40** | 4-[3-[8-[(2*S*)-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril | | **40** |
| **52** | (*R*)-1-[6-[3-[4-Cyan-3-(trifluormethyl)phenyl]-5,5-dimethyl-4-oxo-2-thioxoimidazolidin-1-yl]hexyl]pyrrolidin-4-carboxamid | **36** | **74** |
| **56** | 4-[3-[7-[(2*S*)-4,4-Dimethyl-5-oxo-2-(pyrrolidin-1-ylmethyl)pyrrolidin-1-yl]heptyl]-2-thioxoimidazolidin-1-yl]-2-(trifluormethyl)benzonitril | | **24** |
| **Vergleich US Re 35956 Example 71** | 4-[3-(2-Hydroxyethyl)-4,4-dimethyl-5-oxo-2-thioxoimidazolidin-1-yl]-2-(trifluormethyl)benzonitril | **191** | **164** |
| **Vergleich US Re. 35956 Example 77** | 4-[3-(4-Hydroxybutyl)-4,4-dimethyl-5-oxo-2-thioxoimidazolidin-1-yl]-2-(trifluormethyl)benzonitril | **180** | **230** |
| **Bicalutamic** | *N*-[4-Cyan-3-(trifluormethyl)phenyl]-3-[(4-fluorphenyl)sulfonyl]-2-hydroxy-2-methylpropanamid | **100** | |
| **R1881 (10nM)** | 17β-Hydroxy-17α-methylestra-4,9,11-trien-3-on | **250** | |

Die erfindungsgemäßen Verbindungen eignen sich für eine verlängerte effektive Behandlung oder Prophylaxe von androgenabhängigen Erkrankungen des menschlichen oder tierischen Körpers. Die erfindungsgemäßen Verbindungen eignen sich besonders für die Behandlung oder Prophylaxe von androgenabhängigen proliferativen Erkrankungen insbesondere von Prostatakarzinomen sowie von benigner Prostatahyperplasie (BHP).

Die vorliegende Erfindung umfasst pharmazeutische Präparate enthaltend eine oder mehrere Verbindungen der allgemeinen Formel I oder deren pharmazeutisch verträgliche Salze, gegebenenfalls zusammen mit pharmazeutisch verträglichen Hilfs- und/oder Trägerstoffen, sowie die Verwendung von Verbindungen der allgemeinen Formel I zur Herstellung eines Arzneimittels zur Behandlung oder Prophylaxe von Krankheiten des menschlichen oder tierischen Körpers, die sich durch die Hemmung des Androgenrezeptors beeinflussen lassen.

In diesem Sinne können die erfindungsgemäßen Verbindungen der allgemeinen Formel I, sowie diese enthaltenden pharmazeutischen Präparate auch zur Prophylaxe und/oder Therapie weiterer androgenabhängiger Krankheitsbilder oder Symptomen verwendet werden, die nicht proliferativer Natur sind (z.B. androgenetische Alopezie, Hirsutismus oder androgenabhängige Akne).

### Dosierung

Im allgemeinen sind zufriedenstellende Resultate zu erwarten, wenn die täglichen Dosen einen Bereich von 5 µg bis 50 mg der erfindungsgemäßen Verbindung pro kg Körpergewicht umfassen. Bei größeren Säugetieren, beispielsweise dem Menschen, liegt eine empfohlene tägliche Dosis im Bereich von 10 µg bis 30 mg pro kg Körpergewicht. Geeignete Dosierungen für die erfindungsgemäßen Verbindungen betragen von 0,005 bis 50 mg pro Tag pro kg Körpergewicht, je nach Alter und Konstitution des Patienten, wobei die notwendige Tagesdosis durch Einmal- oder Mehrfachabgabe appliziert werden kann.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage. Für die parenterale Applikation sind Injektions- und Infusionszubereitungen möglich. Für die intraartikuläre Injektion können entsprechend zubereitete Kristallsuspensionen verwendet werden. Für die intramuskuläre Injektion können wässrige und ölige Injektionslösungen oder Suspensionen und entsprechende Depotpräparationen Verwendung finden. Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden. Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0,01% - 20% betragen um eine ausreichende pharmakologische Wirkung zu erzielen. Die topische Anwendung kann auch mittels eines transdermalen Systems beispielsweise eines Pflasters erfolgen.

Die vorliegende Erfindung umfasst pharmazeutische Zusammensetzungen, die mindestens eine Verbindung der allgemeinen Formel I oder mindestens eines von deren pharmazeutisch verträglichen Salzen enthalten, gegebenenfalls zusammen mit pharmazeutisch verträglichen Hilfs- und/oder Trägerstoffen.

Diese pharmazeutischen Zusammensetzungen und Arzneimittel können zur oralen, rektalen, subkutanen, transdermalen, perkutanen, intravenösen oder intramuskulären Applikation vorgesehen sein. Sie enthalten neben üblichen Träger- und/oder Verdünnungsmitteln mindestens eine Verbindung der allgemeinen Formel I.
Die Arzneimittel gemäß vorliegender Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen aus einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.
Die pharmazeutischen Zusammensetzungen, die mindestens eine der erfindungsgemäßen Verbindungen enthalten, werden bevorzugt oral appliziert.
Es kommen auch parenterale Zubereitungen wie Injektionslösungen in Betracht. Weiterhin seien als Zubereitungen beispielsweise auch Suppositorien genannt.
Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.
Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können. Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten.

Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltenden Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen können zur Therapie und/oder Prophylaxe von androgenabhängigen proliferativen Erkrankungen, wie beispielsweise Prostatakarzinomen oder benigner Prostatahyperplasie mit einem oder mehreren der folgenden Wirkstoffen kombiniert verabreicht werden:
1) Gonadotrophormon (GnRH) Agonisten
2) 5α-Reduktase Hemmer wie Finasterid
3) Zytostatika
4) VEGF-Kinase-Inhibitoren
5) Antigestagene
6) Antiestrogene
7) Antisense Oligonukleotide
8) EGF-Antikörper
9) Estrogene
Es ist auch möglich, bei der Behandlung des Prostatakarzinoms mit den erfindungsgemäßen Verbindungen, deren Anwendung mit einer an sich bekannten Methode der klinischen Radiologie zu kombinieren. (Laverdiere J. et al., 1997, Intl. J. of Rad. Onc. Biol. Phys., 37, 247-252; Bolla M. et al., 1997, New Engl. J. Med., 337, 95-300.)

Die erfindungsgemäßen Verbindungen der allgemeinen Formel I lassen sich wie nachstehend beschrieben herstellen.
Die vorliegende Erfindung wird anhand der nachfolgenden Beispiele näher erläutert, ohne sie darauf einzuschränken.

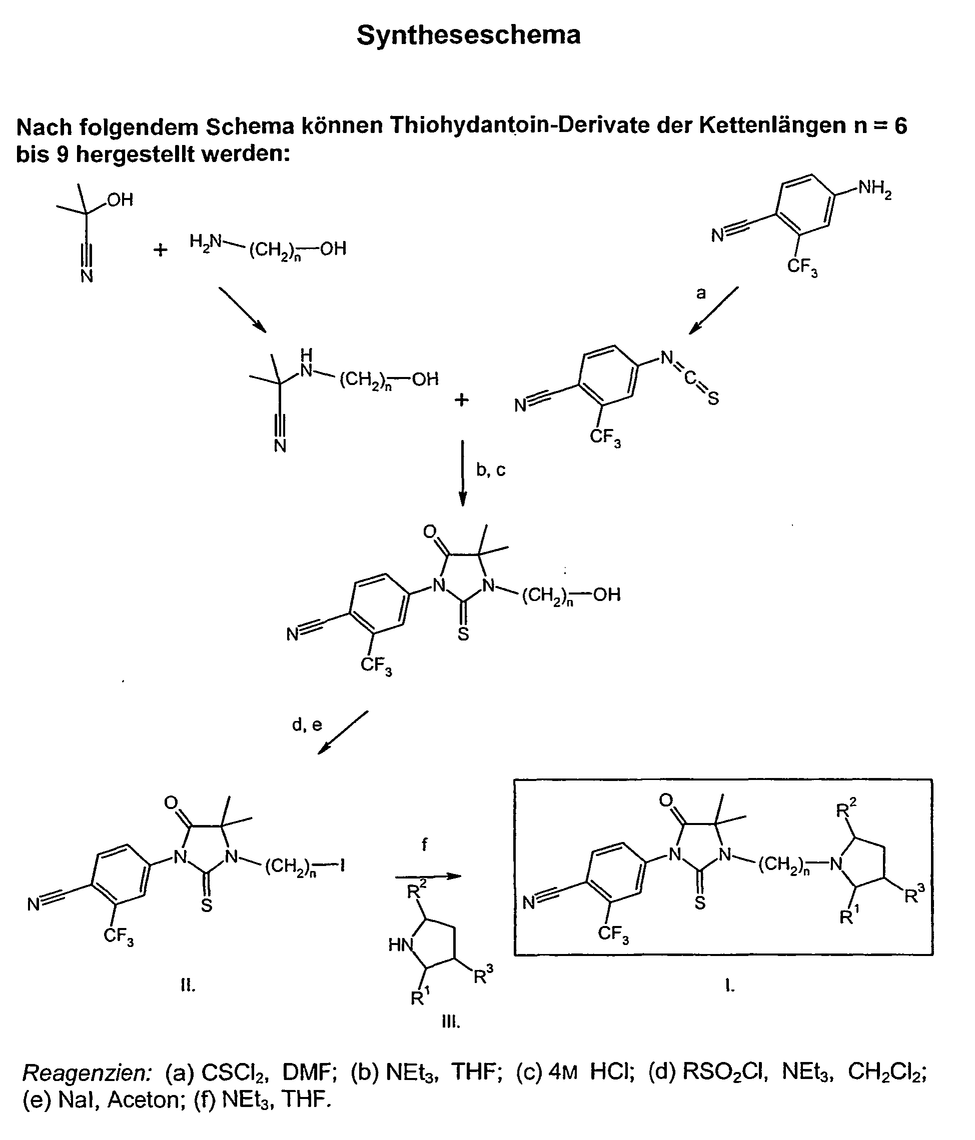

### Herstellungsverfahren

### Beispiel 1

### 4-[4,4-Dimethyl-5-oxo-3-[8-(pyrrolidin-1-yl)octyl]-2-thioxoimidazolidin-1-yl]-2-(trifluormethyl)benzonitril

### 1a) 8-Bromoctan-1-ol

25 g Octan-1,8-diol wurden in 250 ml Cyclohexan mit 22,6 ml 47%iger wässriger Bromwasserstoffsäure sechs Stunden am Wasserabscheider zum Sieden erhitzt. Das Reaktionsgemisch wurde dann auf gesättigte wässrige Natriumhydrogencarbonatlösung gegossen und mit Ethylacetat extrahiert. Die organische Phase wurde mit gesättigter wässriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergab 21,6 g der Titelverbindung als gelbliches Öl.
¹H-NMR (300 MHz, CDCl₃): δ [ppm] = 3,64 t (*J*=6,8 Hz, 2H, CH₂OH) 3,41 t (*J*=6,8 Hz, 2H, CH₂Br); 1,85 tt (*J*=7,3 Hz / 6,8 Hz, 2H, CH₂); 1,56 m (2H, CH₂); 1,49-1,27 m (8H, CH₂).

### 1b) 2-(8-Hydroxyoctyl)-1 H-isoindol-1,3(2H)-dion

Eine Lösung von 15,12 g Phthalsäureimid in 480 ml *N,N*-Dimethylformamid wurde bei Raumtemperatur portionsweise mit 5,04 g 50%igem Natriumhydrid als Dispersion in Mineralöl versetzt. Das Reaktionsgemisch wurde eine Stunde bei Raumtemperatur gerührt. Anschließend wurde eine Lösung von 20 g der unter 1a) hergestellten Verbindung in 480 ml *N,N*-Dimethylformamid hinzugetropft und das Reaktionsgemisch wurde drei Stunden bei Raumtemperatur gerührt. Die Mischung wurde dann auf gesättigte wässrige Natriumhydrogencarbonatlösung gegossen und mit Ethylacetat extrahiert. Die organische Phase wurde mit gesättigter wässriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergab 23,0 g der Titelverbindung als farbloser Schaum.
¹H-NMR (300 MHz, CDCl₃): δ [ppm] = 7,84 m (2H, aryl); 7,71 m (2H, aryl); 3,68 t (*J*=7 Hz, 2H, CH₂N); 3,62 t (*J*=6 Hz, 2H, CH₂OH) 1,67 m (2H, CH₂); 1,56 m (2H, CH₂); 1,32 m (8H, CH₂).

### 1c) 8-Aminooctan-1-ol

Zu einer Lösung von 23 g der unter 1b) hergestellten Verbindung in 400 ml Ethanol wurden 15,2 ml 80%iges wässriges Hydraziniumhydroxid getropft. Das Reaktionsgemisch wurde vier Stunden zum Sieden erhitzt. Der weiße Niederschlag wurde abfiltriert und mit Ethanol nachgewaschen. Das Filtrat wurde im Vakuum eingeengt. Der Rückstand wurde in Ethylacetat aufgenommen und 30 Minuten im Ultraschallbad beschallt. Der weiße Niederschlag wurde wiederum abfiltriert und mit Ethylacetat nachgewaschen. Das Filtrat wurde im Vakuum eingeengt. Man erhielt 8,88 g der Titelverbindung.
¹H-NMR (300 MHz, CDCl₃): δ [ppm] = 3,62 t (*J*=7 Hz, 2H, CH₂OH) 2,68 t (*J*=6 Hz, 2H, CH₂NH₂); 1,56 m (2H, CH₂); 1,32 m (10H, CH₂).

### 1d) 4-[4,4-Dimethyl-5-oxo-3-(8-hydroxyoctyl)-2-thioxoimidazolidin-1-yl]-2-(trifluormethyl)benzonitril

Unter Eisbadkühlung und Stickstoffatmosphäre wurden 2,3 ml Thiophosgen zu einer Lösung von 4,99 g 4-Amino-2-(trifluormethyl)benzonitril in 30 ml *N,N*-Dimethylformamid getropft. Das Reaktionsgemisch wurde eine Stunde bei Raumtemperatur gerührt und anschließend mit Wasser versetzt. Die wässrige Phase wurde mit Ethylacetat extrahiert. Die vereinigten organischen Phasen wurden mit gesättigter wässriger Natriumchloridlösung gewaschen, über Natriumsulfat getrocknet, filtriert und im Vakuum eingeengt. Das so erhaltene rohe Isothiocyanat wurde mit dem durch zweistündiges Rühren von 5,4 ml Acetoncyanhydrin mit 4,29 g der unter 1c) hergestellten Verbindung in Gegenwart von 3 g Molsieb 3 A bei Raumtemperatur erzeugten Cyanamin vereinigt und mit 7,47 ml Triethylamin in 134 ml Tetrahydrofuran eine Stunde zum Sieden erhitzt. Das nach Einengen im Vakuum erhaltene rohe Iminothiohydantoin wurde mit 26,8 ml 4 molarer wässriger Salzsäure in 134 ml Methanol über Nacht bei Raumtemperatur gerührt. Das Reaktionsgemisch wurde dann auf gesättigte wässrige Natriumhydrogencarbonatlösung gegossen und mit Ethylacetat extrahiert. Die organische Phase wurde mit gesättigter wässriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergab 8,1 g der Titelverbindung als farblosen Schaum.
¹H-NMR (300 MHz, CDCl₃): δ [ppm] = 7,94 d (*J*=8 Hz, 1H, aryl); 7,89 d (*J*=2,1 Hz, 1H, aryl); 7,77 dd (*J*=8 Hz / 2,1 Hz, 1H, aryl); 3,67 m (2H, CH₂N); 3,65 t (*J*=6,5 Hz, 2H, CH₂OH) 1,83 m (2H, CH₂); 1,55 m (2H, CH₂); 1,58 s (6H, CH₃); 1,37 m (8H, CH₂).

### 1e) 8-[3-[4-Cyan-3-(trifluormethyl)phenyl]-5,5-dimethyl-4-oxo-2-thioxoimidazolidin-1-yl]octyl 4-methylbenzensulfonat

8,1 g der unter 1d) hergestellten Verbindung wurden mit 21,0 g *p*-Toluolsulfonsäurechlorid und 25,5 ml Triethylamin in 92 ml Dichlormethan eine Stunde bei Raumtemperatur gerührt. Das Reaktionsgemisch wurde in gesättigte wässrige Natriumhydrogencarbonatlösung gegossen und mit Dichlormethan extrahiert. Die organische Phase wurde mit gesättigter wässriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergab 9,21 g der Titelverbindung als farblosen Schaum.
¹H-NMR (300 MHz, CDCl₃): δ [ppm] = 7,94 d (*J*=8 Hz, 1H, aryl); 7,89 d (*J*=2,1 Hz, 1H, aryl); 7,78 d (*J*=8,2 Hz, 2H, aryl); 7,77 dd (*J*=8 Hz / 2,1 Hz, 1H, aryl); 7,34 d (*J*=8,2 Hz, 2H, aryl); 4,03 t (*J*=6,5 Hz, 2H, CH₂O) 3,66 m (2H, CH₂N); 2,45 s (3H, CH₃); 1,81 m (2H, CH₂); 1,65 m (2H, CH₂); 1,58 s (6H, CH₃); 1,33 m (8H, CH₂).

### 1f) 4-[4,4-Dimethyl-5-oxo-3-(8-iodoctyl)-2-thioxoimidazolidin-1-yl]-2-(trifluormethyl)benzonitril

9,21 g der unter 1e) hergestellten Verbindung wurden mit 9,3 g Natriumiodid in 150 ml Aceton eine Stunde zum Sieden erhitzt. Das Reaktionsgemisch wurde bei Raumtemperatur filtriert und im Vakuum eingeengt. Säulenchromatographie an Kieselgel mit einem Gemisch aus Hexan/Ethylacetat ergab 8,55 g der Titelverbindung als gelblichen Schaum.
¹H-NMR (300 MHz, CDCl₃): δ [ppm] = 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,67 m (2H, CH₂N); 3,20 t (*J*=7 Hz, 2H, CH₂l); 1,82 m (2H, CH₂); 1,80 m (2H, CH₂); 1,58 s (6H, CH₃); 1,37 m (8H, CH₂).

### 1g) 4-[4,4-Dimethyl-5-oxo-3-[8-(pyrrolidin-1-yl)octyl]-2-thioxoimidazolidin-1-yl]-2-(trifluormethyl)benzonitril

200 mg der unter 1f) hergestellten Verbindung wurden mit 60 µl Pyrrolidin und 101 µl Triethylamin in 5 ml Tetrahydrofuran vier Stunden zum Sieden erhitzt. Das Reaktionsgemisch wurde im Vakuum eingeengt. Säulenchromatographie an Kieselgel mit einem Gemisch aus Hexan/Ethylacetat ergab 130 mg der Titelverbindung als farbloses Öl.
¹H-NMR (300 MHz, CDCl₃): δ [ppm] = 7,94 d (*J*=8 Hz, 1H, aryl); 7,89 d (*J*=2,1 Hz, 1H, aryl); 7,77 dd (*J*=8 Hz / 2,1 Hz, 1H, aryl); 3,67 m (2H, CH₂N); 2,64 m (4H, CH₂N); 2,53 m (2H, CH₂N); 1,85 m (4H, CH₂); 1,82 m (2H, CH₂); 1,59 m (2H, CH₂); 1,58 s (6H, CH₃); 1,36 m (8H, CH₂).

### Beispiel 2

### 4-[4,4-Dimethyl-5-oxo-3-[8-(pyrrolidin-1-yl)octyl]-2-thioxoimidazolidin-1-yl]-2-(trifluormethyl)benzonitril hydrochlorid

20 mg der unter 1g) hergestellten Verbindung wurden in 1 ml Tetrahydrofuran gelöst und mit 67 µl einer 1,2 molaren Lösung von Chlorwasserstoffsäure in Diethylether eine Stunde bei Raumtemperatur gerührt. Das Reaktionsgemisch wurde im Vakuum eingeengt. Man erhielt 21 mg der Titelverbindung.
¹H-NMR (300 MHz, CDCl₃): δ [ppm] = 7,95 d (*J*=8 Hz, 1H, aryl); 7,89 d (*J*=2,1 Hz, 1H, aryl); 7,78 dd (*J*=8 Hz / 2,1 Hz, 1H, aryl); 3,79 m (2H, CH₂N); 3,67 m (2H, CH₂N); 3,00 m (2H, CH₂N); 2,75 m (2H, CH₂N); 2,23 m (2H, CH₂); 2,06 m (2H, CH₂); 1,90 m (2H, CH₂); 1,82 m (2H, CH₂); 1,59 s (6H, CH₃); 1,38 m (8H, CH₂).

### Beispiel 3

### 4-[4,4-Dimethyl-5-oxo-3-[6-(pyrrolidin-1-yl)hexyl]-2-thioxoimidazolidin-1-yl]-2-(trifluormethyl)benzonitril

### 3a) 4-[3-(6-Iodhexyl)-4,4-dimethyl-5-oxo-2-thioxoimidazolidin-1-yl]-2-(trifluormethyl)benzonitril

Die Herstellung der Titelverbindung erfolgte analog zu dem in Beispiel 1d) bis 1f) beschriebenen Verfahren. Als Kettenbaustein wurde 6-Aminohexan-1-ol verwandt.
¹H-NMR (300 MHz, CDCl₃): δ [ppm] = 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,68 m (2H, CH₂N); 3,21 t (*J*=6,8 Hz, 2H, CH₂l) 1,86 m (2H, CH₂); 1,83 m (2H, CH₂); 1,59 s (6H, CH₃); 1,46 m (4H, CH₂).

### 3b) 4-[4,4-Dimethyl-5-oxo-3-[6-(pyrrolidin-1-yl)hexyl]-2-thioxoimidazolidin-1-yl]-2-(trifluormethyl)benzonitril

20 mg der unter 3a) hergestellten Verbindung wurden mit 6,3 µl Pyrrolidin und 10,6 µl Triethylamin in 1 ml Tetrahydrofuran vier Stunden zum Sieden erhitzt. Das Reaktionsgemisch wurde im Vakuum eingeengt. Säulenchromatographie an Kieselgel mit einem Gemisch aus Hexan/Ethylacetat ergab 15 mg der Titelverbindung als farbloses Öl.
¹H-NMR (300 MHz, CDCl₃): δ [ppm] = 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,68 m (2H, CH₂N); 2,75 m (4H, CH₂N); 2,64 m (2H, CH₂N); 1,90 m (4H, CH₂); 1,84 m (2H, CH₂); 1,68 m (2H, CH₂); 1,59 s (6H, CH₃); 1,43m(4H, CH₂).

### Beispiel 4

### 4-[4,4-Dimethyl-5-oxo-3-[7-(pyrrolidin-1-yl)heptyl]-2-thioxoimidazolidin-1-yl]-2-(trifluormethyl)benzonitril

### 4a) 4-[3-(7-Iodheptyl)-4,4-dimethyl-5-oxo-2-thioxoimidazolidin-1-yl]-2-(trifluormethyl)benzonitril

Die Herstellung der Titelverbindung erfolgte analog zu dem in Beispiel 1a) bis 1f) beschriebenen Verfahren. Als Kettenbaustein wurde Heptan-1,7-diol verwandt.
¹H-NMR (300 MHz, CDCl₃): δ [ppm] = 7,95 d (*J*=8 Hz, 1H, aryl); 7,90 d (*J*=2,1 Hz, 1H, aryl); 7,78 dd (*J*=8 Hz / 2,1 Hz, 1H, aryl); 3,67 m (2H, CH₂N); 3,20 t (*J*=7 Hz, 2H, CH₂l); 1,84 m (4H, CH₂); 1,59 s (6H, CH₃); 1,41 m (6H, CH₂).

### 4b) 4-[4,4-Dimethyl-5-oxo-3-[7-(pyrrolidin-1-yl)heptyl]-2-thioxoimidazolidin-1-yl]-2-(trifluormethyl)benzonitril

13 mg der unter 4a) hergestellten Verbindung wurden mit 4 µl Pyrrolidin und 6,7 µl Triethylamin in 1 ml Tetrahydrofuran vier Stunden zum Sieden erhitzt. Das Reaktionsgemisch wurde im Vakuum eingeengt. Säulenchromatographie an Kieselgel mit einem Gemisch aus Hexan/Ethylacetat ergab 7 mg der Titelverbindung als farbloses Öl.
¹H-NMR (300 MHz, CDCl₃): δ [ppm] = 7,95 d (*J*=8 Hz, 1H, aryl); 7,89 d (*J*=2,1 Hz, 1H, aryl); 7,78 dd (*J*=8 Hz / 2,1 Hz, 1H, aryl); 3,67 m (2H, CH₂N); 2,72 m (4H, CH₂N); 2,60 m (2H, CH₂N); 1,89 m (4H, CH₂); 1,83 m (2H, CH₂); 1,65 m (2H, CH₂); 1,58 s (6H, CH₃); 1,40 m (6H, CH₂).

### Beispiel 5

### 4-[4,4-Dimethyl-5-oxo-3-[9-(pyrrolidin-1-yl)nonyl]-2-thioxoimidazolidin-1-yl]-2-(trifluormethyl)benzonitril

### 5a) 4-[3-(9-Iodnonyl)-4,4-dimethyl-5-oxo-2-thioxoimidazolidin-1-yl]-2-(trifluormethyl)benzonitril

Die Herstellung der Titelverbindung erfolgte analog zu dem in Beispiel 1a) bis 1f) beschriebenen Verfahren. Als Kettenbaustein wurde Nonan-1,9-diol verwandt.
¹H-NMR (300 MHz, CDCl₃): δ [ppm] = 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,67 m (2H, CH₂N); 3,19 t (*J*=7 Hz, 2H, CH₂I) 1,84 m (2H, CH₂); 1,82 m (2H, CH₂); 1,58 s (6H, CH₃); 1,36 m (10H, CH₂).

### 5b) 4-[4,4-Dimethyl-5-oxo-3-[9-(pyrrolidin-1-yl)nonyl]-2-thioxoimidazolidin-1-yl]-2-(trifluormethyl)benzonitril

20 mg der unter 5a) hergestellten Verbindung wurden mit 5,9 µl Pyrrolidin und 9,9 µl Triethylamin in 1 ml Tetrahydrofuran vier Stunden zum Sieden erhitzt. Das Reaktionsgemisch wurde im Vakuum eingeengt. Säulenchromatographie an Kieselgel mit einem Gemisch aus Hexan/Ethylacetat ergab 10 mg der Titelverbindung als farbloses Öl.
¹H-NMR (300 MHz, CDCl₃): δ [ppm] = 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,66 m (2H, CH₂N); 2,47 m (4H, CH₂N); 2,40 m (2H, CH₂N); 1,77 m (4H, CH₂); 1,82 m (2H, CH₂); 1,58 s (6H, CH₃)1,51 m (2H, CH₂); ; 1,31 m (10H, CH₂).

In Analogie zu den ausführlich beschriebenen Herstellungsvorschriften zu den Beispielen 1 bis 5 wurden folgende Verbindungen erhalten:

| **Beispiel** | **Produkt/ Reagenz** | **Vorschrift analog zu** | **¹H-NMR (300 MHz, CDCl₃) δ [ppm]** |
|---|---|---|---|
| **6** | 4-[3-[6-[(2*R*)-2-(Hydroxymethyl)pyrrolidin-1-yl]hexyl]-4,4-dimethyl-5-oxo-2-thioxoimidazolidin-1-yl]-2-(trifluormethyl)benzonitril | 3 | 7,95 d (*J*=8 Hz, 1H, aryl); 7,90 d (*J*=2,1 Hz, 1H, aryl); 7,77 dd (*J*=8 Hz / 2,1 Hz, 1H, aryl); 3,67 m (2H, CH₂N); 3,62 dd (*J*=10,5 Hz / 3,8 Hz, 1H, CH₂OH) 3,39 dd (*J*=10,5 Hz / 2,1 Hz, 1H, CH₂OH) 3,19 m (1H, CH₂N); 2,73 dddbr (*J*=11,8 Hz / 7,6 Hz / 7,6 Hz, 1H, CH₂N); 2,58 m (1H, CH₂N); 2,25 m (2H, CH₂N); 1,96-1,67 m (4H, CH₂); 1,84 m (2H, CH₂); 1,58 s (6H, CH₃); 1,54 m (2H, CH₂); 1,41 m (4H, CH₂) |
| | | | |
| | (2*R*)-Pyrrolidinmethanol | | |
| **7** | 4-[3-[6-[(2*S*)-2-(Hydroxymethyl)pyrrolidin-1-yl]hexyl]-4,4-dimethyl-5-oxo-2-thioxoimidazolidin-1-yl]-2-(trifluormethyl)benzonitril | 3 | 7,95 d (*J*=8 Hz, 1H, aryl); 7,90 d (*J*=2,1 Hz, 1H, aryl); 7,77 dd (*J*=8 Hz / 2,1 Hz, 1H, aryl); 3,67 m (2H, CH₂N); 3,62 dd (*J*=10,5 Hz / 3,8 Hz, 1H, CH₂OH) 3,39 dd (*J*=10,5 Hz / 2,1 Hz, 1H, CH₂OH) 3,19 m (1H, CH₂N); 2,73 dddbr (*J*=11,8 Hz / 7,6 Hz / 7,6 Hz, 1H, CH₂N); 2,58 m (1H, CH₂N); 2,25 m (2H, CH₂N); 1,96-1,67 m (4H, CH₂); 1,84 m (2H, CH₂); 1,58 s (6H, CH₃); 1,54 m (2H, CH₂); 1,41 m (4H, CH₂) |
| | | | |
| | (2*S*)-Pyrrolidinmethanol | | |
| 8 | 4-[3-[7-[(2*R*)-2-(Hydroxymethyl)pyrrolidin-1-yl]heptyl]-4,4-dimethyl-5-oxo-2-thioxoimidazolidin-1-yl]-2-(trifluormethyl)benzonitril | 4 | 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,67 m (2H, CH₂N); 3,62 dd (*J*=10,5 Hz / 3,8 Hz, 1H, CH₂OH) 3,39 dd (*J*=10,5 Hz / 2,5 Hz, 1H, CH₂OH) 3,18 m (1H, CH₂N); 2,71 dddbr (*J*=11,8 Hz / 8 Hz / 8 Hz, 1H, CH₂N); 2,57 m (1H, CH₂N); 2,25 m (1H, CH₂N); 2,24 m (1H, CH₂N); 1,95-1,66 m (4H, CH₂); 1,80 m (2H, CH₂); 1,58 s (6H, CH₃); 1,51 m (2H, CH₂); 1,39 m (6H, CH₂) |
| | | | |
| | (2R)-Pyrrolidinmethanol | | |
| 9 | 4-[3-[7-[(2*S*)-2-(Hydroxymethyl)pyrrolidin-1-yl]heptyl]-4,4-dimethyl-5-oxo-2-thioxoimidazolidin-1-yl]-2-(trifluormethyl)benzonitril | 4 | 7,95 d (*J*=8 Hz, 1H, aryl); 7,89 d (*J*=2,1 Hz, 1H, aryl); 7,77 dd (*J*=8 Hz / 2,1 Hz, 1H, aryl); 3,67 m (2H, CH₂N); 3,62 dd (*J*=10,5 Hz / 3,8 Hz, 1H, CH₂OH) 3,39 dd (*J*=10,5 Hz / 2,5 Hz, 1H, CH₂OH) 3,18 m (1H, CH₂N); 2,71 dddbr (*J*=11,8 Hz / 8 Hz / 8 Hz, 1H, CH₂N); 2,57 m (1H, CH₂N); 2,25 m (1H, CH₂N); 2,24 m (1H, CH₂N); 1,95-1,66 m (4H, CH₂); 1,80 m (2H, CH₂), 1,58 s (6H, CH₃); 1,51 m (2H, CH₂); 1,39 m (6H, CH₂) |
| | | | |
| | (2*S*)-Pyrrolidinmethanol | | |
| **10** | 4-[3-[8-[(2R)-2-(Hydroxymethyl)pyrrolidin-1-yl]octyl]-4,4-dimethyl-5-oxo-2-thioxoimidazolidin-1-yl]-2-(trifluormethyl)benzonitril | 1 | 7,95 d (*J*=8 Hz, 1 H, aryl); 7,89 d (*J*=2,1 Hz, 1 H, aryl); 7,78 dd (*J*=8 Hz / 2,1 Hz, 1 H, aryl); 3,67 m (2H, CH₂N); 3,63 dd (*J*=10,5 Hz/ 3.8 Hz, 1H, CH₂OH); 3,39 dd (*J*=10,5 Hz / 2,5 Hz, 1 H, CH₂OH) 3,18 m (1 H, CH₂N); 2,71 dddbr (J=11,8 Hz / 8 Hz / 8 Hz, 1 H, CH₂N); 2,58 m (1 H, CH₂N); 2,25 m (2H, CH₂N); 1,96-1,67 m (4H, CH₂); -1,82 m (2H, CH₂); 1,58 s (6H, CH₃); 1,50 m (2H, CH₂); 1,36m(8H,CH₂) |
| | | | |
| | (2*R*)-Pyrrolidinmethanol | | |
| **11** | 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril | 1 | 7,95 d (*J*=8 Hz, 1 H, aryl); 7,89 d (*J*=2,1 Hz, 1 H, aryl); 7,78 dd (J=8 Hz / 2,1 Hz, 1 H, aryl); 3,67 m (2H, CH₂N); 3,63 dd (*J*=10,5 Hz / 3,8 Hz, 1 H, CH₂OH) 3,39 dd (*J*=10,5 Hz 2,5 Hz, 1 H, CH₂OH); 3,18 m (1H, CH₂N); 2,71 dddbr (J=11,8 Hz / 8 Hz / 8 Hz, 1 H, CH₂N); 2,58 m (1 H, CH₂N); 2,25 m (2H, CH₂N); 1,96-1,67 m (4H, CH₂); 1,82 m (2H, CH₂); 1,58 s (6H, CH₃); 1,50 m (2H, CH₂); 1,36 m (8H, CH₂) |
| | | | |
| | (2*S*)-Pyrrolidinmethanol | | |
| **12** | 4-[3-[9-[(2*R*)-2-(Hydroxymethyl)pyrrolidin-1-yl]nonyl]-4,4-dimethyl-5-oxo-2-thioxoimidazolidin-1-yl]-2-(trifluormethyl)benzonitril | 5 | 7,95 d (*J*=8 Hz, 1H, aryl); 7,90 d (*J*=2,1 Hz, 1 H, aryl); 7,77 dd (*J*=8 Hz / 2,1 Hz, 1 H, aryl); 3,67 m (2H, CH₂N); 3,62 dd (*J*=10,5 Hz / 3,8 Hz, 1 H, CH₂OH) 3,38 dd (*J*=10,5 Hz / 2,5 Hz, 1 H, CH₂OH); 3,17 m (1H, CH₂N); 2,70 dddbr (*J*=11,8 Hz / 8,4 Hz / 8 Hz, 1 H, CH₂N); 2,56 m (1H, CH₂N); 2,24 m (1H, CH₂N); 2,21 m (1 H, CH₂N); 1,92-1,67 m (4H, CH₂); 1,82 m (2H, CH₂); 1,58 s (6H, CH₃); 1,48 m (2H, CH₂); 1,31 m (10H, CH₂) |
| | | | |
| | (2*R*)-Pyrrolidinmethanol | | |
| **13** | 4-[3-[9-[(2S)-2-(Hydroxymethyl)pyrrolidin-1-yl]nonyl]-4,4-dimethyl-5-oxo-2-thioxoimidazolidin-1-yl]-2-(trifluormethyl)benzonitril | 5 | 7,95 d (*J*=8 Hz, 1 H, aryl); 7,90 d (*J*=2,1 Hz, 1 H, aryl); 7,77 dd (*J*=8 Hz / 2,1 Hz, 1 H, aryl); 3,67 m (2H, CH₂N); 3,62 dd (*J*=10,5 Hz / 3,8 Hz, 1 H, CH₂OH); 3,38 dd (*J*=10,5 Hz / 2,5 Hz, 1 H, CH₂OH 3,17 m (1 H, CH₂N); 2,70 dddbr (J=11,8 Hz / 8,4 Hz / 8 Hz, 1 H, CH₂N); 2,56 m (1H. CH₂N); 2,24 m (1H, CH₂N); 2,21 m (1H, CH₂N); 1,92-1,67 m (4H, CH₂); 1,82 m (2H, CH₂); 1,58 s (6H, CH₃); 1,48 m (2H, CH₂); 1,31 m (10H, CH₂) |
| | | | |
| | (2*S*)-Pyrrolidinmethanol | | |
| **14** | 4-[3-[6-[(2*R*)-2-(Methoxymethyl)pyrrolidin-1-yl]hexyl]-4,4-dimethyl-5-oxo-2-thioxoimidazolidin-1 -yl]-2-(trifluormethyl)benzonitril | 3 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 3,67 m (2H, CH₂N); 3,42 dd (*J*=9,3 Hz / 5,1 Hz, 1 H, CH₂O); 3,35 s (3H, CH₃O); 3,28 dd (*J*=9,3 Hz / 5,9 Hz, 1 H, CH₂O); 3,16 m (1 H, CH₂N); 2,85 dddbr (*J*=11,8 Hz / 8,4 Hz / 8 Hz, 1 H, CH₂N); 2,56 m (1 H, CH₂N); 2,26 dddbr (*J*=11,8 Hz / 7,6 Hz / 7,6 Hz, 1 H, CH₂N); 2,17 m (1 H, CH₂N); 1,92-1,67 m (4H, CH₂); 1,83 m (2H, CH₂); 1,57 s (6H, CH₃); 1,54 m (2H, CH₂); 1,40 m (4H, CH₂) |
| | | | |
| | (2*R*)-2-(Methoxymethyl)-pyrrolidin | | |
| **15** | 4-[3-[6-[(2*S*)-2-(Methoxymethyl)pyrrolidin-1-yl]hexyl]-4,4-dimethyl-5-oxo-2-thioxoimidazolidin-1-yl]-2-(trifluormethyl)benzonitril | 3 | 7,95 d (*J*=8 Hz, 1 H, aryl); 7,89 d (*J*=2,1 Hz, 1H, aryl); 7,77 dd (*J*=8 Hz / 2,1 Hz, 1 H, aryl); 3,67 m (2H, CH₂N); 3,42 dd (*J*=9,3 Hz / 5,1 Hz, 1 H, CH₂O) 3,35 s (3H, CH₃O) 3,28 dd (*J*=9,3 Hz / 5,9 Hz, 1 H, CH₂O); 3,16 m (1 H, CH₂N); 2,85 dddbr (J=11,8 Hz / 8,4 Hz / 8 Hz, 1 H, CH₂N); 2,56 m (1 H, CH₂N); 2,26 dddbr (*J*=11,8 Hz / 7,6 Hz / 7,6 Hz, 1 H, CH₂N); 2,17 m (1H, CH₂N); 1,92-1,67 m (4H, CH₂); 1,83 m (2H, CH₂); 1,57 s (6H, CH₃); 1,54 m (2H, CH₂); 1,40 m (4H, CH₂) |
| | | | |
| | (2*S*)-2-(Methoxymethyl)-pyrrolidin | | |
| **16** | 4-[3-[7-[(2*R*)-2-(Methoxymethyl)pyrrolidin-1- yl]heptyl]-4,4-dimethyl-5-oxo-2-hioxoimidazolidin-1-yl]-2-(trifluormethyl)benzonitril | 4 | 7,95 d (*J*=8 Hz, 1H, aryl); 7,89 d (*J*=2,1 Hz, 1 H, aryl); 7,77 dd (*J*=8 Hz / 2,1 Hz, 1 H, aryl); 3,66 m (2H, CH₂N); 3,42 dd (*J*=9,3 Hz / 5,1 Hz, 1 H, CH₂O) 3,35 s (3H, CH₃O); 3,27 dd (*J*=9,3 Hz / 5,9 Hz, 1 H, CH₂O); 3,14 m (1H, CH₂N); 2,83 dddbr (*J*=11,8 Hz / 8,4 Hz / 8 Hz, 1 H, CH₂N); 2,55 m (1 H, CH₂N); 2,24 dddbr (*J*=11,8 Hz / 7,6 Hz / 7,6 Hz, 1 H, CH₂N); 2,16 m (1H, CH₂N); 1,92-1,67 m (4H, CH₂); 1,83 m (2H, CH₂); 1,58 s (6H, CH₃); 1,51 m (2H, CH₂); 1,38 m (6H, CH₂) |
| | | | |
| | (2*R*)-2-(Methoxymethyl)-pyrrolidin | | |
| **17** | 4-[3-[7-[(2S)-2-(Methoxymethyl)pyrrolidin-1-yl]heptyl]-4,4-dimethyl-5-oxo-2-thioxoimidazolidin-1-yl]-2-(trifluormethyl)benzonitril | 4 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 3,66 m (2H, CH₂N); 3,42 dd (*J*=9,3 Hz / 5,1 Hz, 1 H, CH₂O); 3,35 s (3H, CH₃O) 3,27 dd (*J*=9,3 Hz / 5,9 Hz, 1H, CH₂O) 3,14 m (1 H, CH₂N); 2,83 dddbr (*J*=11,8 Hz / 8,4 Hz / 8 Hz, 1 H, CH₂N); 2,55 m (1 H, CH₂N); 2,24 dddbr (*J*=11,8 Hz / 7,6 Hz / 7,6 Hz, 1 H, CH₂N); 2,16 m (1H, CH₂N); 1,92-1,67 m (4H, CH₂); 1,83 m (2H, CH₂); 1,58 s (6H, CH₃); 1,51 m (2H, CH₂); 1,38 m (6H, CH₂) |
| | | | |
| | (2*S*)-2-(Methoxymethyl)-pyrrolidin | | |
| **18** | 4-[3-[8-[(2*R*)-2-(Methoxymethyl)pyrrolidin-1-yl]octyl]-4,4-dimethyl-5-oxo-2-thioxoimidazolidin-1-yl]-2-(trifluormethyl)benzonitril | 1 | 7,94 d (*J*=8 Hz, 1 H, aryl); 7,89 d (*J*=2,1 Hz, 1H, aryl); 7,77 dd (*J*=8 Hz / 2,1 Hz, 1 H, aryl); 3,66 m (2H, CH₂N); 3,43 dd (*J*=9,3 Hz / 5,1 Hz, 1 H, CH₂O) 3,35 s (3H, CH₃O) 3,27 dd (J=9,3 Hz / 5,9 Hz, 1 H, CH₂O); 3,15 m (1 H, CH₂N); 2,82 dddbr (*J*=11,8 Hz / 8,4 Hz / 8 Hz, 1H, CH₂N); 2,56 m (1H, CH₂N); 2,24 dddbr (*J*=11,8 Hz / 7,6 Hz / 7,6 Hz, 1 H, CH₂N); 2,17 m (1H, CH₂N); 1,92-1,67 m (4H, CH₂); 1,82 m (2H, CH₂); 1,58 s (6H, CH₃); 1,51 m (2H, CH₂); 1,36 m (8H, CH₂) |
| | | | |
| | (2*R*)-2-(Methoxymethyl)-pyrrolidin | | |
| **19** | 4-[3-[8-[(2*S*)-2-(Methoxymethyl)pyrrolidin-1-yl]octyl]-4,4-dimethyl-5-oxo-2-thioxoimidazolidin-1-yl]-2-(trifluormethyl)benzonitril | 1 | 7,94 d (*J*=8 Hz, 1 H, aryl); 7,89 d (*J*=2,1 Hz, 1 H, aryl); 7,77 dd (*J*=8 Hz / 2,1 Hz, 1 H, aryl); 3,66 m (2H, CH₂N); 3,43 dd (*J*=9,3 Hz / 5,1 Hz, 1 H, CH₂O) 3,35 s (3H, CH₃O) 3,27 dd (*J*=9,3 Hz / 5,9 Hz, 1 H, CH₂O); 3,15 m (1 H, CH₂N); 2,82 dddbr (*J*=11,8 Hz / 8,4 Hz / 8 Hz, 1 H, CH₂N); 2,56 m (1 H, CH₂N); 2,24 dddbr (*J*=11,8 Hz / 7,6 Hz / 7,6 Hz, 1H, CH₂N); 2,17 m (1H, CH₂N); 1,92-1,67 m (4H, CH₂); 1,82 m (2H, CH₂); 1,58 s (6H, CH₃); 1,51 m (2H, CH₂); 1,36 m (8H, CH₂) |
| | | | |
| | (2*S*)-2-(Methoxymethyl)-pyrrolidin | | |
| **20** | 4-[3-[9-[(2*R*)-2-(Methoxymethyl)pyrrolidin-1-yl]nonyl]-4,4-dimethyl-5-oxo-2-thioxoimidazolidin-1-yl]-2-(trifluormethyl)benzonitril | 5 | 7,95 d (*J*=8 Hz, 1H, aryl); 7,89 d (*J*=2,1 Hz, 1 H, aryl); 7,77 dd (*J*=8 Hz / 2,1 Hz, 1 H, aryl); 3,66 m (2H, CH₂N); 3,41 dd (J=9,3 Hz / 5,1 Hz, 1 H, CH₂O) 3,35 s (3H, CH₃O) 3,27 dd (*J*=9,3 Hz / 5,9 Hz, 1 H, CH₂O); 3,13 m (1H, CH₂N); 2,80 dddbr (*J*=11,8 Hz / 8,4 Hz / 8 Hz, 1 H, CH₂N); 2,53 m (1 H, CH₂N); 2,22 dddbr (*J*=11,8 Hz / 7,6 Hz / 7,6 Hz, 1 H, CH₂N); 2,15 m (1H, CH₂N); 1,92-1,67 m (4H, CH₂); 1,83 m (2H, CH₂); 1,58 s (6H, CH₃); 1,49 m (2H, CH₂); 1,31 m (10H, CH₂) |
| | | | |
| | (2*R*)-2-(Methoxymethyl)-pyrrolidin | | |
| **21** | 4-[3-[9-[(2*S*)-2-(Methoxymethyl)pyrrolidin-1-yl]nonyl]-4,4-dimethyl-5-oxo-2-thioxoimidazolidin-1-yl]-2-(trifluormethyl)benzonitril | 5 | 7,95 d (*J*=8 Hz, 1 H, aryl); 7,89 d (*J*=2,1 Hz, 1 H, aryl); 7,77 dd (J=8 Hz / 2,1 Hz, 1 H, aryl); 3,66 m (2H, CH₂N); 3,41 dd (*J*=9,3 Hz / 5,1 Hz, 1 H, CH₂O) 3,35 s (3H, CH₃O) 3,27 dd (*J*=9,3 Hz / 5,9 Hz, 1 H, CH₂O) 3,13 m (1 H, CH₂N); 2,80 dddbr (*J*=11,8 Hz / 8,4 Hz / 8 Hz, 1 H, CH₂N); 2,53 m (1 H, CH₂N); 2,22 dddbr (*J*=11,8 Hz / 7,6 Hz / 7,6 Hz, 1 H, CH₂N); 2,15 m (1H, CH₂N); 1,92-1,67 m (4H, CH₂); 1,83 m (2H, CH₂); 1,58 s (6H, CH₃); 1,49 m (2H, CH₂); 1,31 m (10H, CH₂) |
| | | | |
| | (2*S*)-2-(Methoxymethyl)-pyrrolidin | | |
| **22** | 4-[3-[6-(3-Hydroxy-8-azabicyclo[3.2.1]oct-8-yl)hexyl]-4,4-dimethyl-5-oxo-2-thioxoimidazolidin-1-yl]-2-(trifluormethyl)benzonitril | 3 | 7,95 d (*J*=8 Hz, 1H, aryl); 7,90 d (*J*=2,1 Hz, 1 H, aryl); 7,79 dd (*J*=8 Hz / 2,1 Hz, 1 H, aryl); 4,04 m (1 H, CHOH); 3,69 m (2H, CH₂N); 3,19 m (2H, CHN); 2,35 m (2H, CH₂N); 2,05 m (4H, CH₂); 1,91 m (2H, CH₂); 1,86 m (2H, CH₂); 1,62 m (2H, CH₂); 1,58 s (6H, CH₃); 1,53 m (2H, CH₂); 1,40 m (4H, CH₂) |
| | | | |
| | 8-Azabicyclo[3.2.1]octan-3-ol | | |
| **23** | 4-[3-[7-(3-Hydroxy-8-azabicyclo[3.2.1]oct-8-yl)heptyl]-4,4-dimethyl-5-oxo-2-thioxoimidazolidin-1-yl]-2-(trifluormethyl)benzonitril | 4 | 7,95 d (*J*=8 Hz, 1H, aryl); 7,89 d (*J*=2,1 Hz, 1 H, aryl); 7,77 dd (*J*=8 Hz / 2,1 Hz, 1 H, aryl); 4,05 m (1 H, CHOH); 3,66 m (2H, CH₂N); 3,19 m (2H, CHN); 2,33 m (2H, CH₂N); 2,07 m (4H, CH₂); 1,92 m (2H, CH₂); 1,82 m (2H, CH₂); 1,62 m (2H, CH₂); 1,58 s (6H, CH₃); 1,49 m (2H, CH₂); 1,38 m (6H, CH₂) |
| | | | |
| | 8-Azabicyclo[3.2.1]octan-3-ol | | |
| **24** | 4-[3-[8-(3-Hydroxy-8-azabicyclo[3.2.1 ]oct-8-yl)octyl]-4,4-dimethyl-5-oxo-2-thioxoimidazolidin-1-yl]-2-(trifluormethyl)benzonitril | 1 | 7,95 d (*J*=8 Hz, 1 H, aryl); 7,89 d (*J*=2,1 Hz, 1 H, aryl); 7,78 dd (*J*=8 Hz / 2,1 Hz, 1 H, aryl); 4,05 m (1 H, CHOH); 3,66 m (2H, CH₂N); 3,19 m (2H, CHN); 2,33 m (2H, CH₂N); 2,07 m (4H, CH₂); 1,92 m (2H, CH₂); 1,81 m (2H, CH₂); 1,62 m (2H, CH₂); 1,59 s (6H, CH₃); 1,48 m (2H, CH₂); 1,38 m (8H, CH₂) |
| | | | |
| | 8-Azabicyclo[3.2.1]octan-3-ol | | |
| **25** | 4-[3-[9-(3-Hydroxy-8-azabicyclo[3.2.1]oct-8-yl)nonyl]-4,4-dimethyl-5-oxo-2-thioxoimidazolidin-1-yl]-2-(trifluormethyl)benzonitril | 5 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 4,04 m (1 H, CHOH); 3,66 m (2H, CH₂N); 3,19 m (2H, CHN); 2,33 m (2H, CH₂N); 2,08 m (4H, CH₂); 1,91 m (2H, CH₂); 1,82 m (2H, CH₂); 1,62 m (2H, CH₂); 1,58 s (6H, CH₃); 1,48 m (2H, CH₂); 1,31 m(10H,CH₂) |
| | | | |
| | 8-Azabicyclo[3.2.1]octan-3-ol | | |
| **26** | 4-[3-[6-[(*R*)-3-Hydroxypyrrolidin-1-yl]hexyl]-4,4-dimethyl-5-oxo-2-thioxoimidazolidin-1-yl]-2-(trifluormethyl)benzonitril | 3 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 4,34 m (1 H, CHOH); 3,67 m (2H, CH₂N); 2,89 m (1 H, CH₂N); 2,69 m (1 H, CH₂N); 2,50 m (1 H, CH₂N); 2,46 m (2H, CH₂N); 2,28 m (1 H, CH₂N); 2,20 m (1H, CH₂); 1,83 m (2H, CH₂); 1,74 m (1 H, CH₂); 1,57 s (6H, CH₃); 1,56 m (2H, CH₂); 1,41 m (4H, CH₂) |
| | | | |
| | (*R*)-Pyrrolidin-3-ol | | |
| **27** | 4-[3-[7-[(*R*)-3-Hydroxypyrrolidin-1-yl]heptyl]-4,4-dimethyl-5-oxo-2-thioxoimidazolidin-1-yl]-2-(trifluormethyl)benzonitril | 4 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 4,34 m (1 H, CHOH); 3,66 m (2H, CH₂N); 2,89 m (1 H, CH₂N); 2,69 m (1H, CH₂N); 2,52 m (1 H, CH₂N); 2,45 m (2H, CH₂N); 2,30 m (1 H, CH₂N); 2,17 m (1 H, CH₂); 1,83 m (2H, CH₂); 1,74 m (1 H, CH₂); 1,58 s (6H, CH₃); 1,52 m (2H, CH₂); 1,39 m (6H, CH₂) |
| | | | |
| | (*R*)-Pyrrolidin-3-ol | | |
| **28** | 4-[3-[8-[(*R*)-3-Hydroxypyrrolidin-1-yl]octyl]-4,4-dimethyl-5-oxo-2-thioxoimidazolidin-1-yl]-2-(trifluormethyl)benzonitril | 1 | 7,95 d (*J*=8 Hz, 1 H, aryl); 7,89 d (J=2,1 Hz, 1 H, aryl); 7,77 dd (*J*=8 Hz / 2,1 Hz, 1 H, aryl); 4,34 m (1 H, CHOH); 3,66 m (2H, CH₂N); 2,89 m (1 H, CH₂N); 2,70 m (1 H, CH₂N); 2,53 m (1 H, CH₂N); 2,45 m (2H, CH₂N); 2,30 m (1H, CH₂N); 2,19 m (1 H, CH₂); 1,82 m (2H, CH₂); 1,75 m (1H, CH₂); 1,58 s (6H, CH₃); 1,51 m (2H, CH₂); 1,35 m (8H, CH₂) |
| | | | |
| | (*R*)-Pyrrolidin-3-ol | | |
| **29** | 4-[3-[9-[(*R*)-3-Hydroxypyrrolidin-1-yl]nonyl]-4,4-dimethyl-5-oxo-2-thioxoimidazolidin-1-yl]-2-(trifluormethyl)benzonitril | 5 | 7,95 d (*J*=8 Hz, 1 H, aryl); 7,89 d (*J*=2,1 Hz, 1H, aryl); 7,77 dd (*J*=8 Hz / 2,1 Hz, 1 H, aryl); 4,32 m (1 H, CHOH); 3,66 m (2H, CH₂N); 2,87 m (1H, CH₂N); 2,66 m (1 H, CH₂N); 2,48 m (1 H, CH₂N); 2,41 m (2H, CH₂N); 2,25 m (1 H, CH₂N); 2,17 m (1H, CH₂); 1,82 m (2H, CH₂); 1,72 m (1H, CH₂); 1,58 s (6H, CH₃); 1,49 m (2H, CH₂); 1,31 m (10H, CH₂) |
| | | | |
| | (*R*)-Pyrrolidin-3-ol | | |
| **30** | 4-[3-[6-[(*S*)-3-Hydroxypyrrolidin-1-yl]hexyl]-4,4-dimethyl-5-oxo-2-thioxoimidazolidin-1-yl]-2-(trifluormethyl)benzonitril | 3 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 4,37 m (1 H, CHOH); 3,67 m (2H, CH₂N); 2,94 m (1 H, CH₂N); 2,75 m (1 H, CH₂N); 2,59 m (1 H, CH₂N); 2,51 m (2H, CH₂N); 2,37 m (1 H, CH₂N); 2,21 m (1 H, CH₂); 1,83 m (2H, CH₂); 1,81 m (1 H, CH₂); 1,58 s (6H, CH₃); 1,57 m (2H, CH₂); 1,42 m (4H, CH₂) |
| | | | |
| | (*S*)-Pyrrolidin-3-ol | | |
| **31** | 4-[3-[7-[(*S*)-3-Hydroxypyrrolidin-1-yl]heptyl]-4,4-dimethyl-5-oxo-2-thioxoimidazolidin-1-yl]-2-(trifluormethyl)benzonitril | 4 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 4,37 m (1 H, CHOH); 3,66 m (2H, CH₂N); 2,95 m (1 H, CH₂N); 2,75 m (1 H, CH₂N); 2,61 m (1 H, CH₂N); 2,52 m (2H, CH₂N); 2,40 m (1 H, CH₂N); 2,21 m (1 H, CH₂); 1,83 m (2H, CH₂); 1,81 m (1 H, CH₂); 1,58 s (6H, CH₃); 1,56 m (2H, CH₂); 1,39 m (6H, CH₂) |
| | | | |
| | (*S*)-Pyrrolidin-3-ol | | |
| **32** | 4-[3-[8-[(*S*)-3-Hydroxypyrrolidin-1-yl]octyl]-4,4-dimethyl-5-oxo-2-thioxoimidazolidin-1-yl]-2-(trifluormethyl)benzonitril | 1 | 7,95 d (*J*=8 Hz, 1 H, aryl); 7,89 d (*J*=2,1 Hz, 1H, aryl); 7,77 dd (*J*=8 Hz / 2,1 Hz, 1 H, aryl); 4,34 m (1 H, CHOH); 3,67 m (2H, CH₂N); 2,89 m (1 H, CH₂N); 2,70 m (1 H, CH₂N); 2,53 m (1 H, CH₂N); 2,45 m (2H, CH₂N); 2,30 m (1 H, CH₂N); 2,19 m (1 H, CH₂); 1,82 m (2H, CH₂); 1,75 m (1 H, CH₂); 1,58 s (6H, CH₃); 1,51 m (2H, CH₂); 1,36 m (8H, CH₂) |
| | | | |
| | (*S*)-Pyrrolidin-3-ol | | |
| **33** | 4-[3-[9-[(*S*)-3-Hydroxypyrrolidin-1-yl]nonyl]-4,4-dimethyl-5-oxo-2-thioxoimidazolidin-1-yl]-2-(trifluormethyl)benzonitril | 5 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 4,33 m (1 H, CHOH); 3,66 m (2H, CH₂N); 2,87 m (1 H, CH₂N); 2,67 m (1 H, CH₂N); 2,49 m (1 H, CH₂N); 2,42 m (2H, CH₂N); 2,26 m (1 H, CH₂N); 2,17 m (1H, CH₂); 1,82 m (2H, CH₂); 1,72 m (1H, CH₂); 1,58 s (6H, CH₃); 1,49 m (2H, CH₂); 1,31 m (10H. CH₂) |
| | | | |
| | (*S*)-Pyrrolidin-3-ol | | |
| **34** | 4-[4,4-Dimethyl-3-[6-(2-methylpyrrolidin-1-yl)hexyl]-5-oxo-2-thioxoimidazolidin-1-yl]-2-(trifluormethyl)benzonitril | 3 | 7,95 d (*J*=8 Hz, 1 H, aryl); 7,89 d (*J*=2,1 Hz, 1 H, aryl); 7,77 dd (*J*=8 Hz / 2,1 Hz, 1H, aryl); 3,67 m (2H, CH₂N); 3,17 m (1 H, CH₂N); 2,79 m (1 H, CH₂N); 2,27 m (1H, CHN); 2,06 m (1H, CH₂N); 2,03 m (1H, CH₂N); 2,00-1,35 m (4H, CH₂); 1,83 m (2H, CH₂); 1,57 s (6H, CH₃); 1,57 m (2H, CH₂); 1,41 m (4H, CH₂); 1,10 d (*J*=6 Hz, 3H, CH₃) |
| | | | |
| | 2-Methylpyrrolidin | | |
| **35** | 4-[4,4-Dimethyl-3-[7-(2-methylpyrrolidin-1-yl)heptyl]-5-oxo-2-thioxoimidazolidin-1-yl]-2-(trifluormethyl)benzonitril | 4 | 7,94 d (*J*=8 Hz, 1H, aryl); 7,89 d (*J*=2,1 Hz, 1 H, aryl); 7,77 dd (*J*=8 Hz / 2,1 Hz, 1 H, aryl); 3,66 m (2H, CH₂N); 3,16 m (1 H, CH₂N); 2,78 m (1 H, CH₂N); 2,24 m (1H. CHN); 2,06 m (1 H, CH₂N); 2,02 m (1 H, CH₂N); 2,00-1,35 m (4H, CH₂); 1,82 m (2H, CH₂); 1,57 s (6H, CH₃); 1,52 m (2H, CH₂); 1,38 m (6H, CH₂); 1,10 d (*J*=6 Hz, 3H, CH₃) |
| | | | |
| | 2-Methylpyrrolidin | | |
| **36** | 4-[4,4-Dimethyl-3-[8-(2-methylpyrrolidin-1-yl)octyl]-5-oxo-2-thioxoimidazolidin-1-yl]-2-(trifluormethyl)benzonitril | 1 | 7,94 d (*J*=8 Hz, 1 H, aryl); 7,89 d (*J*=2,1 Hz, 1 H, aryl); 7,77 dd (*J*=8 Hz / 2,1 Hz, 1H, aryl); 3,66 m (2H, CH₂N); 3,16 m (1 H, CH₂N); 2,77 m (1 H, CH₂N); 2,25 m (1H, CHN); 2,05 m (1H, CH₂N); 2,03 m (1 H, CH₂N); 2,00-1,35 m (4H, CH₂); 1,82 m (2H, CH₂); 1,58 s (6H, CH₃); 1,51 m (2H, CH₂); 1,35 m (8H, CH₂); 1,10 d (*J*=6 Hz, 3H, CH₃) |
| | | | |
| | 2-Methylpyrrolidin | | |
| **37** | 4-[3-[8-[(2*R*,5*S*)-*rel*-2,5-Dimethylpyrrolidin-1-yl]octyl]-4,4-dimethyl-5-oxo-2-thioxoimidazolidin-1-yl]-2-(trifluormethyl)benzonitril | 1 | 7,95 d (*J*=8 Hz, 1 H, aryl); 7,89 d (*J*=2,1 Hz, 1 H, aryl); 7,78 dd (*J*=8 Hz, / 2,1 Hz, 1 H, aryl); 3,67 m (2H, CH₂N); 2,57 m (2H, CHN); 2,55 m (2H, CH₂N); 1,80 m (2H, CH₂); 1,82 m (2H, CH₂); 1,58 s (6H, CH₃); 1,46 m (2H, CH₂); 1,30 m (2H, CH₂); 1,35 m (8H, CH₂); 1,11 d (*J*=6 Hz, 6H, CH₃) |
| | | | |
| | (2*R*,5*S*)-*rel*-2,5-Dimethyl-pyrrolidin | | |
| **38** | 4-[3-[6-[(2*S*)-2-(1-Hydroxy-1-methylethyl)pyrrolidin-1-yl]hexyl]-4,4-dimethyl-5-oxo-2-thioxoimidazolidin-1-yl]-2-(trifluormethyl)benzonitril | 3 | 1,12 (s, 3H); 1,21 (s, b, 3H); 1,30-1,48 (b, 4H); 1,58 (s, 6H); 1,49-1,95 (b, 9H); 2,33-2,72 (b, 3H); 2,73-2,96 (b, 1 H); 2,99-3,28 (b, 1 H); 3,65-3,70 (m, 2H); 7,77 (dd, J=2,11 Hz, 8,43 Hz; 1 H); 7,89 (d, J=1,68 Hz; 1H); 7,95 (d, J=8,43 Hz; 1 H) |
| | | | |
| | (2*S*)-α,α-Dimethylpyrrolidin-methanol, hergestellt aus L-Prolinmethylester nach K. Nakayama, W. J. Thompson, J. Am. Chem. Soc. 112, 6936-6942 (1990) | | |
| **39** | 4-[3-[7-[(2*S*)-2-(1-Hydroxy-1-methylethyl)pyrrolidin-1-yl]heptyl]-4,4-dimethyl-5-oxo-2-thioxoimidazolidin-1-yl]-2-(trifluormethyl)benzonitril | 4 | 1,11 (s, 3H); 1,20 (s, b, 3H); 1,30-1,47 (b, 6H); 1,58 (s, 6H); 1,50-1,92 (b, 9H); 2,35-2,68 (b, 3H); 2,70-2,91 (b, 1 H); 3,00-3,22 (b, 1 H); 3,64-3,69 (m, 2H); 7,77 (dd, J=1,90 Hz, 8,26 Hz; 1 H); 7,89 (d, J=2,10 Hz; 1 H); 7,95 (d, J=8,43 Hz; 1 H) |
| | | | |
| | (2*S*)-α,α-Dimethylpyrrolidin-methanol | | |
| **40** | 4-[3-[8-[(2*S*)-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril | 1 | 1,12 (s, 3H); 1,22 (s, b, 3H); 1,27-1,44 (b, 8H); 1,58 (s, 6H); 1,50-1,92 (b, 10H); 2,36-2,70 (b, 2H); 2,71-2,95 (b, 1 H); 3,00-3,23 (b, 1 H); 3,64-3,70 (m, 2H); 7,77 (dd, J=2,11 Hz, 8,43 Hz; 1 H); 7,89 (d, J=2,11 Hz; 1 H); 7,95 (d, J=8,43 Hz; 1 H) |
| | | | |
| | (2*S*)-α,α-Dimethylpyrrolidin-methanol | | |
| **41** | 4-[3-[9-[(2*S*)-2-(1-Hydroxy-1-methylethyl)pyrrolidin-1-yl]nonyl]-4,4-dimethyl-5-oxo-2-thioxoimidazolidin-1-yl]-2-(trifluormethyl)benzonitril | 5 | 1,11 (s, 3H); 1,18 (s, b, 3H); 1,24-1,44 (b, 10H); 1,45-1,55 (b, 2H); 1,58 (s, 6H); 1,63-1,90 (b, 7H); 2,30-3,20 (b, 5H); 3,64-3,69 (m, 2H); 7,78 (dd, J=2,10 Hz, 8,43 Hz; 1 H); 7,90 (d, J=2,53 Hz; 1 H); 7,95 (d, J=8,01 Hz; 1 H) |
| | | | |
| | (2*S*)-α,α-Dimethylpyrrolidin-methanol | | |
| **42** | 4-[3-[6-[(2*R*)-2-(1-Hydroxy-1-methylethyl)pyrrolidin-1-yl]hexyl]-4,4-dimethyl-5-oxo-2-thioxoimidazolidin-1-yl]-2-(trifluormethyl)benzonitril | 3 | 1,10 (s, 3H); 1,18 (s, b, 3H); 1,29-1,47 (b, 4H); 1,58 (s, 6H); 1,48-1,91 (b, 9H); 2,30-2,65 (b, 3H); 2,68-2,92 (b, 1 H); 2,95-3,18 (b, 1 H); 3,64-3,70 (m, 2H); 7,77 (dd, J=1,90 Hz, 8,01 Hz; 1H); 7,89 (d, J=1,68 Hz; 1H); 7,95 (d, J=8,43 Hz; 1 H) |
| | | | |
| | (2*R*)-α,α-Dimethylpyrrolidin-methanol, hergestellt aus D-Prolinmethylester nach K. Nakayama, W. J. Thompson, J. Am. Chem. Soc. 112, 6936-6942 (1990) | | |
| **43** | 4-[3-[7-[(2*R*)-2-(1-Hydroxy-1-methylethyl)pyrrolidin-1-yl]heptyl]-4,4-dimethyl-5-oxo-2-thioxoimidazolidin-1-yl]-2-(trifluormethyl)benzonitril | 4 | 1,09 (s, 3H); 1,17 (s, b, 3H); 1,29-1,44 (b, 6H); 1,58 (s, 6H); 1,46-1,90 (b, 9H); 2,33-2,64 (b, 3H); 2,68-2,86 (b, 1 H); 3,97-3,15 (b, 1 H); 3,64-3,69 (m, 2H); 7,77 (dd, J=2,11 Hz, 8,01 Hz; 1 H); 7,89 (d, J=2,1 0 Hz; 1 H); 7,95 (d, J=8,01 Hz; 1 H) |
| | | | |
| | (2*R*)-α,α-Dimethylpyrrolidin-methanol | | |
| **44** | 4-[3-[8-[(2*R*)-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril | 1 | 1,11 (s, 3H); 1,18 (s, b, 3H); 1,23-1,44 (b, 8H); 1,58 (s, 6H); 1,45-1,90 (b, 9H); 2,30-3,15 (b, 5H); 3,64-3,69 (m, 2H); 7,78 (dd, J=2,10 Hz, 8,43 Hz; 1H); 7,90 (d, J=2,11 Hz; 1 H); 7,95 (d, J=8,43 Hz; 1 H) |
| | | | |
| | (2*R*)-α,α-Dimethylpyrrolidin-methanol | | |
| **45** | 4-[3-[6-[(2*S*)-2-(1-Hydroxy-1-ethylpropyl)pyrrolidin-1-yl]hexyl]-4,4-dimethyl-5-oxo-2-thioxoimidazolidin-1-yl]-2-(trifluormethyl)benzonitril | 3 | 0,83 (t, J=8,22 Hz; 6H); 1,25-1,46 (b, 6H); 1,59 (s, 6H); 1,47-1,94 (b, 11H); 2,36-2,56 (b, 2H); 2,58-2,77 (b, 2H); 2,87-3,03 (b, 1 H); 3,64-3,70 (m, 2H); 7,77 (dd, J=2,11 Hz, 8,01 Hz; 1 H); 7,90 (d, J=2,11 Hz; 1 H); 7,95 (d, J=8,00 Hz; 1 H) |
| | | | |
| | (2*S*)-α,α-Diethylpyrrolidin- methanol, hergestellt aus L-Prolinmethylester nach K. Nakayama, W. J. Thompson, J. Am. Chem. Soc. 112, 6936-6942 (1990) | | |
| **46** | 4-[3-[7-[(2*S*)-2-(1-Hydroxy-1-ethylpropyl)pyrrolidin-1-yl]heptyl]-4,4-dimethyl-5-oxo-2-thioxoimidazolidin-1-yl]-2-(trifluormethyl)benzonitril | 4 | 0,80-0,91 (m, 6H); 1,22-1,47 (b, 8H); 1,46-1,95 (b, 11H); 1,58 (s, 6H); 2,30-3,30 (b, 5H); 3,64-3,70 (m, 2H); 7,77 (dd, J=2,11 Hz, 8,01 Hz; 1H); 7,89 (d, J=1,68 Hz; 1H); 7,95 (d, J=8,01 Hz; 1 H) |
| | | | |
| | (2*S*)-α,α-Diethylpyrrolidin-methanol | | |
| **47** | 4-[3-[8-[(2*S*)-2-(1-Hydroxy-1-ethylpropyl)pyrrolidin-1-yl]octyl]-4,4-dimethyl-5-oxo-2-thioxoimidazolidin-1-yl]-2-(trifluormethyl)benzonitril | 1 | 0,84 (t, J=7,80 Hz; 6H); 1,20-1,43 (b, 10H); 1,44-1,62 (b, 4H); 1,59 (s, 6H); 1,62-1,90 (b, 7H); 2,35-2,56 (b, 2H); 2,57-2,76 (b, 2H); 2,85-3,04 (b, 1 H); 3,64-3,70 (m, 2H); 7,78 (dd, J=2,32 Hz, 8,43 Hz; 1 H); 7,90 (d, J=1,68 Hz; 1 H); 7,95 (d, J=8,43 Hz; 1 H) |
| | | | |
| | (2*S*)-α,α-Diethylpyrrolidin-methanol | | |
| **48** | (*S*)-1-[7-[3-[4-Cyan-3-(trifluormethyl)phenyl]-5,5-dimethyl-4-oxo-2-thioxoimidazolidin-1-yl]heptyl]pyrrolidin-2-carbonsäuremethylester | 4 | 7,93 d (J=8 Hz, 1 H, aryl); 7,88 d (J=2,1 Hz, 1 H, aryl); 7,77 dd (*J*=8 Hz / 2,1 Hz, 1 H, aryl); 3,70 s (3H, OCH₃); 3,65 m (2H, CH₂N); 3,17 m (1 H, CH₂N); 3,11 1 dd (J=9 Hz / 6 Hz, 1 H, CHN); 2,64 m (1 H, CH₂N); 2,33 m (1 H, CH₂N); 2,31 m (1 H, CH₂N); 2,09 m (1 H, CH₂); 1,90 m (2H, CH₂); 1,82 m (2H, CH₂); 1,80 m (1H, CH₂); 1,56 s (6H, CH₃); 1,49 m (2H, CH₂); 1,35 m (6H, CH₂) |
| | | | |
| | L-Prolinmethylester, Hydrochlo-rid | | |
| **49** | (*S*)-1-[6-[3-[4-Cyan-3-(trifluormethyl)phenyl]-5,5-dimethyl-4-oxo-2-thioxoimidazolidin-1-yl]hexyl]pyrrolidin-4-carboxamid | 3 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 7,22 m (1 H, CONH₂); 5,61 m (1H, CONH₂); 3,66 m (2H, CH₂N); 3,18 m (1 H); 2,99 m (1 H); 2,64 m (1 H); 2,44 m (1H); 2,28 m (1H); 2,17 m (1H); 1,83 m (2H, CH₂); 1,80 m (3H, CH₂); 1,58 s (6H, CH₃); 1,51 m (2H, CH₂); 1,40 m (4H, CH₂) |
| | | | |
| | L-Prolinamid | | |
| **50** | (*S*)-1-[7-[3-[4-Cyan-3-(trifluormethyl)phenyl]-5,5-dimethyl-4-oxo-2-thioxoimidazolidin-1-yl]heptyl]pyrrolidin-4-carboxamid | 4 | 7,95 d (*J*=8 Hz, 1H, aryl); 7,89 d (*J*=2,1 1 Hz, 1 H, aryl); 7,77 dd (*J*=8 Hz / 2,1 Hz, 1 H, aryl); 7,26 m (1 H, CONH₂); 5,39 m (1H, CONH₂); 3,66 m (2H, CH₂N); 3,18 m (1 H); 3,00 m (1 H); 2,63 m (1 H); 2,45 m (1 H); 2,30 m (1 H); 2,18 m (1 H); 1,89 m (1 H, CH₂); 1,81 m (2H, CH₂); 1,80 m (2H, CH₂); 1,58 s (6H, CH₃); 1,52 m (2H, CH₂); 1,38 m (6H, CH₂) |
| | | | |
| | L-Prolinamid | | |
| **51** | (*S*)-1-[8-[3-[4-Cyan-3-(trifluormethyl)phenyl]-5,5-dimethyl-4-oxo-2-thioxoimidazolidin-1-yl]octyl]pyrrolidin-4-carboxamid | 1 | 7,95 d (*J*=8 Hz, 1H, aryl); 7,89 d (*J*=2,1 Hz, 1 H, aryl); 7,77 dd (*J*=8 Hz / 2,1 Hz, 1 H, aryl); 7,27 m (1 H, CONH₂); 5,46 m (1H, CONH₂); 3,66 m (2H, CH₂N); 3,19 m (1 H); 3,00 m (1 H); 2,63 m (1 H); 2,44 m (1H); 2,30 m (1H); 2,18 m (1H); 1,90 m (1H, CH₂); 1,81 m (3H, CH₂); 1,66 m (1 H, CH₂); 1,58 s (6H, CH₃); 1,49 m (2H, CH₂); 1,35 m (8H, CH₂) |
| | | | |
| | L-Prolinamid | | |
| **52** | (*R*)-1-[6-[3-[4-Cyan-3-(trifluormethyl)phenyl]-5,5-dimethyl-4-oxo-2-thioxoimidazolidin-1-yl]hexyl]pyrrolidin-4-carboxamid | 3 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 7,22 m (1 H, CONH₂); 5,57 m (1H, CONH₂); 3,66 m (2H, CH₂N); 3,18 m (1 H); 2,99 m (1 H); 2,64 m (1 H); 2,44 m (1H); 2,28 m (1H); 2,17 m (1H); 1,83 m (2H, CH₂); 1,80 m (3H, CH₂); 1,58 s (6H, CH₃); 1,51 m (2H, CH₂); 1,40 m (4H, CH₂) |
| | | | |
| | D-Prolinamid | | |
| **53** | (*R*)-1-[7-[3-[4-Cyan-3-(trifluormethyl)phenyl]-5,5-dimethyl-4-oxo-2-thioxoimidazolidin-1-yl]heptyl]pyrrolidin-4-carboxamid | 4 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 7,26 m (1 H, CONH₂); 5,36 m (1H, CONH₂); 3,66 m (2H, CH₂N); 3,18 m (1 H); 3,00 m (1 H); 2,63 m (1H); 2,45 m (1 H); 2,30 m (1 H); 2,18 m (1 H); 1,89 m (1 H, CH₂); 1,81 m (2H, CH₂); 1,80 m (2H, CH₂); 1,58 s (6H, CH₃); 1,52 m (2H, CH₂); 1,38 m (6H, CH₂) |
| | | | |
| | D-Prolinamid | | |
| **54** | (*R*)-1-[8-[3-[4-Cyan-3-(trifluormethyl)phenyl]-5,5-dimethyl-4-oxo-2-thioxoimidazolidin-1-yl]octyl]pyrrolidin-4-carboxamid | 1 | 7,95 d (*J*=8 Hz, 1 H, aryl); 7,89 d (*J*=2,1 Hz, 1 H, aryl); 7,77 dd (*J*=8 Hz / 2,1 Hz, 1 H, aryl); 7,24 m (1 H, CONH₂); 5,53 m (1H, CONH₂); 3,66 m (2H, CH₂N); 3,19 m (1 H); 3,00 m (1 H); 2,63 m (1 H); 2,44 m (1H); 2,30 m (1H); 2,18 m (1H); 1,90 m (1H, CH₂); 1,81 m (3H, CH₂); 1,66 m (1 H, CH₂); 1,58 s (6H, CH₃); 1,49 m (2H, CH₂); 1,35 m (8H, CH₂) |
| | | | |
| | D-Prolinamid | | |
| **55** | 4-[3-[6-[(2*S*)-4,4-Dimethyl-5-oxo-2-(pyrrolidin-1-ylmethyl)-pyrrolidin-1-yl]hexyl]-2-thioxo-imidazolidin-1-yl]-2-(trifluor-methyl)benzonitril | 3 | 1,34-1,49 (b, 6H); 1,58 (s, 6H); 1,65-1,89 (b, 8H); 1,90-2,09 (b, 2H); 2,10-2,37 (b, 2H); 2,38-2,78 (b, 7H); 2,84-3,01 (b, 1 H); 3,15-3,26 (b, 1 H); 3,64-3,70 (m, 2H); 7,77 (dd, J=2,11 Hz, 8,43 Hz; 1 H); 7,89 (d, J=1,68 Hz; 1 H); 7,95 (d, J=8,01 Hz; 1 H) |
| | | | |
| | (2*S*)-2-(Pyrrolidin-1-ylmethyl)-pyrrolidin, hergestellt aus L-Prolin nach M. Amedjkouh, P. Ahlberg, Tetrahedron: Asymmetry13, 2229-2234 (2002) | | |
| **56** | 4-[3-[7-[(2*S*)-4,4-Dimethyl-5-oxo-2-(pyrrolidin-1-ylmethyl)-pyrrolidin-1-yl]heptyl]-2-thioxo-imidazolidin-1-yl]-2-(trifluormethyl)benzonitril | 4 | 1,28-1,44 (b, 6H); 1,44-1,88 (b, 10H); 1,58 (s, 6H); 1,89-2,07 (b, 2H); 2,08-2,25 (b, 2H); 2,31-2,70 (b, 7H); 2,81-2,93 (b, 1 H); 3,11-3,21 (b, 1H); 3,64-3,69 (m, 2H); 7,77 (dd, J=2,11 Hz, 8,43 Hz; 1 H); 7,89 (d, J=1,68 Hz; 1 H); 7,95 (d, J=8,43 Hz; 1 H) |
| | | | |
| | (2*S*)-2-(Pyrrolidin-1-ylmethyl)-pyrrolidin | | |
| **57** | 4-[3-[8-[(2*S*)-4,4-Dimethyl-5-oxo-2-(pyrrolidin-1-ylmethyl)-pyrrolidin-1-yl]octyl]-2-thioxo-imidazolidin-1-yl]-2-(trifluormethyl)benzonitril | 1 | 1,22-1,43 (b, 8H); 1,48-1,60 (b, 2H); 1,58 (s, 6H); 1,65-1,87 (b, 8H); 1,92-2,08 (b, 2H); 2,09-2,29 (b, 2H); 2,33-2,62 (b, 6H); 2,62-2,76 (b, 1 H); 2,80-2,97 (b, 1 H); 3,11-3,25 (b, 1 H); 3,64-3,69 (m, 2H); 7,77 (dd, J=1,69 Hz, 8,01 Hz; 1 H); 7,89 (d, J=1,26 Hz; 1 H); 7,95 (d, J=8,43 Hz; 1H) |
| | | | |
| | (2*S*)-2-(Pyrrolidin-1-ylmethyl)-pyrrolidin | | |

### Beispiel 58

### (S)-1-[7-[3-[4-Cyan-3-(trifluormethyl)phenyl]-5,5-dimethyl-4-oxo-2-thioxoimidazolidin-1-yl]heptyl]pyrrolidin-2-carbonsäure hydrochlorid

20 mg der unter **48** hergestellten Verbindung werden mit einer Spatelspitze Kaliumcarbonat in 1 ml Methanol über Nacht bei Raumtemperatur gerührt. Das Reaktionsgemisch wird mit 4 molarer wässriger Salzsäure angesäuert und mit Ethylacetat extrahiert. Die organische Phase wird mit gesättigter Natriumchloridlösung gewaschen und im Vakuum eingeengt. Man erhält 12 mg der Titelverbindung.
¹H-NMR (300 MHz, CD₃OD) δ [ppm] = 8,15 d (*J*=8 Hz, 1 H, aryl); 8,10 d (*J*=2,1 Hz, 1H, aryl); 7,93 dd (*J*=8 Hz, 7 2,1 Hz, 1 H, aryl); 3,90 dd (*J*=9 Hz / 6 Hz, 1 H, CHN); 3,77 m (3H, CH₂N); 3,14 m (1H CH₂N); 3,26 m (1 H, CH₂N); 3,14 m (2H, CH₂N); 2,45 m (1 H, CH₂); 2,14 m (2H, CH₂); 1,98 m (1 H, CH₂); 1,89 m (2H, CH₂); 1,77 m (2H, CH₂); 1,60 s (6H, CH₃); 1,47 m (6H, CH₂).

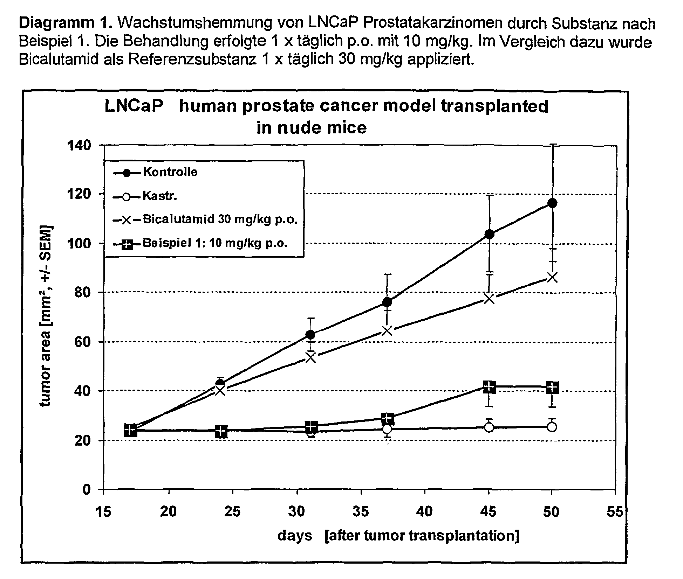

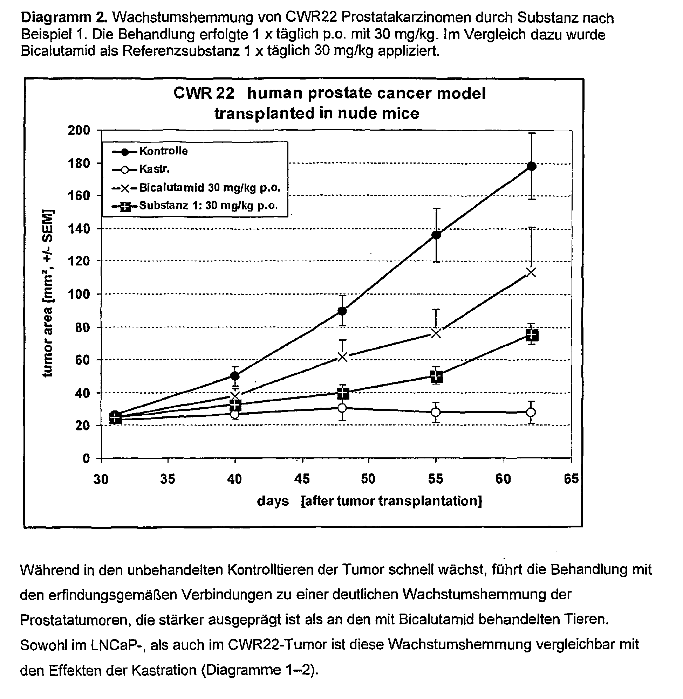

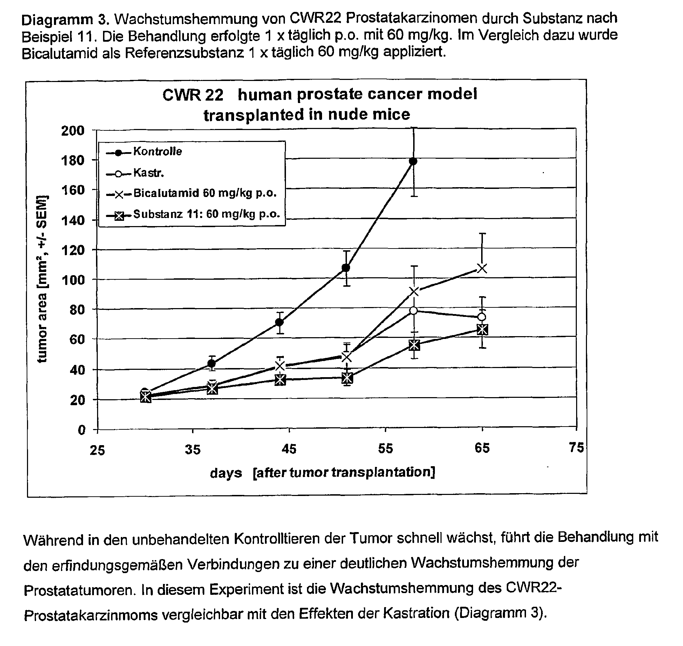

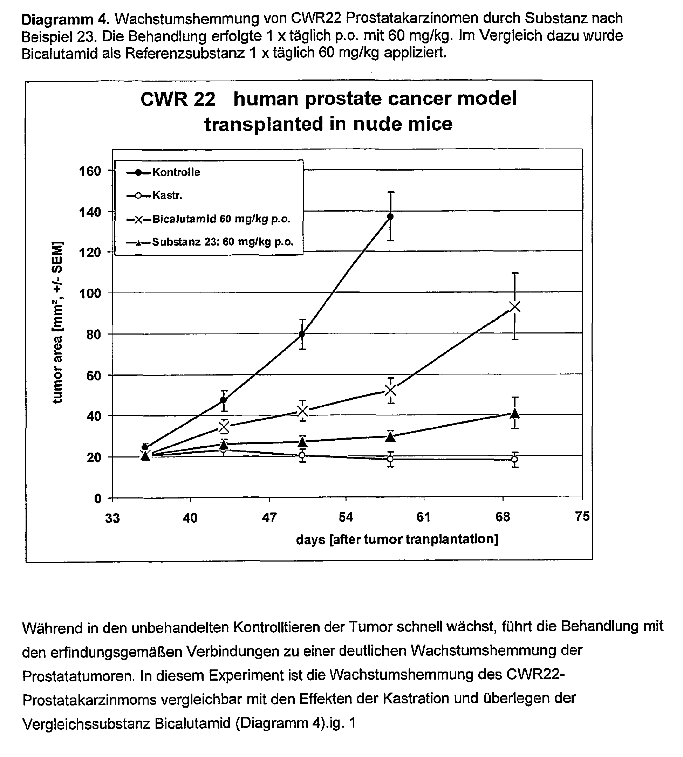

## Patentansprüche

1. Verbindungen der allgemeinen Formel I 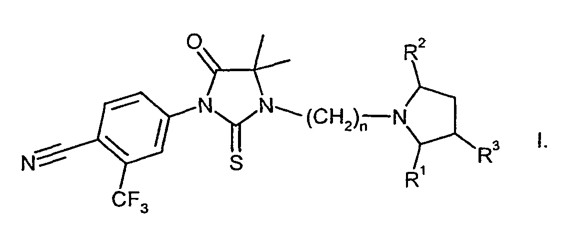 worin
n eine ganze Zahl zwischen 6 und 9,
R¹ und R² unabhängig voneinander ein Wasserstoffatom, eine unverzweigte C₁-C₄-Alkylgruppe, eine verzweigte C₃-C₅-Alkylgruppe, eine unverzweigte Hydroxy-C₁-C₄-alkylgruppe, eine verzweigte Hydroxy-C₃-C₅-alkylgruppe, eine unverzweigte C₁-C₄-Alkoxy-C₁-C₄-alkylgruppe, eine verzweigte C₁-C₄-Alkoxy-C₃-C₅-alkylgruppe, eine unverzweigte C₁-C₄-Alkanoyloxy-C₁-C₄-alkylgruppe, eine verzweigte C₁-C₄-Alkanoyloxy-C₃-C₅-alkylgruppe, eine (Pyrrolidin-1-yl)methylgruppe, eine Carboxygruppe, eine C₁-C₄-Alkoxycarbonylgruppe oder eine Aminocarbonylgruppe,
oder
R¹ und R² gemeinsam eine 2-Hydroxypropan-1,3-diylbrücke,
R³ ein Wasserstoffatom oder eine Hydroxygruppe bedeuten,
sowie deren pharmakologisch verträgliche Salze.

2. Verbindungen der allgemeinen Formel I nach Anspruch 1, **dadurch gekennzeichnet, dass** R¹ ein Wasserstoffatom, eine Hydroxymethyl-, eine Aminocarbonyl- oder eine Methoxymethylgruppe; R² und R³ jeweils ein Wasserstoffatom darstellen.

3. Verbindungen der allgemeinen Formel I nach Anspruch 1, **dadurch gekennzeichnet, dass** R¹ ein Wasserstoffatom, eine Methylgruppe; R² und R³ jeweils ein Wasserstoffatom darstellen.

4. Verbindungen der allgemeinen Formel I nach Anspruch 1, **dadurch gekennzeichnet, dass** R¹ und R² zusammen eine 2-Hydroxypropan-1,3-diylbrücke; R³ ein Wasserstoffatom darstellen.

5. Verbindungen nach einem der vorherigen Ansprüche, nämlich
**1** 4-[4,4-Dimethyl-5-oxo-3-[8-(pyrrolidin-1-yl)octyl]-2-thioxoimidazolidin-1-yl]-2-(trifluormethyl)benzonitril
**2** 4-[4,4-Dimethyl-5-oxo-3-[8-(pyrrolidin-1-yl)octyl]-2-thioxoimidazolidin-1-yl]-2-(trifluormethyl)benzonitril hydrochlorid
**3** 4-[4,4-Dimethyl-5-oxo-3-[6-(pyrrolidin-1-yl)hexyl]-2-thioxoimidazolidin-1-yl]-2-(trifluormethyl)benzonitril
**4** 4-[4,4-Dimethyl-5-oxo-3-[7-(pyrrolidin-1-yl)heptyl]-2-thioxoimidazolidin-1-yl]-2-(trifluormethyl)benzonitril
**5** 4-[4,4-Dimethyl-5-oxo-3-[9-(pyrrolidin-1-yl)nonyl]-2-thioxoimidazolidin-1-yl]-2-(trifluormethyl)benzonitril
**6** 4-[3-[6-[(2*R*)-2-(Hydroxymethyl)pyrrolidin-1-yl]hexyl]-4,4-dimethyl-5-oxo-2-thioxo-imidazolidin-1-yl]-2-(trifluormethyl)benzonitril
**7** 4-[3-[6-[(2*S*)-2-(Hydroxymethyl)pyrrolidin-1-yl]hexyl]-4,4-dimethyl-5-oxo-2-thioxoimidazolidin-1-yl]-2-(trifluormethyl)benzonitril
**8** 4-[3-[7-[(2*R*)-2-(Hydroxymethyl)pyrrolidin-1-yl]heptyl]-4,4-dimethyl-5-oxo-2-thioxo-imidazolidin-1-yl]-2-(trifluormethyl)benzonitril
**9** 4-[3-[7-[(2*S*)-2-(Hydroxymethyl)pyrrolidin-1-yl]heptyl]-4,4-dimethyl-5-oxo-2-thioxoimidazolidin-1-yl]-2-(trifluormethyl)benzonitril
**10** 4-[3-[8-[(2*R*)-2-(Hydroxymethyl)pyrrolidin-1-yl]octyl]-4,4-dimethyl-5-oxo-2-thioxo-imidazolidin-1-yl]-2-(trifluormethyl)benzonitril
**11** 4-[3-[8-[(2*S*)-2-(Hydroxymethyl)pyrrolidin-1-yl]octyl]-4,4-dimethyl-5-oxo-2-thioxoimidazolidin-1-yl]-2-(trifluormethyl)benzonitril
**12** 4-[3-[9-[(2*R*)-2-(Hydroxymethyl)pyrrolidin-1-yl]nonyl]-4,4-dimethyl-5-oxo-2-thioxoimidazolidin-1-yl]-2-(trifluormethyl)benzonitril
**13** 4-[3-[9-[(2*S*)-2-(Hydroxymethyl)pyrrolidin-1-yl]nonyl]-4,4-dimethyl-5-oxo-2-thioxoimidazolidin-1-yl]-2-(trifluormethyl)benzonitril
**14** 4-[3-[6-[(2*R*)-2-(Methoxymethyl)pyrrolidin-1-yl]hexyl]-4,4-dimethyl-5-oxo-2-thioxo-imidazolidin-1-yl]-2-(trifluormethyl)benzonitril
**15** 4-[3-[6-[(2*S*)-2-(Methoxymethyl)pyrrolidin-1-yl]hexyl]-4,4-dimethyl-5-oxo-2-thioxa-imidazolidin-1-yl]-2-(trifluormethyl)benzonitril
**16** 4-[3-[7-[(2*R*)-2-(Methoxymethyl)pyrrolidin-1-yl]heptyl]-4,4-dimethyl-5-oxo-2-thioxoimidazolidin-1-yl]-2-(trifluormethyl)benzonitril
**17** 4-[3-[7-[(2*S*)-2-(Methoxymethyl)pyrrolidin-1-yl]heptyl]-4,4-dimethyl-5-oxo-2-thioxoimidazolidin-1-yl]-2-(trifluormethyl)benzonitril
**18** 4-[3-[8-[(2*R*)-2-(Methoxymethyl)pyrrolidin-1-yl]octyl]-4,4-dimethyl-5-oxo-2-thioxoimidazolidin-1-yl]-2-(trifluormethyl)benzonitril
**19** 4-[3-[8-[(2*S*)-2-(Methoxymethyl)pyrrolidin-1-yl]octyl]-4,4-dimethyl-5-oxo-2-thioxo-imidazolidin-1-yl]-2-(trifluormethyl)benzonitril
**20** 4-[3-[9-[(2*R*)-2-(Methoxymethyl)pyrrolidin-1-yl]nonyl]-4,4-dimethyl-5-oxo-2-thioxo-imidazolidin-1-yl]-2-(trifluormethyl)benzonitril
**21** 4-[3-[9-[(2*S*)-2-(Methoxymethyl)pyrrolidin-1-yl]nonyl]-4,4-dimethyl-5-oxo-2-thioxoimidazolidin-1-yl]-2-(trifluormethyl)benzonitril
**22** 4-[3-[6-(3-Hydroxy-8-azabicyclo[3.2.1]oct-8-yl)hexyl]-4,4-dimethyl-5-oxo-2-thioxoimidazolidin-1-yl]-2-(trifluormethyl)benzonitril
**23** 4-[3-[7-(3-Hydroxy-8-azabicyclo[3.2.1]oct-8-yl)heptyl]-4,4-dimethyl-5-oxo-2-thioxoimidazolidin-1-yl]-2-(trifluormethyl)benzonitril
**24** 4-[3-[8-(3-Hydroxy-8-azabicyclo[3.2.1]oct-8-yl)octyl]-4,4-dimethyl-5-oxo-2-thioxoimidazolidin-1-yl]-2-(trifluormethyl)benzonitril
**25** 4-[3-[9-(3-Hydroxy-8-azabicyclo[3.2.1]oct-8-yl)nonyl]-4,4-dimethyl-5-oxo-2-thioxoimidazolidin-1-yl]-2-(trifluormethyl)benzonitril
**26** 4-[3-[6-[(*R*)-3-Hydroxypyrrolidin-1-yl]hexyl]-4,4-dimethyl-5-oxo-2-thioxoimidazolid-in-1-yl]-2-(trifluormethyl)benzonitril
**27** 4-[3-[7-[(*R*)-3-Hydroxypyrrolidin-1-yl]heptyl]-4,4-dimethyl-5-oxo-2-thioxoimidazolidin-1-yl]-2-(trifluormethyl)benzonitril
**28** 4-[3-[8-[(*R*)-3-Hydroxypyrrolidin-1-yl]octyl]-4,4-dimethyl-5-oxo-2-thioxoimidazolidin-1-yl]-2-(trifluormethyl)benzonitril
**29** 4-[3-[9-[(*R*)-3-Hydroxypyrrolidin-1-yl]nonyl]-4,4-dimethyl-5-oxo-2-thioxoimidazolidin-1-yl]-2-(trifluormethyl)benzonitril
**30** 4-[3-[6-[(*S*)-3-Hydroxypyrrolidin-1-yl]hexyl]-4,4-dimethyl-5-oxo-2-thioxoimidazolidin-1-yl]-2-(trifluormethyl)benzonitril
**31** 4-[3-[7-[(*S*)-3-Hydroxypyrrolidin-1-yl]heptyl]-4,4-dimethyl-5-oxo-2-thioxoimidazolidin-1-yl]-2-(trifluormethyl)benzonitril
**32** 4-[3-[8-[(*S*)-3-Hydroxypyrrolidin-1-yl]octyl]-4,4-dimethyl-5-oxo-2-thioxoimidazolidin-1-yl]-2-(trifluormethyl)benzonitril
**33** 4-[3-[9-[(*S*)-3-Hydroxypyrrolidin-1-yl]nonyl]-4,4-dimethyl-5-oxo-2-thioxoimidazolidin-1-yl]-2-(trifluormethyl)benzonitril
**34** 4-[4,4-Dimethyl-3-[6-(2-methylpyrrolidin-1-yl)hexyl]-5-oxo-2-thioxoimidazolidin-1-yl]-2-(trifluormethyl)benzonitril
**35** 4-[4,4-Dimethyl-3-[7-(2-methylpyrrolidin-1-yl)heptyl]-5-oxo-2-thioxoimidazolidin-1-yl]-2-(trifluormethyl)benzonitril
**36** 4-[4,4-Dimethyl-3-[8-(2-methylpyrrolidin-1-yl)octyl]-5-oxo-2-thioxoimidazolidin-1-yl]-2-(trifluormethyl)benzonitril
**37** 4-[3-[8-[(2*R*,5*S*)-rel-2,5-Dimethylpyrrolidin-1-yl]octyl]-4,4-dimethyl-5-oxo-2-thioxoimidazolidin-1-yl]-2-(trifluormethyl)benzonitril
**38** 4-[3-[6-[(2*S*)-2-(1-Hydroxy-1-methylethyl)pyrrolidin-1-yl]hexyl]-4,4-dimethyl-5-oxo-2-thioxoimidazolidin-1-yl]-2-(trifluormethyl)benzonitril
**39** 4-[3-[7-[(2*S*)-2-(1-Hydroxy-1-methylethyl)pyrrolidin-1-yl]heptyl]-4,4-dimethyl-5-oxo-2-thioxoimidazolidin-1-yl]-2-(trifluormethyl)benzonitril
**40** 4-[3-[8-[(2*S*)-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril
**41** 4-[3-[9-[(2*S*)-2-(1-Hydroxy-1-methylethyl)pyrrolidin-1-yl]nonyl]-4,4-dimethyl-5-oxo-2-thioxoimidazolidin-1-yl]-2-(trifluormethyl)benzonitril
**42** 4-[3-[6-[(2*R*)-2-(1-Hydroxy-1-methylethyl)pyrrolidin-1-yl]hexyl]-4,4-dimethyl-5-oxo-2-thioxoimidazolidin-1-yl]-2-(trifluormethyl)benzonitril
**43** 4-[3-[7-[(2*R*)-2-(1-Hydroxy-1-methylethyl)pyrrolidin-1-yl]heptyl]-4,4-dimethyl-5-oxo-2-thioxoimidazolidin-1-yl]-2-(trifluormethyl)benzonitril
**44** 4-[3-[8-[(2*R*)-2-(1-Hydroxy-1-methylethyl)pyrrolidin-1-yl]octyl]-4,4-dimethyl-5-oxo-2-thioxoimidazolidin-1-yl]-2-(trifluormethyl)benzonitril
**45** 4-[3-[6-[(2*S*)-2-(1-Hydroxy-1-ethylpropyl)pyrrolidin-1-yl]hexyl]-4,4-dimethyl-5-oxo-2-thioxoimidazolidin-1-yl]-2-(trifluormethyl)benzonitril
**46** 4-[3-[7-[(2*S*)-2-(1-Hydroxy-1-ethylpropyl)pyrrolidin-1-yl]heptyl]-4,4-dimethyl-5-oxo-2-thioxoimidazolidin-1-yl]-2-(trifluormethyl)benzonitril
**47** 4-[3-[8-[(2*S*)-2-(1-Hydroxy-1-ethylpropyl)pyrrolidin-1-yl]octyl]-4,4-dimethyl-5-oxo-2-thioxoimidazolidin-1-yl]-2-(trifluormethyl)benzonitril
**48** (*S*)-1-[7-[3-[4-Cyan-3-(trifluormethyl)phenyl]-5,5-dimethyl-4-oxo-2-thioxoimidazolidin-1-yl]heptyl]pyrrolidin-2-carbonsäuremethylester
**49** (*S*)-1-[6-[3-[4-Cyan-3-(trifluormethyl)phenyl]-5,5-dimethyl-4-oxo-2-thioxoimidazol-idin-1-yl]hexyl]pyrrolidin-4-carboxamid
**50** (*S*)-1-[7-[3-[4-Cyan-3-(trifluormethyl)phenyl]-5,5-dimethyl-4-oxo-2-thioxoimidazolidin-1-yl]heptyl]pyrrolidin-4-carboxamid
**51** (*S*)-1-[8-[3-[4-Cyan-3-(trifluormethyl)phenyl]-5,5-dimethyl-4-oxo-2-thioxoimidazol-idin-1-yl]octyl]pyrrolidin-4-carboxamid
**52** (*R*)-1-[6-[3-[4-Cyan-3-(trifluormethyl)phenyl]-5,5-dimethyl-4-oxo-2-thioxoimidazolidin-1-yl]hexyl]pyrrolidin-4-carboxamid
**53** (*R*)-1-[7-[3-[4-Cyan-3-(trifluormethyl)phenyl]-5,5-dimethyl-4-oxo-2-thioxoimidazol-idin-1-yl]heptyl]pyrrolidin-4-carboxamid
**54** (*R*)-1-[8-[3-[4-Cyan-3-(trifluormethyl)phenyl]-5,5-dimethyl-4-oxo-2-thioxoimidazol-idin-1-yl]octyl]pyrrolidin-4-carboxamid
**55** 4-[3-[6-[(2*S*)-4,4-Dimethyl-5-oxo-2-(pyrrolidin-1-ylmethyl)pyrrolidin-1-yl]hexyl]-2-thioxoimidazolidin-1-yl]-2-(trifluormethyl)benzonitril
**56** 4-[3-[7-[(2*S*)-4,4-Dimethyl-5-oxo-2-(pyrrolidin-1-ylmethyl)pyrrolidin-1-yl]heptyl]-2-thioxoimidazolidin-1-yl]-2-(trifluormethyl)benzonitril
**57** 4-[3-[8-[(2*S*)-4,4-Dimethyl-5-oxo-2-(pyrrolidin-1-ylmethyl)pyrrolidin-1-yl]octyl]-2-thioxoimidazolidin-1-yl]-2-(trifluormethyl)benzonitril
**58** (*S*)-1-[7-[3-[4-Cyan-3-(trifluormethyl)phenyl]-5,5-dimethyl-4-oxo-2-thioxoimidazolidin-1-yl]heptyl]pyrrolidin-2-carbonsäure hydrochlorid

6. Pharmazeutische Zusammensetzungen enthaltend mindestens eine Verbindung der allgemeinen Formel I oder deren pharmakologisch verträgliche Salze nach einem der vorherigen Ansprüche, zusammen mit pharmazeutisch verträglichen Hilfs- und/oder Trägerstoffen.

7. Verwendung der Verbindungen der allgemeinen Formel I nach einem der vorherigen Ansprüche zur Herstellung eines Arzneimittels zur Behandlung oder Prophylaxe von Krankheiten des menschlichen oder tierischen Körpers, die sich durch die Hemmung des Androgenrezeptors beeinflussen lassen.

8. Verwendung nach Anspruch 7 **dadurch gekennzeichnet, dass** die vorzubeugenden oder zu behandelnden Krankheiten androgenabhängige proliferative Erkrankungen sind.

9. Verwendung nach den Ansprüchen 7 oder 8 **dadurch gekennzeichnet, dass** die zu behandelnden Krankheiten Tumorerkrankungen sind.

10. Verwendung nach einem der Ansprüche 7. bis 9 **dadurch gekennzeichnet, dass** die zu behandelnde Krankheit Prostatakarzinom ist.

11. Verwendung nach Anspruch 7 **dadurch gekennzeichnet, dass** die vorzubeugenden oder zu behandelnden Krankheiten androgenabhängige nicht-proliferative Erkrankungen sind.

12. Verwendung nach dem Anspruch 11 **dadurch gekennzeichnet, dass** die vorzubeugenden oder zu behandelnden Krankheiten androgenetische Alopezie, Hirsutismus oder Akne sind.

13. Verwendung nach den Ansprüchen 7 oder 8 **dadurch gekennzeichnet, dass** die Erkrankung benigne Prostatahyperplasie ist.

14. Verfahren zur Herstellung der erfindungsgemäßen Verbindungen der allgemeinen Formel I nach Anspruch 1 **dadurch gekennzeichnet, dass** Verbindungen der allgemeinen Formel II 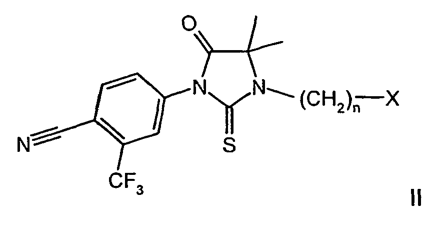 worin
n eine ganze Zahl zwischen 6 und 9,
X eine Abgangsgruppe,
mit Verbindungen der allgemeinen Formel III 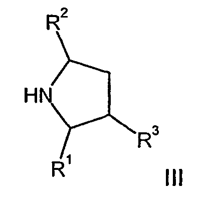 worin
R¹ und R² unabhängig voneinander ein Wasserstoffatom, eine unverzweigte C₁-C₄-Alkylgruppe, eine verzweigte C₃-C₅-Alkylgruppe, eine unverzweigte Hydroxy-C₁-C₄-alkylgruppe, eine verzweigte Hydroxy-C₃-C₅-alkylgruppe, eine unverzweigte C₁-C₄-Alkoxy-C₁-C₄-alkylgruppe, eine verzweigte C₁-C₄-Alkoxy-C₃-C₅-alkylgruppe, eine unverzweigte C₁-C₄-Alkanoyloxy-C₁-C₄-alkylgruppe, eine verzweigte C₁-C₄-Alkanoyloxy-C₃-C₅-alkylgruppe, eine (Pyrrolidin-1-yl)methylgruppe, eine Carboxygruppe, eine C₁-C₄-Alkoxycarbonylgruppe oder eine Aminocarbonylgruppe,
oder
R¹ und R² gemeinsam eine 2-Hydroxypropan-1,3-diylbrücke;
R³ ein Wasserstoffatom oder eine Hydroxygruppe
bedeuten können,
in Gegenwart einer organischen Base umgesetzt werden.

## Claims

1. Compounds of the general formula I 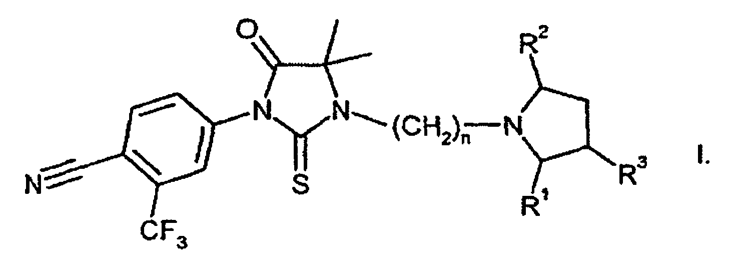 in which
n means an integer between 6 and 9,
R¹ and R², independently of one another, mean a hydrogen atom, an unbranched C₁-C₄-alkyl group, a branched C₃-C₅-alkyl group, an unbranched hydroxy-C₁-C₄-alkyl group, a branched hydroxy-C₃-C₅-alkyl group, an unbranched C₁-C₄-alkoxy-C₁-C₄-alkyl group, a branched C₁-C₄-alkoxy-C₃-C₅-alkyl group, an unbranched C₁-C₄-alkanoyloxy-C₁-C₄-alkyl group, a branched C₁-C₄-alkanoyloxy-C₃-C₅-alkyl group, a (pyrrolidin-1-yl)methyl group, a carboxy group, a C₁-C₄-alkoxycarbonyl group or an aminocarbonyl group,
or
R¹ and R² together mean a 2-hydroxypropane-1,3-diyl bridge;
R³ means a hydrogen atom or a hydroxy group,
and also their pharmacologically acceptable salts.

2. Compounds of the general formula I according to Claim 1, **characterized in that** R¹ represents a hydrogen atom, a hydroxymethyl group, an aminocarbonyl group or a methoxymethyl group; R² and R³ each represent a hydrogen atom.

3. Compounds of the general formula I according to Claim 1, **characterized in that** R¹ represents a hydrogen atom or a methyl group; R² and R³ each represent a hydrogen atom.

4. Compounds of the general formula I according to Claim 1, **characterized in that** R¹ and R² together represent a 2-hydroxypropane-1,3-diyl bridge; R³ represents a hydrogen atom.

5. Compounds according to any one of the preceding claims, namely
**1** 4-[4,4-dimethyl-5-oxo-3-[8-(pyrrolidin-1-yl)octyl]-2-thioxoimidazolidin-1-yl]-2-(trifluoromethyl)benzonitrile
**2** 4-[4,4-dimethyl-5-oxo-3-[8-(pyrrolidin-1-yl)octyl]-2-thioxoimidazolidin-1-yl]-2-(bifluoromethyl)benzonitrile hydrochloride
**3** 4-[4,4-dimethyl-5-oxo-3-[6-(pyrrolidin-1-yl)hexyl]-2-thioxoimidazolidin-1-yl)-2-(trifluoromethyl)benzonitrile
**4** 4-[4,4-dimethyl-5-oxo-3-[7-(pyrrolidin-1-yl)heptyl]-2-thioxoimidazolidin-1-yl-2-4 (trifluoromethyl)benzonitrile
**5** 4-[4,4-dimethyl-5-oxo-3-[9-(pyrrolidin-1-yl)nonyl]-2-thioxoimidazolidin-1-yl-2-(trifluoromethyl)benzonitrile
**6** 4-[3-[6-[(2*R*)-2-(hydroxymethyl)pyrrolidin-1-yl]hexyl]-4,4-dimethyl-5-oxo-2-thioxo-6 imidazolidin-1-yl-2-(trifluoromethyl)benzonitrile
**7** 4-[3-[6-[(2*S*)-2-(hydroxymethyl)pyrrolidin-1-yl]hexyl]-4,4-dimethyl-5-oxo-2-thioxoimidazolidin-1-yl]-2-(trifluoromethyl)benzonitrile
**8** 4-[3-[7-[(2*R*)-2-(hydroxymethyl)pyrrolidin-1-yl]heptyl]-4.4-dimethyl-5-oxo-2-thioxo-imidazolidin-1-yl]-2-(trifluoromethyl)benzonitrile
**9** 4-[3-[7-[(2*S*)-2-(hydroxymethyl)pyrrolidin-1-yl]heptyl]-4,4-dimethyl-5-oxo-2-thioxoimidazolidin-1-yl]-2-(trifluoromethyl)benzonitrile
**10** 4-[3-[8-[(2*R*)-2-(hydroxymethyl)pyrrolidin-1-yl]octyl]-4,4-dimethyl-5-oxo-2-thioxoimidazolidin-1-yl]-2-(trifluoromethyl)benzonitrile
**11** 4-[3-[8-[(2*S*)-2-(hydroxymethyl)pyrrolidin-1-yl]octyl]-4,4-dimethyl-5-oxo-2-thioxoimidazolidin-1-yl]-2-(trifluoromethyl)benzonitrile
**12** 4-[3-[9-[(2*R*)-2-(hydroxymethyl)pyrrolidin-1-yl]nonyl]-4,4-dimethyl-5-oxo-2-thioxoimidazolidin-1-yl]-2-(trifluoromethyl)benzonitrile
**13** 4-[3-[9-[(2*S*)-2-(hydroxymethyl)pyrrolidin-1-yl]nonyl]-4,4-dimethyl-5-oxo-2-thioxoimidazolidin-1-yl]-2-(trifluoromethyl)benzonitrile
**14** 4-[3-[6[(2*R*)-2-(methoxymethyl)pyrrolidin-1-yl]hexyl]-4,4-dimethyl-5-oxo-2-thioxoimidazolidin-1-yl]-2-(trifluoromethyl)benzonitrile
**15** 4-[3-[6-[(2*S*)-2-(methoxymethyl)pyrrolidin-1-yl]hexyl]-4,4-dimethyl-5-oxo-2-thioxoimidazolidin-1-yl]-2-(trifluoromethyl)benzonitrile
**16** 4-[3-[7-[(2*R*)-2-(methoxymethyl)pyrrolidin-1-yl]heptyl]-4,4-dimethyl-5-oxo-2-thioxoimidazolidin-1-yl]-2-(trifluoromethyl)benzonitrile
**17** 4-[3-[7-[(2*S*)-2-(methoxymethyl)pyrrolidin-1-yl]heptyl]-4,4-dimethyl-5-oxo-2-thioxoimidazolidin-1-yl]-2-(trifluoromethyl)benzonitrile
**18** 4-[3-[8-[(2*R*)-2-(methoxymethyl)pyrrolidin-1-yl]octyl]-4,4-dimethyl-5-oxo-2-thioxoimidazolidin-1-yl]-2-(trifluoromethyl)benzonitrile
**19** 4-[3-[8-[(2*S*)-2-(methoxylmethyl)pyrrolidin-1-yl]octyl]-4,4-dimethyl-5-oxo-2-thioxoimidazolidin-1-yl]-2-(trifluoromethyl)benzonitrile
**20** 4-[3-[9-[(2*R*)-2-(methoxymethyl)pyrrolidin-1-yl]nonyl]-4,4-dimethyl-5-oxo-2-thioxoimidazolidin-1-yl]-2-(trifluoromethyl)benzonitrile
**21** 4-[3-[9-[(2S)-2-(methoxymethyl)pyrrolidin-1-yl]nonyl]-4,4-dimethyl-5-oxo-2-thioxoimidazolidin-1-yl]-2-(trifluoromethyl)benzonitrile
**22** 4-[3-[6-(3-hydroxy-8-azabicyclo[3.2.1]oct-8-yl)hexyl]-4,4-dimethyl-5-oxo-2-thioxoimidazolidin-1-yl]-2-(trifluoromethyl)benzonitrile
**23** 4-[3-[7-(3-hydroxy-8-azabicyclo[3.2.1]oct-8-yl)heptyl]-4,4-dimethyl-5-oxo-2-thioxoimidazolidin-1-yl]-2-(trifluoromethyl)benzonitrile
**24** 4-[3-[8-(3-hydroxy-8-azabicyclo[3.2.1]oct-8-yl)octyl]-4,4-dimethyl-5-oxo-2-thioxoimidazolidin-1-yl]-2-(trifluoromethyl)benzonitrile
**25** 4-[3-[9-(3-hydroxy-8-azabicydo[3.2.1]oct-8-yl)nonyl]-4,4-dimethyl-5-oxo-2-thioxoimidazolidin-1-yl]-2-(trifluoromethyl)benzonitrile
**26** 4-[3-[6-[(*R*)-3-hydroxypyrrolidin-1-yl]-hexyl]-4,4-dimethyl-5-oxo-2-thioxoimidazolidin-1-yl]-2-(trifluoromethyl)benzonitrile
**27** 4-[3-[7-[(*R*)-3-hydroxypyrrolidin-1-yl]heptyl]-4,4-dimethyl-5-oxo-2-thioxoimidazolidin-1-yl]-2-(trifluoromethyl)benzonitrile
**28** 4-[3-[8-[(*R*)-3-hydroxypyrrolidin-1-yl]octyl]-4,4-dimethyl-5-oxo-2-thioxoimidazolidin-1-yl]-2-(trifluoromethyl)benzonitrile
**29** 4-[3-[9-[(*R*)-3-hydroxypyrrolidin-1-yl]nonyl]-4,4-dimethyl-5-oxo-2-thioxoimidazolid-in-1-yl]-2-(trifluoromethyl)benzonitrile
**30** 4-[3-[6-[(*S*)-3-hydroxypyrrolidin-1-yl]hexyl]-4,4-dimethyl-5-oxo-2-thioxoimidazolidin-1-yl]-2-(trifluoromethyl)benzonitrile
**31** 4-[3-[7-[(*S*)-3-hydroxypyrrolidin-1-yl]heptyl]-4,4-dimethyl-5-oxo-2-thioxoimidazolidin-1-yl]-2-(trifluoromethyl)benzonitrile
**32** 4-[3-[8-[(*S*)-3-hydroxypyrrolidin-1-yl]octyl]-4,4-dimethyl-5-oxo-2-thioxoimidazolidin-1-yl]-2-(trifluoromethyl)benzonitrile
**33** 4-[3-[9-[(*S*)-3-hydroxypyrrolidin-1-yl]nonyl]-4,4-dimethyl-5-oxo-2-thioxoimidazolidin-1-yl]-2-(trifluoromethyl)benzonitrile
**34** 4-[4,4-dimethyl-3-[6-(2-methylpyrrolidin-1-yl)hexyl]-5-oxo-2-thioxoimidazolidin-1-yl]-2-(trifluoromethyl)benzonitrile
**35** 4-[4,4-dimethyl-3-[7-(2-methylpyrrolidin-1-yl)heptyl]-5-oxo-2-thioxoimidazolidin-1-yl]-2-(trifluoromethyl)benzonitrile
**36** 4-[4,4-dimethyl-3-[8-(2-methylpyrrolidin-1-yl)octyl]-5-oxo-2-thioxoimidazolidin-1-yl]-2-(trifluoromethyl)benzonitrile
**37** 4-[3-[8-[(2*R*,5*S*)-*rel*-2,5-dimethylpyrrolidin-1-yl]octyl]-4,4-dimethyl-5-oxo-2-thioxo-imidazolidin-1-yl]-2-(trifluoromethyl)benzonitrile
**38** 4-[3-[6-[(2*S*)-2-(1-hydroxy-1-methylethyl)pyrrolidin-1-yl]hexyl]-4,4-dimethyl-5-oxo-2-thioxoimidazolidin-1-yl]-2-(trifluoromethyl)benzonitrile
**39** 4-[3-[7-[(2*S*)-2-(1-hydroxy-1-methylethyl)pyrrolidin-1-yl]heptyl]-4,4-dimethyl-5-oxo-2-thioxoimidazolidin-1-yl]-2-(trifluoromethyl)benzonitrile
**40** 4-[3-[8-[(2*S*)-2-(1-hydroxy-1-methylethyl)pyrrolidin-1-yl]octyl]-4,4-dimethyl-5-oxo-2-thioxoimidazolidin-1-yl]-2-(trifluoromethyl)benzonitrile
**41** 4-[3-[9-[(2*S*)-2-(1-hydroxy-1-methylethyl)pyrrolidin-1-yl]nonyl]-4,4-dimethyl-5-oxo-2-thioxoimidazolidin-1-yl]-2-(trifluoromethyl)benzonitrile
**42** 4-[3-[6-[(2*R*)-2-(1-hydroxy-1-methylethyl)pyrrolidin-1-yl]hexyl-4,4-dimethyl-5-oxo-2-thioxoimidazolidin-1-yl]-2-(trifluoromethyl)benzonitrile
**43** 4-[3-[7-[(2*R*)-2-(1-hydroxy-1-methylethyl)pyrrolidin-1-yl]heptyl]-4,4-dimethyl-5-oxo-2-thioxoimidazolidin-1-yl]-2-(trifluoromethyl)benzonitrile
**44** 4-[3-[8[(2*R*)-2-(1-hydroxy-1-methylethyl)pyrrolidin-1-yl)octyl]-4,4-dimethyl-5-oxo-2-thioxoimidazolidin-1-yl]-2-(trifluoromethyl)benzonitrile
**45** 4-[3-[6-[(2*S*)-2-(1-hydroxy-1-ethylpropyl)pyrrolidin-1-yl]hexyl]-4,4-dimethyl-5-oxo-2-thioxoimidazolidin-1-yl]-2-(trifluoromethyl)benzonitrile
**46** 4-[3-[7-[(2*S*)-2-(1-hydroxy-1-ethyl)propyl)pyrrolidin-1-yl]heptyl-4,4-dimethyl-5-oxo-2-thioxoimidazolidin-1-yl]-2-(trifluoromethyl)benzonitrile
**47** 4-[3-[8-[(2*S*)-2-(1-hydroxy-1-ethylpropyl)pyrrolidin-1-yl]octyl]-4,4-dimethyl-5-oxo-2-thioxoimidazolidin-1-yl]-2-(trifluoromethyl)benzonitrile
**48** methyl(*S*)-1-[7-[3-[4-cyano-3-(trifluoromethyl)phenyl]-5,5-dimethyl-4-oxo-2-thioxoimidazol-idin-1-yl]heptyl]pyrrolidine-2-carboxylate
**49** (*S*)-1-[6-[3-[4-cyano-3-(trifluoromethyl)phenyl]-5,5-dimethyl-4-oxo-2-thioxoimidazolidin-1-yl]hexyl]pyrrolidine-4-carboxamide
**50** (*S*)-1-[7-[3-[4-cyano-3-(trifluoromethyl)phenyl]-5,5-dimethyl-4-oxo-2-thioxoimidazolidin-1-yl]heptyl]pyrrolidine-4-carboxamide
**51** (*S*)-1-[8-[3-[4-cyano-3-(trifluoromethyl)pheny]-5,5-dimethyl-4-oxo-2-thioxonidazolidin-1-yl]octyl]pyrrolidine-4-carboxamide
**52** (*R*)-1-[6-[3-[4-cyano-3-(trifluoromethyl)phenyl]-5,5-dimethyl-4-oxo-2-thioxonidazolidin-1-yl]hexyl]pyrrolidine-4-carboxamide
**53** (*R*)-1-[7-[3-[4-cyano-3-(trifluoromethyl)phenyl]-5,5-dimethyl-4-oxo-2-thioxonidazolidin-1-yl]heptyl]pyrrolidine-4-carboxamide
**54** (*R*)-1-[8-[3-[4-cyano-3-(trifluoromethyl)phenyl]-5,5-dimethyl-4-oxo-2-thioxoimiazolidin-1-yl]octyl]pyrrolidine-4-carboxamide
**55** 4-[3-[6-[(2*S*)-4,4-dimethyl-5-oxo-2-(pyrrolidin-1-ylmethyl)pyrrolidin-1-yl]hexyl]-2-thioxoimidazolidin-1-yl]-2-(trifluoromethyl)benzonitrile
**56** 4-[3-[7-[(2*S*)-4,4-dimethyl-5-oxo-2-(pyrrolidin-1-ylmethyl)pyrrolidin-1-yl]heptyl]-2-thioxoimidazolidin-1-yl]-2-(trifluoromethyl)benzonitrile
**57** 4-[3-[8-[(2*S*)-4,4-dimethyl-5-oxo-2-(pyrrolidin-1-ylmethyl)pyrrolidin-1-yl]octyl]-2-thioxoimidazolidin-1-yl]-2-(trifluoromethyl)benzonitrile
**58** (*S*)-1-[7-[3-[4-cyano-3-(trifluoromethyl)phenyl]-5,5-dimethyl-4-oxo-2-thioxoimidazolidin-1-yl]heptyl] pyrrolidine-2-carboxylic acid hydrochloride

6. Pharmaceutical compositions containing at least one compound of the general formula I or its pharmacologically acceptable salts according to any one of the preceding claims, together with pharmaceutically acceptable adjuvant and/or vehicle materials.

7. Use of the compounds of the general formula I according to any one of the preceding claims in the manufacture of a medicament for treatment or prophylaxis of diseases of the human or animal body which are influenceable through inhibition of the androgen receptor.

8. Use according to Claim 7, **characterized in that** the diseases to be prevented or treated are androgen-dependent proliferative diseases.

9. Use according to Claims 7 or 8, **characterized in that** the diseases to be treated are tumour diseases.

10. Use according to any one of Claims 8 to 9, **characterized in that** the disease to be treated is prostate carcinoma.

11. Use according to Claim 7, **characterized in that** the diseases to be prevented or treated are androgen-dependent nonproliferative diseases.

12. Use according to Claim 11, **characterized in that** the diseases to be prevented or treated are androgenetic alopecia, hirsutism or acne.

13. Use according to Claims 7 or 8, **characterized in that** the disease is benign prostate hyperplasia.

14. Process for producing the compounds of the general formula I according to Claim 1 of the present invention, **characterized in that** compounds of the general formula II 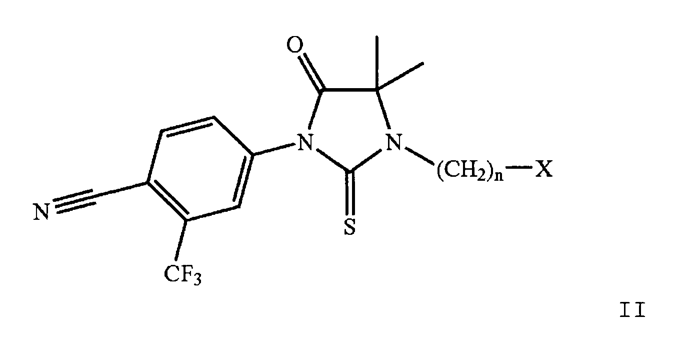 in which
n can mean an integer between 6 and 9,
X can mean a leaving group,
are reacted with compounds of the general formula III 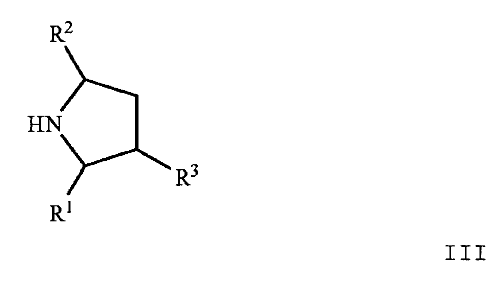 in which
R¹ and R², independently of one another, can mean a hydrogen atom, an unbranched C₁-C₄-alkyl group, a branched C₃-C₅-alkyl group, an unbranched hydroxy-C₁-C₄-alkyl group, a branched hydroxy-C₃-C₅-alkyl group, an unbranched C₁-C₄-alkoxy-C₁-C₄-alkyl group, a branched C₁-C₄-alkoxy-C₃-C₅-alkyl group, an unbranched C₁-C₄-alkanoyloxy-C₁-C₄-alkyl group, a branched C₁-C₄-alkanoyloxy-C₃-C₅-alkyl group, a (pyrrolidin-1-yl)methyl group, a carboxy group, a C₁-C₄-alkoxycarbonyl group or an aminocarbonyl group,
or
R¹ and R² together mean a 2-hydroxypropane-1,3-diyl bridge;
R³ means a hydrogen atom or a hydroxy group,
in the presence of an organic base.

## Revendications

1. Composés de formule générale I 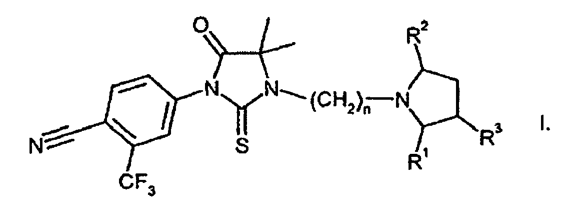 dans laquelle
n représente un nombre entier compris entre 6 et 9,
R¹ et R² représentent, indépendamment l'un de l'autre, un atome d'hydrogène, un groupe alkyle en C₁-C₄ non ramifié, un groupe alkyle en C₃-C₅ ramifié, un groupe hydroxy-alkyle en C₁-C₄ non ramifié, un groupe hydroxy-alkyle en C₃-C₅ ramifié, un groupe alcoxy(C₁-C₄)-alkyle(C₁-C₄) non ramifié, un groupe alcoxy(C₁-C₄)-alkyle(C₃-C₅) ramifié, un groupe alcanoyl (C₁-C₄) oxy-alkyle (C₁-C₄) non ramifié, un groupe alcanoyl(C₁-C₄)oxy-alkyle(C₃-C₅) ramifié, un groupe (pyrrolidin-1-yl)méthyle, un groupe carboxy, un groupe alcoxy(C₁-C₄)carbonyle ou un groupe aminocarbonyle,
ou
R¹ et R² forment ensemble un pont 2-hydroxy-propane-1,3-diyle,
R³ représente un atome d'hydrogène ou un groupe hydroxy,
ainsi que leurs sels physiologiquement acceptables.

2. Composés de formule générale I selon la revendication 1, **caractérisés en ce que** R¹ représente un atome d'hydrogène, un groupe hydroxyméthyle, un groupe aminocarbonyle ou un groupe méthoxyméthyle ; R² et R³ représentent chacun un atome d'hydrogène.

3. Composés de formule générale I selon la revendication 1, **caractérisés en ce que** R¹ représente un atome d'hydrogène, un groupe méthyle ; R² et R³ représentent chacun un atome d'hydrogène.

4. Composés de formule générale I selon la revendication 1, **caractérisés en ce que** R¹ et R² forment ensemble un pont 2-hydroxypropane-1,3-diyle ; R³ représente un atome d'hydrogène.

5. Composés selon l'une quelconque des revendications précédentes, à savoir
**1** 4-[4,4-diméthyl-5-oxo-3-[8-(pyrrolidin-1-yl)-octyl]-2-thioxo-imidazolidin-1-yl]-2-(trifluorométhyl)benzonitrile
**2** chlorhydrate de 4-[4,4-diméthyl-5-oxo-3-[8-(pyrrolidin-1-yl)octyl]-2-thioxoimidazolidin-1-yl]-2-(trifluorométhyl)-benzonitrile
**3** 4-[4,4-diméthyl-5-oxo-3-[6-(pyrrolidin-1-yl)-hexyl]-2-thioxo-imidazolidin-1-yl]-2-(trifluorométhyl)benzonitrile
**4** 4-[4,4-diméthyl-5-oxo-3-[7-(pyrrolidin-1-yl)-heptyl]-2-thioxo-imidazolidin-1-yl]-2-(trifluorométhyl)benzonitrile
**5** 4-[4,4-diméthyl-5-oxo-3-[9-(pyrrolidin-1-yl)nonyl]-2-thioxo-imidazolidin-1-yl]-2-(trifluorométhyl)benzonitrile
**6** 4-[3-[6-[(2R)-2-(hydroxyméthyl)pyrrolidin-1-yl]hexyl]-4,4-diméthyl-5-oxo-2-thioxoimidazolidin-1-yl]-2-(trifluorométhyl)-benzonitrile
**7** 4-[3-[6-[(2S)-2-(hydroxyméthyl)pyrrolidin-1-yl]hexyl]-4,4-diméthyl-5-oxo-2-thioxoimidazolidin-1-yl)-2-(trifluorométhyl)-benzonitrile
**8** 4-[3-[7-[(2R)-2-(hydroxyméthyl)pyrrolidin-1-yl]heptyl]-4,4-diméthyl-5-oxo-2-thioxoimidazolidin-1-yl]-2-(trifluorométhyl)-benzonitrile
**9** 4-[3-[7-[(2S)-2-(hydroxyméthyl)pyrrolidin-1-yl]heptyl]-4,4-diméthyl-5-oxo-2-thioxoimidazolidin-1-yl]-2-(trifluorométhyl)-benzonitrile
**10** 4-[3-[8-[(2R)-2-(hydroxyméthyl)pyrrolidin-1-yl]octyl]-4,4-diméthyl-5-oxo-2-thioxoimidazolidin-1-yl]-2-(trifluorométhyl)-benzonitrile
**11** 4-[3-[8-[(2S)-2-(hydroxyméthyl)pyrrolidin-1-yl]octyl]-4,4-diméthyl-5-oxo-2-thioxoimidazolidin-1-yl]-2-(trifluorométhyl)-benzonitrile
**12** 4-[3-[9-[(2R)-2-(hydroxyméthyl)pyrrolidin-1-yl)nonyl]-4,4-diméthyl-5-oxo-2-thioxoimidazolidin-1-yl]-2-(trifluorométhyl)-benzonitrile
**13** 4-[3-[9-[(2S)-2-(hydroxyméthyl)pyrrolidin-1-yl]nonyl]-4,4-diméthyl-5-oxo-2-thioxoimidazolidin-1-yl]-2-(trifluorométhyl)-benzonitrile
**14** 4-[3-[6-[(2R)-2-(méthoxyméthyl)pyrrolidin-1-yl]hexyl]-4,4-diméthyl-5-oxo-2-thioxoimidazolidin-1-yl]-2-(trifluorométhyl)-benzonitrile
**15** 4-[3-[6-[(2S)-2-(méthoxyméthyl)pyrrolidin-1-yl]hexyl]-4,4-diméthyl-5-oxo-2-thioxoimidazolidin-1-yl]-2-(trifluorométhyl)-benzonitrile
**16** 4-[3-[7-[(2R)-2-(méthoxyméthyl)pyrrolidin-1-yl]heptyl]-4,4-diméthyl-5-oxo-2-thioxoimidazolidin-1-yl]-2-(trifluorométhyl)-benzonitrile
**17** 4-[3-[7-[(2S)-2-(méthoxyméthyl)pyrrolidin-1-yl]heptyl]-4,4-diméthyl-5-oxo-2-thioxoimidazolidin-1-yl]-2-(trifluorométhyl)-benzonitrile
**18** 4-[3-[8-[(2R)-2-(méthoxyméthyl)pyrrolidin-1-yl]octyl]-4,4-diméthyl-5-oxo-2-thioxoimidazolidin-1-yl]-2-(trifluorométhyl)-benzonitrile
**19** 4-[3-[8-[(2S)-2-(méthoxyméthyl)pyrrolidin-1-yl]octyl]-4,4-diméthyl-5-oxo-2-thioxoimidazolidin-1-yl]-2-(trifluorométhyl)-benzonitrile
**20** 4-[3-[9-[(2R)-2-(méthoxyméthyl)pyrrolidin-1-yl]nonyl]-4,4-diméthyl-5-oxo-2-thioxoimidazolidin-1-yl]-2-(trifluorométhyl)-benzonitrile
**21** 4-[3-[9-[(2S)-2-(méthoxyméthyl)pyrrolidin-1-yl]nonyl]-4,4-diméthyl-5-oxo-2-thioxoimidazolidin-1-yl]-2-(trifluorométhyl)-benzonitrile
**22** 4-[3-[6-(3-hydroxy-8-azabicyclo[3.2.1]oct-8-yl)hexyl]-4,4-diméthyl-5-oxo-2-thioxoimidazolidin-1-yl]-2-(trifluorométhyl)-benzonitrile
**23** 4-[3-[7-(3-hydroxy-8-azabicyclo[3.2.1]oct-8-yl)heptyl]-4,4-diméthyl-5-oxo-2-thioxoimidazolidin-1-yl]-2-(trifluorométhyl)-benzonitrile
**24** 4-[3-[8-(3-hydroxy-8-azabicyclo[3.2.1]oct-8-yl)octyl]-4,4-diméthyl-5-oxo-2-thioxoimidazolidin-1-yl]-2-(trifluorométhyl)-benzonitrile
**25** 4-[3-[9-(3-hydroxy-8-azabicyclo[3.2.1]oct-8-yl)nonyl]-4,4-diméthyl-5-oxo-2-thioxoimidazolidin-1-yl]-2-(trifluorométhyl)-benzonitrile
**26** 4-[3-[6-[(R)-3-hydroxypyrrolidin-1-yl]hexyl]-4,4-diméthyl-5-oxo-2-thioxo-imidazolidin-1-yl]-2-(trifluorométhyl)benzonitrile
**27** 4-[3-[7-[(R)-3-hydroxypyrrolidin-1-yl]heptyl]-4,4-diméthyl-5-oxo-2-thioxo-imidazolidin-1-yl]-2-(trifluorométhyl)benzonitrile
**28** 4-[3-[8-[(R)-3-hydroxypyrrolidin-1-yl]octyl]-4,4-diméthyl-5-oxo-2-thioxo-imidazolidin-1-yl]-2-(trifluorométhyl)benzonitrile
**29** 4-[3-[9-[(R)-3-hydroxypyrrolidin-1-yl]nonyl]-4,4-diméthyl-5-oxo-2-thioxo-imidazolidin-1-yl]-2-(trifluorométhyl)benzonitrile
**30** 4-[3-[6-[(S)-3-hydroxypyrrolidin-1-yl]hexyl]-4,4-diméthyl-5-oxo-2-thioxo-imidazolidin-1-yl]-2-(trifluorométhyl)benzonitrile
**31** 4-[3-[7-[(S)-3-hydroxypyrrolidin-1-yl]heptyl]-4,4-diméthyl-5-oxo-2-thioxo-imidazolidin-1-yl]-2-(trifluorométhyl)benzonitrile
**32** 4-[3-[8-[(S)-3-hydroxypyrrolidin-1-yl]octyl]-4,4-diméthyl-5-oxo-2-thioxo-imidazolidin-1-yl]-2-(trifluorométhyl)benzonitrile
**33** 4-[3-[9-[(S)-3-hydroxypyrrolidin-1-yl]nonyl]-4,4-diméthyl-5-oxo-2-thioxo-imidazolidin-1-yl]-2-(trifluorométhyl)benzonitrile
**34** 4-[4,4-diméthyl-3-[6-(2-méthylpyrrolidin-1-yl)hexyl]-5-oxo-2-thioxo-imidazolidin-1-yl]-2-(trifluorométhyl)benzonitrile
**35** 4-[4,4-diméthyl-3-[7-(2-méthylpyrrolidin-1-yl)heptyl]-5-oxo-2-thioxo-imidazolidin-1-yl]-2-(trifluorométhyl)benzonitrile
**36** 4-[4,4-diméthyl-3-[8-(2-méthylpyrrolidin-1-yl)octyl]-5-oxo-2-thioxo-imidazolidin-1-yl]-2-(trifluorométhyl)benzonitrile
**37** 4-[3-[8-[(2R,5S)-rel-2,5-diméthylpyrrolidin-1-yl]octyl]-4,4-diméthyl-5-oxo-2-thioxoimidazolidin-1-yl]-2-(trifluorométhyl)-benzonitrile
**38** 4-[3-[6-[(2S)-2-(l-hydroxy-1-méthyléthyl)-pyrrolidin-1-yl]hexyl]-4,4-diméthyl-5-oxo-2-thioxo-imidazolidin-1-yl]-2-(trifluorométhyl)benzonitrile
**39** 4-[3-[7-[(2S)-2-(1-hydroxy-1-méthyléthyl)-pyrrolidin-1-yl]heptyl]-4,4-diméthyl-5-oxo-2-thioxo-imidazolidin-1-yl]-2-(trifluorométhyl)benzonitrile
**40** 4-[3-[8-[(2S)-2-(1-hydroxy-1-méthyléthyl)-pyrrolidin-1-yl]octyl]-4,4-diméthyl-5-oxo-2-thioxo-imidazolidin-1-yl]-2-(trifluorométhyl)benzonitrile
**41** 4-[3-[9-[(2S)-2-(1-hydroxy-1-méthyléthyl)-pyrrolidin-1-yl]nonyl]-4,4-diméthyl-5-oxo-2-thioxo-imidazolidin-1-yl]-2-(trifluorométhyl)benzonitrile
**42** 4-[3-[6-[(2R)-2-(1-hydroxy-1-méthyléthyl)-pyrrolidin-1-yl]hexyl]-4,4-diméthyl-5-oxo-2-thioxo-imidazolidin-1-yl]-2-(trifluorométhyl)benzonitrile
**43** 4-[3-[7-[(2R)-2-(1-hydroxy-1-méthyléthyl)-pyrrolidin-1-yl]heptyl]-4,4-diméthyl-5-oxo-2-thioxo-imidazolidin-1-yl]-2-(trifluorométhyl)benzonitrile
**44** 4-[3-[8-[(2R)-2-(1-hydroxy-1-méthyléthyl)-pyrrolidin-1-yl]octyl]-4,4-diméthyl-5-oxo-2-thioxo-imidazolidin-1-yl]-2-(trifluorométhyl)benzonitrile
**45** 4-[3-[6-[(2S)-2-(1-hydroxy-1-éthylpropyl)-pyrrolidin-1-yl]hexyl]-4,4-diméthyl-5-oxo-2-thioxo-imidazolidin-1-yl]-2-(trifluorométhyl)benzonitrile
**46** 4-[3-[7-[(2S)-2-(1-hydroxy-1-éthylpropyl)-pyrrolidin-1-yl]heptyl]-4,4-diméthyl-5-oxo-2-thioxo-imidazolidin-1-yl]-2-(trifluorométhyl)benzonitrile
**47** 4-[3-[8-[(2S)-2-(1-hydroxy-1-éthylpropyl)-pyrrolidin-1-yl]octyl]-4,4-diméthyl-5-oxo-2-thioxo-imidazolidin-1-yl]-2-(trifluorométhyl)benzonitrile
**48** (S)-1-[7-[3-[4-cyano-3-(trifluorométhyl)-phényl]-5,5-diméthyl-4-oxo-2-thioxoimidazolidin-1-yl]heptyl]pyrrolidine-2-carboxylate de méthyle
**49** (S)-1-[6-[3-[4-cyano-3-(trifluorométhyl)-phényl]-5,5-diméthyl-4-oxo-2-thioxoimidazolidin-1-yl]hexyl]pyrrolidine-4-carboxamide
**50** (S)-1-[7-[3-[4-cyano-3-(trifluorométhyl)-phényl]-5,5-diméthyl-4-oxo-2-thioxoimidazolidin-1-yl]heptyl]pyrrolidine-4-carboxamide
**51** (S)-1-[8-[3-[4-cyano-3-(trifluorométhyl)-phényl]-5,5-diméthyl-4-oxo-2-thioxoimidazolidin-1-yl]octyl]pyrrolidine-4-carboxamide
**52** (R)-1-[6-[3-[4-cyano-3-(trifluorométhyl)-phényl]-5,5-diméthyl-4-oxo-2-thioxoimidazolidin-1-yl]hexyl]pyrrolidine-4-carboxamide
**53** (R)-1-[7-[3-[4-cyano-3-(trifluorométhyl)-phényl]-5,5-diméthyl-4-oxo-2-thioxoimidazolidin-1-yl]heptyl]pyrrolidine-4-carboxamide
**54** (R)-1-[8-[3-[4-cyano-3-(trifluorométhyl)-phényl]-5,5-diméthyl-4-oxo-2-thioxoimidazolidin-1-yl]octyl]pyrrolidine-4-carboxamide
**55** 4-[3-[6-[(2S)-4,4-diméthyl-5-oxo-2-(pyrrolidin-1-ylméthyl)pyrrolidin-1-yl]hexyl]-2-thioxoimidazolidin-1-yl]-2-(trifluorométhyl)benzonitrile
**56** 4-[3-[7-[(2S)-4,4-diméthyl-5-oxo-2-(pyrrolidin-1-ylméthyl)pyrrolidin-1-yl]heptyl]-2-thioxoimidazolidin-1-yl]-2-(trifluorométhyl)benzonitrile
**57** 4-[3-[8-[(2S)-4,4-diméthyl-5-oxo-2-(pyrrolidin-1-ylméthyl)pyrrolidin-1-yl]octyl]-2-thioxoimidazolidin-1-yl]-2-(trifluorométhyl)benzonitrile
**58** chlorhydrate d'acide (S)-1-[7-[3-[4-cyano-3-(trifluorométhyl)phényl]-5,5-diméthyl-4-oxo-2-thioxoimidazolidin-1-yl]heptyl]pyrrolidin-2-carboxylique.

6. Compositions pharmaceutiques contenant au moins un composé de formule générale I ou ses sels pharmacologiquement acceptables selon l'une quelconque des revendications précédentes, conjointement avec des adjuvants et/ou véhicules pharmaceutiquement acceptables.

7. Utilisation des composés de formule I selon l'une quelconque des revendications précédentes, pour la fabrication d'un médicament destiné au traitement ou à la prophylaxie de maladies de l'organisme humain ou animal, qui peuvent être influencées par l'inhibition du récepteur d'androgène.

8. Utilisation selon la revendication 7, **caractérisée en ce que** les maladies à prévenir ou à traiter sont des maladies prolifératives androgéno-dépendantes.

9. Utilisation selon la revendication 7 ou 8, **caractérisée en ce que** les maladies à traiter sont des maladies tumorales.

10. Utilisation selon l'une quelconque des revendications 7 à 9, **caractérisée en ce que** la maladie à traiter est le carcinome de la prostate.

11. Utilisation selon la revendication 7, **caractérisée en ce que** les maladies à prévenir ou à traiter sont des maladies non prolifératives androgéno-dépendantes.

12. Utilisation selon la revendication 11, **caractérisée en ce que** les maladies à prévenir ou à traiter sont l'alopécie androgénétique, l'hirsutisme ou l'acné.

13. Utilisation selon la revendication 7 ou 8, **caractérisée en ce que** la maladie est l'hyperplasie bénigne de la prostate.

14. Procédé pour la préparation des composés selon l'invention, de formule générale I selon la revendication 1, **caractérisé en ce qu'**on fait réagir en présence d'une base organique des composés de formule générale II 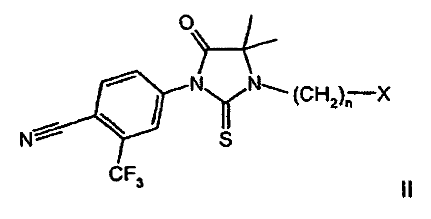 dans laquelle
n est un nombre entier compris entre 6 et 9,
X représente un groupe partant,
avec des composés de formule générale III 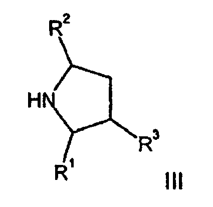 dans laquelle
R¹ et R² peuvent représenter, indépendamment l'un de l'autre, un atome d'hydrogène, un groupe alkyle en C₁-C₄ non ramifié, un groupe alkyle en C₃-C₅ ramifié, un groupe hydroxy-alkyle en C₁-C₄ non ramifié, un groupe hydroxy-alkyle en C₃-C₅ ramifié, un groupe alcoxy(C₁-C₄)-alkyle(C₁-C₄) non ramifié, un groupe alcoxy(C₁-C₄)-alkyle(C₃-C₅) ramifié, un groupe alcanoyl (C₁-C₄) oxy-alkyle (C₁-C₄) non ramifié, un groupe alcanoyl(C₁-C₄)oxy-alkyle(C₃-C₅) ramifié, un groupe (pyrrolidin-1-yl)méthyle, un groupe carboxy, un groupe alcoxy(C₁-C₄)carbonyle ou un groupe aminocarbonyle,
ou
R¹ et R² peuvent former ensemble un pont 2-hydroxypropane-1,3-diyle ;
R³ peut représenter un atome d'hydrogène ou un groupe hydroxy.
